# EUROPEAN PATENT SPECIFICATION

(11) **EP 3 493 809 B1**
(45) Date of publication and mention of the grant of the patent: **23.08.2023**
(21) Application number: 17752530.0
(22) Date of filing: 02.08.2017
(51) Int. Cl.: A61K 31/53, A61K 9/00, A61K 9/02, A61K 9/06, A61K 9/12, A61K 9/28, A61K 9/48, A61K 9/70, A61K 9/20, A61P 35/00

(54) **ENASIDENIB FOR TREATMENT OF MYELODYSPLASTIC SYNDROME**
ENASIDENIB ZUR BEHANDLUNG VON MYELODYSPLASTISCHEM SYNDROM
ENASIDENIB POUR LE TRAITEMENT DU SYNDROME MYÉLODYSPLASIQUE

(30) Priority: 03.08.2016 US 201662370673 P; 02.12.2016 US 201662429649 P
(43) Date of publication of application: 12.06.2019
(73) Proprietor: Celgene Corporation, Summit, NJ 07901 (US)
(72) Inventor: AMATANGELO, Michael, Madison NJ 07940 (US); HU, Xiaolan, Skillman NJ 08558 (US); THAKURTA, Anjan, Basking Ridge NJ 07920 (US)
(74) Representative: Jones Day
(86) International application number: PCT/US2017/045055
(87) International publication number: WO 2018/026894

(56) References cited:
- WO-A1-2014/183122
- WO-A1-2016/126798
- WO-A2-2012/174419
- WO-A2-2015/017821
- PRAJWAL BODDU ET AL: "Therapeutic targeting of isocitrate dehydrogenase mutant AML", EXPERT OPINION ON INVESTIGATIONAL DRUGS, vol. 26, no. 5, 19 April 2017 (2017-04-19) , pages 525-530, XP055414093, UK ISSN: 1354-3784, DOI: 10.1080/13543784.2017.1317745

## Description

### FIELD

Provided herein is compound 1 for use in a method of treating a myelodysplastic syndrome characterized by the presence of a mutant allele of IDH2 and a mutant allele of a second gene, wherein the second gene is selected from the group consisting of ASXL1 and SRSF2; or a myelodysplastic syndrome characterized by the presence of a mutant allele of IDH2 and the absence of a mutant allele of at least one other gene, wherein the other gene is selected from the group consisting of KRAS, TP53, SETBP1, and U2AF1; or a myelodysplastic syndrome, characterized by the presence of a mutant allele of IDH2 and a mutant allele of a second gene, wherein the second gene is selected from the group consisting of ASXL1 and SRSF2, and the absence of a mutant allele of at least one other gene, wherein the other gene is selected from the group consisting of KRAS, TP53, SETBP1, and U2AF1. In certain examples, described herein are methods of treating a myelodysplastic syndrome, characterized by the presence of a mutant allele of IDH2 and a mutant allele of at least one second gene, wherein the second gene is selected from the group consisting of ASXL1, SRSF2, KRAS, DNMT3A, MPL, CSF3R, FAT3, and CBL; or a myelodysplastic syndrome, characterized by the presence of a mutant allele of IDH2 and the absence of a mutant allele of at least one other gene, wherein the other gene is selected from the group consisting of ASXL1, SRSF2, KRAS, TP53, SETBP1, U2AF1, TCF3, STAG2, NRAS, JAK2 and BRAF; or a myelodysplastic syndrome, characterized by the presence of a mutant allele of IDH2 and a mutant allele of at least one second gene, wherein the second gene is selected from the group consisting of ASXL1, SRSF2, DNMT3A, MPL, CSF3R, FAT3, and CBL and the absence of a mutant allele of at least one other gene, wherein the other gene is selected from the group consisting of KRAS, TP53, SETBP1, U2AF1, TCF3 STAG2, NRAS, JAK2 and BRAF. In certain embodiments, provided herein is compound 1 for use in a method of treating a myelodysplastic syndrome characterized by the presence of a mutant allele of IDH2 and a mutant allele of at least one second gene, wherein the second gene is selected from the group consisting of MPL, DNMT3A, CSF3R, FAT3, or CBL. In certain examples, described herein are methods of treating a myelodysplastic syndrome characterized by the presence of a mutant allele of IDH2 and the absence of a mutant allele of at least one other gene, wherein the other gene is selected from the group consisting of TP53, SETBP1, U2AF1, TCF3, STAG2, NRAS, JAK2 and BRAF. Also provided herein is COMPOUND 1 for use in such methods of treating a myelodysplastic syndrome, wherein COMPOUND 1 is 2-methyl-1-[(4-[6-(trifluoromethyl)pyridin-2-yl]-6- f [2-(trifluoromethyl)pyridin-4-yl]amino}-1,3,5-triazin-2-yl)amino]propan-2-ol or a pharmaceutically acceptable salt, solvate, tautomer, metabolite, or a polymorph thereof. 2-Methyl-1-[(4-[6-(trifluoromethyl)pyridin-2-yl]-6-{[2-(trifluoromethyl)pyridin-4-yl]amino}-1,3,5-triazin-2-yl)amino]propan-2-ol has the following formula:

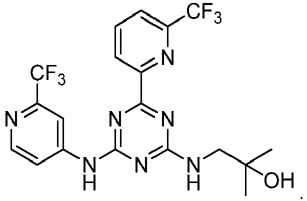

### BACKGROUND

### BACKGROUND OF THE DISCLOSURE

WO 2015/017821 A2 describes methods of treating advanced hematologic malignancies, such as acute myelogenous leukemia (AML), myelodysplastic syndrome (MDS), chronic myelomonocytic leukemia (CMML), myeloid sarcoma, multiple myeloma, or lymphoma (e.g T-cell lymphoma or B-cell lymphoma), each characterized by the presence of a mutant allele of IDH2.

WO 2012/174419 A2 is related to the identification of SIGNATURES that when somatically mutated associated with adverse prognosis for subjects with hematological disorders, such as myelodysplastic syndromes (MDS), acute myelogenous leukemia (AML), Acute lymphoblastic leukemia (ALL), Chronic lymphocytic leukemia (CLL), Chronic myelogenous leukemia (CML), or at risk of developing hematological disorders.

WO 2014/183122 A1 describes methods for predicting a response of a subject with myelodysplastic syndrome to treatment with a hypomethylating agent, for example using a sample obtained from the subject. The methods can be used to select a subject for treatment with a hypometylating agent, and/or measure a subject's response to therapy and/or disease progression/regression.

Isocitrate dehydrogenases (IDHs) catalyze the oxidative decarboxylation of isocitrate to 2-oxoglutarate (i.e., α-ketoglutarate). These enzymes belong to two distinct subclasses, one of which utilizes NAD(+) as the electron acceptor and the other NADP(+). Five isocitrate dehydrogenases have been reported: three NAD(+)-dependent isocitrate dehydrogenases, which localize to the mitochondrial matrix, and two NADP(+)-dependent isocitrate dehydrogenases, one of which is mitochondrial and the other predominantly cytosolic. Each NADP(+)-dependent isozyme is a homodimer.

IDH2 (isocitrate dehydrogenase 2 (NADP+), mitochondrial) is also known as IDH; IDP; IDHM; IDPM; ICD-M; or mNADP-IDH. The protein encoded by this gene is the NADP(+)-dependent isocitrate dehydrogenase found in the mitochondria. It plays a role in intermediary metabolism and energy production. This protein may tightly associate or interact with the pyruvate dehydrogenase complex. Human IDH2 gene encodes a protein of 452 amino acids. The nucleotide and amino acid sequences for IDH2 can be found as GenBank entries NM_002168.2 and NP_002159.2 respectively. The nucleotide and amino acid sequence for human IDH2 are also described in, *e.g.,* Huh et al., Submitted (NOV-1992) to the EMBL/GenBank/DDBJ databases; and The MGC Project Team, Genome Res. 14:2121-2127 (2004).

Non-mutant, e.g., wild type, IDH2 catalyzes the oxidative decarboxylation of isocitrate to α-ketoglutarate (α-KG) thereby reducing NAD⁺ (NADP⁺) to NADH (NADPH), *e.g.,* in the forward reaction:

Isocitrate + NAD⁺ (NADP⁺) → α-KG + CO₂ + NADH (NADPH) + H⁺.

It has been discovered that mutations of IDH2 present in certain cancer cells result in a new ability of the enzyme to catalyze the NADPH-dependent reduction of α-ketoglutarate to R(-)-2-hydroxyglutarate (2HG). 2HG is not formed by wild-type IDH2. The production of 2HG is believed to contribute to the formation and progression of cancer (Dang, L. et al., Nature 462:739-44, 2009).

The development of selective inhibitors of IDH2 mutant enzyme has provided the possibility of therapeutic benefit to cancer patients carrying the IDH2 mutations. There is a need for improved therapies for treating cancer patients carrying IDH2 mutations.

### SUMMARY

In one embodiment, the invention is directed to a compound for use in a method of treating a myelodysplastic syndrome in a subject, wherein the compound is 2-methyl-1-[(4-[6 (trifluoromethyl)pyridin-2-yl]-6-{[2-(trifluoromethyl)pyridin-4-yl]amino}-1,3,5-triazin-2 yl)amino]propan-2-ol having the following formula: 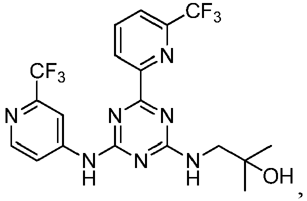 or a pharmaceutically acceptable salt, solvate, tautomer, or a polymorph thereof (COMPOUND 1), wherein the myelodysplastic syndrome is characterized by the presence of a mutant allele of IDH2 and the absence of a mutant allele of at least one other gene, wherein the other gene is selected from the group consisting of KRAS, TP53, SETBP1, and U2AF1.

In another embodiment, the myelodysplastic syndrome is further characterized by the presence of one or more mutant alleles of one or more additional genes.

In another embodiment, the myelodysplastic syndrome is characterized by the absence of a mutant allele of KRAS or the myelodysplastic syndrome is characterized by the absence of a mutant allele of TP53 and/or the myelodysplastic syndrome is characterized by the absence of a mutant allele of SETBP1 and/or the myelodysplastic syndrome is characterized by the absence of a mutant allele of U2AF1.

In another embodiment, the myelodysplastic syndrome is characterized by the absence of a mutant allele of KRAS and TP53.

In another embodiment, the myelodysplastic syndrome is characterized by the presence of a mutant allele of a second gene, wherein the second gene is selected from the group consisting of ASXL1 and SRSF2.

In another embodiment, the myelodysplastic syndrome is characterized by the presence of a mutant allele of ASXL1 or the myelodysplastic syndrome is characterized by the presence of a mutant allele of SRSF2 or the myelodysplastic syndrome is characterized by the presence of mutant alleles of ASXL1 and SRSF2.

In another embodiment, the mutant allele of IDH2 is mIDH2-R140 or mIDH2-R172, preferably, wherein the mutant allele of IDH2 is mIDH2-R140Q, mIDH2-R140W, mIDH2-R140L, mIDH2-R172K, or mIDH2-R172G.

In another embodiment, COMPOUND 1 is administered at a dose of about 20 to 2000 mg/day or COMPOUND 1 is administered at a dose of about 50 to 500 mg/day or the dose is about 60 mg/day, 100 mg/day, 150 mg/day, 200 mg/day or 300 mg/day.

In another embodiment, COMPOUND 1 is administered once daily and/or COMPOUND 1 is administered for 1 to 25 cycles or COMPOUND 1 is administered in 28-day cycles.

In another embodiment, COMPOUND 1 is administered orally, preferably COMPOUND 1 is administered once daily orally in 28-day cycles at the dose of about 100 mg/day.

In another embodiment, COMPOUND 1 is a mesylate salt of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol.

In another embodiment, the myelodysplastic syndrome is with excess blasts, subtype RAEB-1 or RAEB-2 and/or the myelodysplastic syndrome is untreated.

In another embodiment, COMPOUND 1 is administered as a first line treatment for MDS or COMPOUND 1 is administered as a second line, third line, or fourth line of treatment for the myelodysplastic syndrome.

In another embodiment, the myelodysplastic syndrome is subsequent to acute myeloid leukemia.

In another embodiment, the invention is directed to a pharmaceutical composition for use in a method of treating a myelodysplastic syndrome in a subject, wherein the pharmaceutical composition comprises a therapeutically effective amount of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol having the following formula: 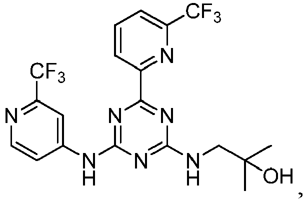 or a pharmaceutically acceptable salt, solvate, tautomer, or a polymorph thereof (COMPOUND 1) and an excipient, wherein the myelodysplastic syndrome is characterized by the presence of a mutant allele of IDH2 and the absence of a mutant allele of at least one other gene, wherein the other gene is selected from the group consisting of KRAS, TP53, SETBP1, and U2AF1.

Described herein is COMPOUND 1 which is 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol or a pharmaceutically acceptable salt, solvate, tautomer, or a polymorph thereof. 2-Methyl-1-[(4-[6-(trifluoromethyl)pyridin-2-yl]-6- f [2-(trifluoromethyl)pyridin-4-yl]amino}-1,3,5-triazin-2-yl)amino]propan-2-ol has the following formula:

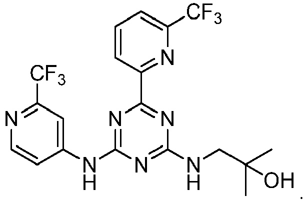

Described herein is COMPOUND A which is a mesylate salt of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol.

In certain embodiments, provided herein is COMPOUND 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2.

In certain embodiments, provided herein is COMPOUND 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2, and the absence of a mutant allele of at least one other gene, wherein the other gene is selected from the group consisting of KRAS, TP53, SETBP1, and U2AF1.

In certain embodiments, provided herein is COMPOUND 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of at least one other gene, wherein the other gene is selected from the group consisting of KRAS, TP53, SETBP1, and U2AF1.

In certain examples, described herein are methods of treating a myelodysplastic syndrome, characterized by the presence of a mutant allele of IDH2 and a mutant allele of at least one second gene, wherein the second gene is selected from the group consisting of ASXL1, SRSF2, KRAS, DNMT3A, MPL, CSF3R, FAT3, and CBL. Accordingly, described herein is COMPOUND 1 for use in said methods of treating a myelodysplastic syndrome, characterized by the presence of a mutant allele of IDH2 and a mutant allele of at least one second gene, wherein the second gene is selected from the group consisting of ASXL1, SRSF2, KRAS, DNMT3A, MPL, CSF3R, FAT3, and CBL.

In certain examples, described herein are methods of treating a myelodysplastic syndrome, characterized by the presence of a mutant allele of IDH2 and the absence of a mutant allele of at least one other gene, wherein the other gene is selected from the group consisting of ASXL1, SRSF2, KRAS, TP53, SETBP1, U2AF1, TCF3, STAG2, NRAS, JAK2 and BRAF. Accordingly, described herein is COMPOUND 1 for use in said methods of treating a myelodysplastic syndrome, characterized by the presence of a mutant allele of IDH2 and the absence of a mutant allele of at least one other gene, wherein the other gene is selected from the group consisting of ASXL1, SRSF2, KRAS, TP53, SETBP1, U2AF1, TCF3, STAG2, NRAS, JAK2 and BRAF.

In certain examples, described herein are methods of treating a myelodysplastic syndrome, characterized by the presence of a mutant allele of IDH2 and a mutant allele of at least one second gene, wherein the second gene is selected from the group consisting of ASXL1, SRSF2, DNMT3A, MPL, CSF3R, FAT3, and CBL and the absence of a mutant allele of at least one other gene, wherein the other gene is selected from the group consisting of KRAS, TP53, SETBP1, U2AF1, TCF3, STAG2, NRAS, JAK2 and BRAF. Accordingly, described herein is COMPOUND 1 for use in said methods of treating a myelodysplastic syndrome, characterized by the presence of a mutant allele of IDH2 and a mutant allele of at least one second gene, wherein the second gene is selected from the group consisting of ASXL1, SRSF2, DNMT3A, MPL, CSF3R, FAT3, and CBL and the absence of a mutant allele of at least one other gene, wherein the other gene is selected from the group consisting of KRAS, TP53, SETBP1, U2AF1, TCF3, STAG2, NRAS, JAK2 and BRAF.

In certain embodiments, provided herein is COMPOUND 1 for use in a method of treating a myelodysplastic syndrome characterized by the presence of a mutant allele of IDH2 and a mutant allele of at least one second gene, wherein the second gene is selected from the group consisting of MPL, DNMT3A, CSF3R, FAT3, or CBL.

Described herein is COMPOUND 1 for use in methods of treating a myelodysplastic syndrome characterized by the presence of a mutant allele of IDH2 and the absence of a mutant allele of at least one other gene, wherein the other gene is selected from the group consisting of TP53, SETBP1, U2AF1, TCF3, STAG2, NRAS, JAK2 and BRAF.

In certain embodiments, the mutant allele of IDH2 is mIDH2-R140 or mIDH2-R172.

In certain embodiments, the mutant allele of IDH2 is mIDH2-R140Q, mIDH2-R140W, mIDH2-R140L, mIDH2-R172K, or mIDH2-R172G.

### BRIEF DESCRIPTION OF THE DRAWINGS

**Figure 1** is an X-ray powder diffractogram of COMPOUND 1 form 1.
**Figure 2** is an X-ray powder diffractogram of COMPOUND 1 form 2.
**Figure 3** is an X-ray powder diffractogram of COMPOUND 1 form 3.
**Figure 4** is an X-ray powder diffractogram of COMPOUND 1 form 4.
**Figure 5** is an X-ray powder diffractogram of COMPOUND 1 form 5.
**Figure 6** is an X-ray powder diffractogram of COMPOUND 1 form 6.
**Figure 7** is an X-ray powder diffractogram of COMPOUND A form 3.
**Figure 8** illustrates duration of treatment, time to first response, best response, and study outcomes for the study of COMPOUND A monotherapy in patients with MDS and mIDH2.
**Figure 9** illustrates most frequent co-occurring known somatic gene mutations, sorted by response, for the study of COMPOUND A monotherapy in patients with MDS and mIDH2.
**Figure 10** illustrates duration of treatment and study outcomes for the study of COMPOUND A monotherapy in patients with MDS and a mutant allele of IDH2.

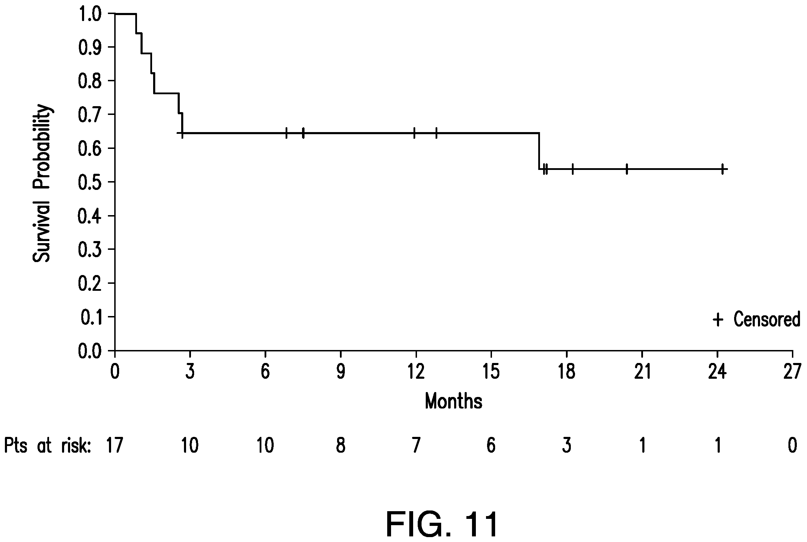

**Figure 11** illustrates overall survival for the study of COMPOUND A monotherapy in patients with MDS and a mutant allele of IDH2.
**Figure 12** illustrates co-occurring somatic gene mutations and clinical response, for the study of COMPOUND A monotherapy in MDS patients having a mutant allele of IDH2. Filled tile indicates presence of mutation, different colors of tiles used to highlight mutations associated with response: Green = Response, and Red = No Response
**Figure 13** illustrates co-mutational frequency in MDS patients having a mutant allele of IDH2.

### DETAILED DESCRIPTION

The details of construction and the arrangement of components set forth in the following description or illustrated in the drawings are not meant to be limiting. Other examples and different ways to practice the methods and compounds are expressly included. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

### Definitions

As used herein, the terms "comprising" and "including" can be used interchangeably. The terms "comprising" and "including" are to be interpreted as specifying the presence of the stated features or components as referred to, but do not preclude the presence or addition of one or more features, or components, or groups thereof. Additionally, the terms "comprising" and "including" are intended to include examples encompassed by the term "consisting of'. Consequently, the term "consisting of" can be used in place of the terms "comprising" and "including" to provide for more specific embodiments.

The term "consisting of" means that a subject-matter has at least 90%, 95%, 97%, 98% or 99% of the stated features or components of which it consists. In another embodiment the term "consisting of" excludes from the scope of any succeeding recitation any other features or components, excepting those that are not essential to the technical effect to be achieved.

As used herein, the term "or" is to be interpreted as an inclusive "or" meaning any one or any combination. Therefore, "A, B or C" means any of the following: "A; B; C; A and B; A and C; B and C; A, B and C". An exception to this definition will occur only when a combination of elements, functions, steps or acts are in some way inherently mutually exclusive.

As used herein, the term "wild type" refers to the typical or most common form of a characteristic (for example, gene sequence or presence, or protein sequence, presence, level or activity), as it occurs in nature, and the reference against which all others are compared. As will be understood by one skilled in the art, when used herein, wild type refers to the typical gene sequence(s) or gene expression levels as they most commonly occur in nature.

The term "elevated levels of 2HG" means 10%, 20% 30%, 50%, 75%, 100%, 200%, 500% or more 2HG is present in a subject that carries a mutant IDH2 allele than is present in a subject that does not carry a mutant IDH2 allele. The term "elevated levels of 2HG" may refer to the amount of 2HG within a cell, within a tumor, within an organ comprising a tumor, or within a bodily fluid.

The term "bodily fluid" includes one or more of amniotic fluid surrounding a fetus, aqueous humor, blood (*e*.*g*., blood plasma), serum, cerebrospinal fluid, cerumen, chyme, Cowper's fluid, female ejaculate, interstitial fluid, lymph, breast milk, mucus (*e*.*g*., nasal drainage or phlegm), pleural fluid, pus, saliva, sebum, semen, serum, sweat, tears, urine, vaginal secretion, or vomit.

The terms "inhibit" or "prevent" include both complete and partial inhibition and prevention. An inhibitor may completely or partially inhibit the intended target.

The term "subject" is intended to include human and non-human animals. Exemplary human subjects include a human patient (referred to as a patient) having a disorder, e.g., a disorder described herein or a normal subject. The term "non-human animals" in one aspect includes all vertebrates, e.g., non-mammals (such as chickens, amphibians, reptiles) and mammals, such as non-human primates, domesticated and/or agriculturally useful animals, e.g., sheep, dog, cat, cow, pig, etc. In one aspect, the patient is an adult patient.

The term "treat" means decrease, suppress, attenuate, diminish, arrest, or stabilize the development or progression of a disease/disorder (e.g., a myelodysplastic syndrome (MDS), characterized by the presence of a mutant allele of IDH2 and the absence of a mutant allele of at least one other gene, wherein the other gene is selected from the group consisting of KRAS, TP53, SETBP1, and U2AF1; or a myelodysplastic syndrome, characterized by the presence of a mutant allele of IDH2 and a mutant allele of a second gene, for example, ASXL1 and SRSF2, and the absence of a mutant allele of at least one other gene, wherein the other gene is selected from the group consisting of KRAS, TP53, SETBP1, and U2AF1, lessen the severity of the disease/disorder or improve the symptoms associated with the disease/disorder.

An amount of a compound, including a pharmaceutically acceptable salt, solvate, tautomer, or a polymorph thereof, effective to treat a disorder, or a "therapeutically effective amount" or "therapeutically effective dose" refers to an amount of the compound, including a pharmaceutically acceptable salt, solvate, tautomer, or a polymorph thereof, which is effective, upon single or multiple dose administration to a subject, in treating a cell, or in curing, alleviating, relieving or improving a subject with a disorder beyond that expected in the absence of such treatment.

The term "substantially free of other stereoisomers" as used herein means a preparation enriched in a compound having a selected stereochemistry at one or more selected stereocenters by at least about 60%, 65%, 70%, 75%, 80%, 85%, 90%, 95%, 96%, 97%, 98%, or 99%.

The term "enriched" means that at least the designated percentage of a preparation is the compound having a selected stereochemistry at one or more selected stereocenters.

The term "crystalline" refers to a solid having a highly regular chemical structure. In particular, a crystalline COMPOUND 1 may be produced as one or more single crystalline forms of COMPOUND 1. For the purposes of this application, the terms "crystalline form", "single crystalline form" and "polymorph" are synonymous; the terms distinguish between crystals that have different properties (e.g., different XRPD patterns and/or different DSC scan results). The term "polymorph" includes pseudopolymorphs, which are typically different solvates of a material, and thus their properties differ from one another. Thus, each distinct polymorph and pseudopolymorph of COMPOUND 1 is considered to be a distinct single crystalline form herein.

The term "substantially crystalline" refers to forms that may be at least a particular weight percent crystalline. Particular weight percentages are 10%, 20%, 30%, 40%, 50%, 60%, 70%, 75%, 80%, 85%, 87%, 88%, 89%, 90%, 91%, 92%, 93%, 94%, 95%, 96%, 97%, 98%, 99%, 99.5%, 99.9%, or any percentage between 10% and 100%. In some embodiments, substantially crystalline COMPOUND 1 refers to a COMPOUND 1 that is at least 70% crystalline. In other embodiments, substantially crystalline COMPOUND 1 refers to a COMPOUND 1 that is at least 90% crystalline.

The term "isolated" refers to forms that may be at least a particular weight percent of a particular crystalline form of a compound. Particular weight percentages are 90%, 91%, 92%, 93%, 94%, 95%, 96%, 97%, 98%, 99%, 99.5%, 99.9%, or any percentage between 90% and 100%.

The term "solvate or solvated" means a physical association of a compound, including a crystalline form thereof, provided herein with one or more solvent molecules. This physical association includes hydrogen bonding. In certain instances the solvate will be capable of isolation, for example when one or more solvent molecules are incorporated in the crystal lattice of the crystalline solid. "Solvate or solvated" encompasses both solution-phase and isolable solvates. Representative solvates include, for example, a hydrate, ethanolates or a methanolate.

The term "hydrate" is a solvate wherein the solvent molecule is H₂O that is present in a defined stoichiometric amount, and may, for example, include hemihydrate, monohydrate, dihydrate, or trihydrate.

The term "mixture" is used to refer to the combined elements of the mixture regardless of the phase-state of the combination (e.g., liquid or liquid/ crystalline).

The term "seeding" is used to refer to the addition of a crystalline material to initiate recrystallization or crystallization.

The term "antisolvent" is used to refer to a solvent in which compounds, including crystalline forms thereof, are poorly soluble.

The term "pharmaceutically acceptable carrier or adjuvant" refers to a carrier or adjuvant that may be administered to a subject, together with a compound of one aspect, and which does not destroy the pharmacological activity thereof and is nontoxic when administered in doses sufficient to deliver a therapeutic amount of the compound.

The term "a pharmaceutically-acceptable salt" as used herein refers to non-toxic acid or base addition salts of the compound to which the term refers. Examples of pharmaceutically acceptable salts are discussed in Berge et al., 1977, "Pharmaceutically Acceptable Salts." J. Pharm. Sci. Vol. 66, pp. 1-19. In one embodiment, the pharmaceutically-acceptable salt is a mesylate salt.

The term "about" means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. As used herein, and unless otherwise specified, the terms "about" and "approximately," when used in connection with a numeric value or range of values which is provided to characterize a particular solid form, *e.g.,* a specific temperature or temperature range, such as, for example, that describes a melting, dehydration, desolvation, or glass transition temperature; a mass change, such as, for example, a mass change as a function of temperature or humidity; a solvent or water content, in terms of, for example, mass or a percentage; or a peak position, such as, for example, in analysis by, for example, IR or Raman spectroscopy or XRPD; indicate that the value or range of values may deviate to an extent deemed reasonable to one of ordinary skill in the art while still describing the solid form. Techniques for characterizing crystal forms and amorphous solids include, but are not limited to, thermal gravimetric analysis (TGA), differential scanning calorimetry (DSC), X-ray powder diffractometry (XRPD), single-crystal X-ray diffractometry, vibrational spectroscopy, e.g., infrared (IR) and Raman spectroscopy, solid-state and solution nuclear magnetic resonance (NMR) spectroscopy, optical microscopy, hot stage optical microscopy, scanning electron microscopy (SEM), electron crystallography and quantitative analysis, particle size analysis (PSA), surface area analysis, solubility studies, and dissolution studies. In certain embodiments, the terms "about" and "approximately," when used in this context, indicate that the numeric value or range of values may vary within 30%, 20%, 15%, 10%, 9%, 8%, 7%, 6%, 5%, 4%, 3%, 2%, 1.5%, 1%, 0.5%, or 0.25% of the recited value or range of values. For example, in some embodiments, the value of an XRPD peak position may vary by up to ±0.1° 2θ (or ±0.2° 2θ) while still describing the particular XRPD peak.

### COMPOUND 1

In one embodiment, COMPOUND 1 is 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol, or a pharmaceutically acceptable salt, solvate, tautomer, metabolite, or a polymorph thereof, having the following formula:

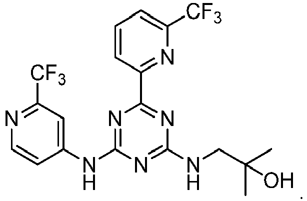

COMPOUND 1 may also comprise one or more isotopic substitutions ("Isotopologues"). For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; and the like. For example, COMPOUND 1 is enriched in a specific isotopic form of H, C and/or O by at least about 60%, 65%, 70%, 75%, 80%, 85%, 90%, 95%, 96%, 97%, 98%, or 99%.

COMPOUND 1 in certain embodiments may also be represented in multiple tautomeric forms, in such instances, one aspect expressly includes all tautomeric forms of COMPOUND 1 described herein, even though only a single tautomeric form may be represented (e.g., keto-enol tautomers). All such isomeric forms of COMPOUND 1 are expressly included herein. Synthesis of COMPOUND 1 is described in US published application US-2013-0190287-A1 published July 25, 2013.

It may be convenient or desirable to prepare, purify, and/or handle a corresponding salt of COMPOUND 1, for example, a pharmaceutically-acceptable salt. Examples of pharmaceutically acceptable salts are discussed in Berge et al., 1977, "Pharmaceutically Acceptable Salts." J. Pharm. Sci. Vol. 66, pp. 1-19.

For example, if COMPOUND 1 is anionic, or has a functional group which may be anionic (e.g., -NH- may be -N-⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺. Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If COMPOUND 1 is cationic, or has a functional group that may be cationic (*e*.*g*., -NHR may be -NH₂R⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucoheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. In one embodiment, COMPOUND 1 comprises the mesylate salt of 2-methyl-1-[(4-[6-(trifluoromethyl)pyridin-2-yl]-6-{ [2-(trifluoromethyl)pyridin-4-yl]amino}-1,3,5-triazin-2-yl)amino]propan-2-ol (COMPOUND A). Examples of suitable polymeric organic anions include, but are not limited to, those derived from the following polymeric acids: tannic acid, carboxymethyl cellulose.

COMPOUND 1 for use in a method of treating a myelodysplastic syndrome in a subject and pharmaceutical compositions provided herein therefore includes the COMPOUND 1 itself, as well as its pharmaceutically acceptable salts, solvates, tautomers, or polymorphs. Metabolites of COMPOUND 1 are disclosed in patent application publication WO2015/006592. COMPOUND 1 described herein may be modified and converted to a prodrug by appending appropriate functionalities to enhance selected biological properties, e.g., targeting to a particular tissue. Such modifications (i.e., prodrugs) are known in the art and include those which increase biological penetration into a given biological compartment (e.g., blood, lymphatic system, central nervous system), increase oral availability, increase solubility to allow administration by injection, alter metabolism and alter rate of excretion. Examples of prodrugs include esters (e.g., phosphates, amino acid (e.g.,valine) esters), carbamates and other pharmaceutically acceptable derivatives, which, upon administration to a subject, are capable of providing active compounds.

It has been found that COMPOUND 1 can exist in a variety of solid forms. In one embodiment, provided herein are solid forms that include neat crystal forms. In another embodiment, provided herein are solid forms that include solvated forms and amorphous forms. The present disclosure provides certain solid forms of COMPOUND 1. In certain embodiments, the present disclosure provides compositions comprising COMPOUND 1 in a form described herein. In some embodiments of provided compositions, COMPOUND 1 is present as a mixture of one or more solid forms; in some embodiments of provided compositions, COMPOUND 1 is present in a single form.

In one embodiment, COMPOUND 1 is a single crystalline form, or any one of the single crystalline forms described herein. Synthesis of crystalline forms of COMPOUND 1 is described in the international application publication WO 2015/017821 published February 5, 2015 and the international application Serial No. WO 2016/126798, published August 11, 2016. Also described are pharmaceutical compositions comprising at least one pharmaceutically acceptable carrier or diluent; and COMPOUND 1, wherein COMPOUND 1 is a single crystalline form, or any one of the crystalline forms being described herein. Also described are uses of COMPOUND 1, wherein COMPOUND 1 is a single crystalline form, or any one of the single crystalline forms described herein, to prepare a pharmaceutical composition.

Provided herein is an assortment of characterizing information to describe the crystalline forms of COMPOUND 1. It should be understood, however, that not all such information is required for one skilled in the art to determine that such particular form is present in a given composition, but that the determination of a particular form can be achieved using any portion of the characterizing information that one skilled in the art would recognize as sufficient for establishing the presence of a particular form, e.g., even a single distinguishing peak can be sufficient for one skilled in the art to appreciate that such particular form is present.

In one embodiment, at least a particular percentage by weight of COMPOUND 1 is crystalline. Particular weight percentages may be 10%, 20%, 30%, 40%, 50%, 60%, 70%, 75%, 80%, 85%, 87%, 88%, 89%, 90%, 91%, 92%, 93%, 94%, 95%, 96%, 97%, 98%, 99%, 99.5%, 99.9%, or any percentage between 10% and 100%. When a particular percentage by weight of COMPOUND 1 is crystalline, the remainder of COMPOUND 1 is the amorphous form of COMPOUND 1. Non-limiting examples of crystalline COMPOUND 1 include a single crystalline form of COMPOUND 1 or a mixture of different single crystalline forms. In some embodiments, COMPOUND 1 is at least 90% by weight crystalline. In some other embodiments, COMPOUND 1 is at least 95% by weight crystalline.

In another embodiment, a particular percentage by weight of the crystalline COMPOUND 1 refers to a particular percentage by weight of a specific single crystalline form or a combination of single crystalline forms. Particular weight percentages may be 10%, 20%, 30%, 40%, 50%, 60%, 70%, 75%, 80%, 85%, 87%, 88%, 89%, 90%, 91%, 92%, 93%, 94%, 95%, 96%, 97%, 98%, 99%, 99.5%, 99.9%, or any percentage between 10% and 100%. In another embodiment, COMPOUND 1 is at least 90% by weight of a single crystalline form. In another embodiment, COMPOUND 1 is at least 95% by weight of a single crystalline form.

In the following description of COMPOUND 1, embodiments may be described with reference to a particular crystalline form of COMPOUND 1, as characterized by one or more properties as discussed herein. The descriptions characterizing the crystalline forms may also be used to describe the mixture of different crystalline forms that may be present in a crystalline COMPOUND 1. However, the particular crystalline forms of COMPOUND 1 may also be characterized by one or more of the characteristics of the crystalline form as described herein, with or without regard to referencing a particular crystalline form.

The crystalline forms are further illustrated by the detailed descriptions and illustrative examples given below. The XRPD peaks described in Tables 1 to 6a may vary by ± 0.2° depending upon the instrument used to obtain the data. The intensity of the XRPD peaks described in Tables 1 to 6a may vary by 10%.

### Form 1 of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol

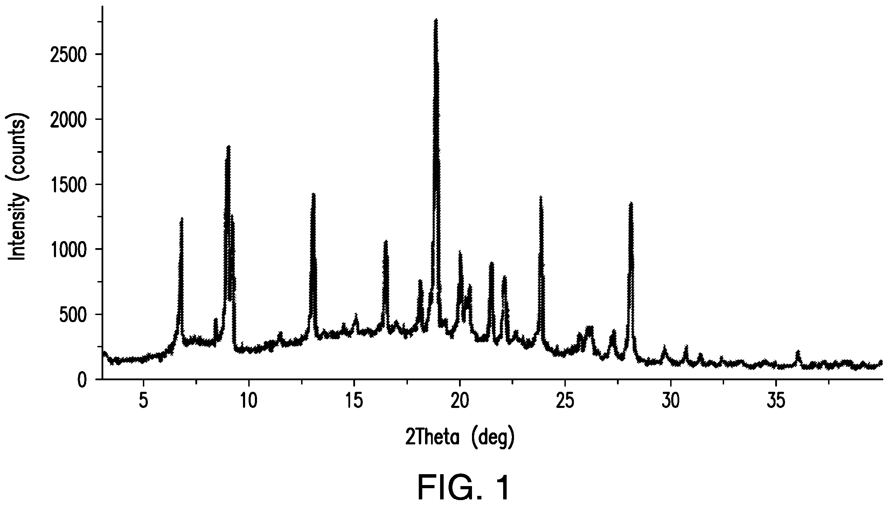

In one embodiment, a single crystalline form, Form 1, of COMPOUND 1 is characterized by the X-ray powder diffraction (XRPD) pattern shown in FIG. 1, and data shown in Table 1 obtained using CuKα radiation. In a particular embodiment, the polymorph can be characterized by one or more of the peaks taken from FIG. 1, as shown in Table 1. For example, the polymorph can be characterized by one or two or three or four or five or six or seven or eight or nine of the peaks shown in Table 1.

**Table 1**

| **Angle** | **Intensity %** |
|---|---|
| 6.7 | 42.2 |
| 8.9 | 61.8 |
| 9.1 | 41.9 |
| 13.0 | 46.7 |
| 16.4 | 33.2 |
| 18.9 | 100.0 |
| 21.4 | 27.3 |
| 23.8 | 49.2 |
| 28.1 | 47.5 |

In another embodiment, Form 1 can be characterized by the peaks identified at 2θ angles of 8.9, 13.0, 18.9, 23.8, and 28.1°. In another embodiment, Form 1 can be characterized by the peaks identified at 2θ angles of 8.9, 18.9, and 23.8°.

### Form 2 of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol

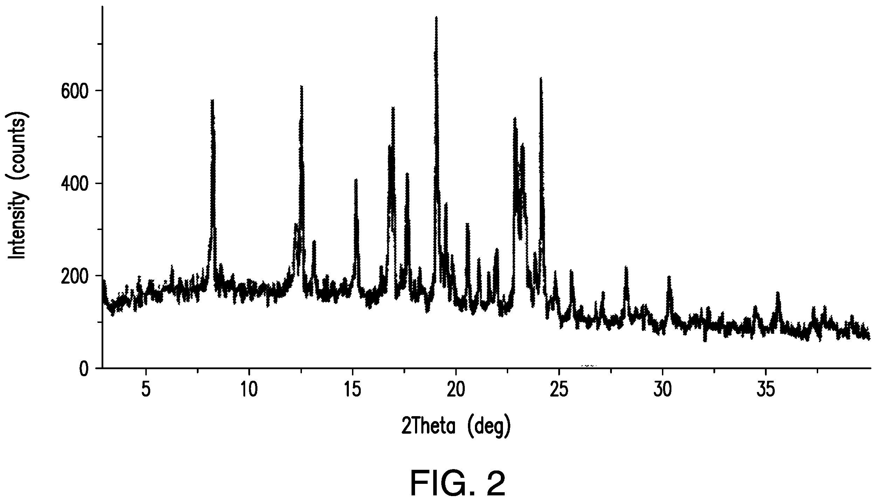

In one embodiment, a single crystalline form, Form 2, of COMPOUND 1 is characterized by the XRPD pattern shown in FIG. 2, and data shown in Table 2, obtained using CuKα radiation. In a particular embodiment, the polymorph can be characterized by one or more of the peaks taken from FIG. 2, as shown in Table 2. For example, the polymorph can be characterized by one or two or three or four or five or six or seven or eight or nine of the peaks shown in Table 2.

**Table 2**

| **Angle** | **Intensity%** |
|---|---|
| 8.4 | 65.2 |
| 12.7 | 75.5 |
| 16.9 | 57.9 |
| 17.1 | 69.4 |
| 17.7 | 48.6 |
| 19.2 | 100.0 |
| 23.0 | 69.7 |
| 23.3 | 61.1 |
| 24.2 | 87.3 |

In another embodiment, Form 2 can be characterized by the peaks identified at 2θ angles of 12.7, 17.1, 19.2, 23.0, and 24.2°. In another embodiment, Form 2 can be characterized by the peaks identified at 2θ angles of 12.7, 19.2, and 24.2°.

### Form 3 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol

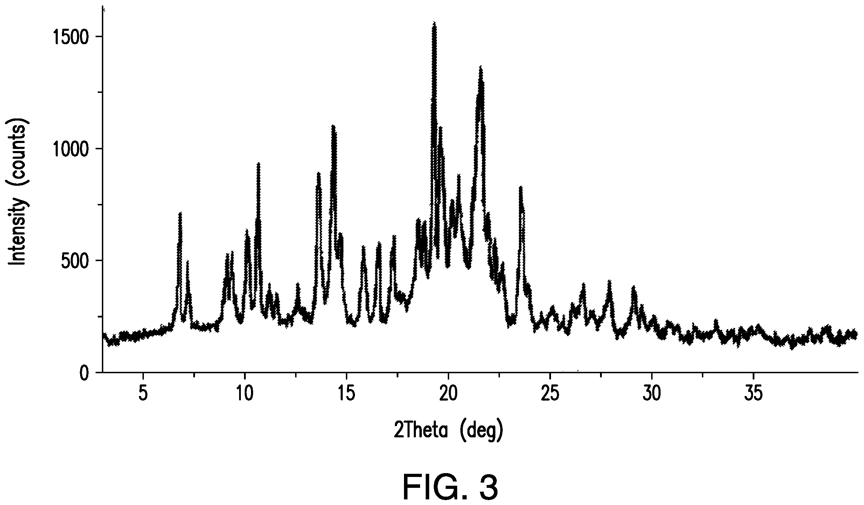

In one embodiment, a single crystalline form, Form 3, of COMPOUND 1 is characterized by the XRPD pattern shown in FIG. 3, and data shown in Table 3, obtained using CuKα radiation. In a particular embodiment, the polymorph can be characterized by one or more of the peaks taken from FIG. 3, as shown in Table 3. For example, the polymorph can be characterized by one or two or three or four or five or six or seven or eight or nine of the peaks shown in Table 3.

**Table 3**

| **Angle** | **Intensity %** |
|---|---|
| 6.8 | 35.5 |
| 10.1 | 30.7 |
| 10.6 | 53.1 |
| 13.6 | 46.0 |
| 14.2 | 63.8 |
| 17.2 | 26.4 |
| 18.4 | 34.0 |
| 19.2 | 100.0 |
| 23.5 | 3.8 |

In another embodiment, Form 3 can be characterized by the peaks identified at 2θ angles of 6.8, 10.6, 13.6, 14.2, and 19.2°. In another embodiment, Form 3 can be characterized by the peaks identified at 2θ angles of 10.6, 14.2, and 19.2°.

### Form 4 of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol

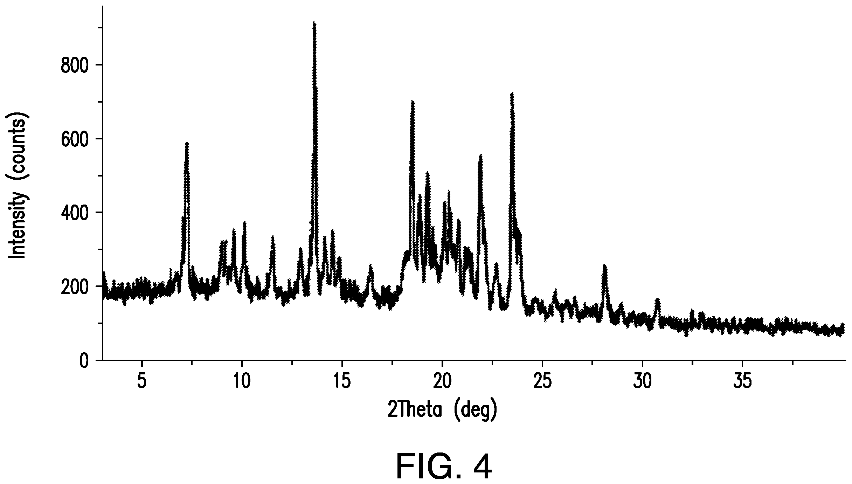

In one embodiment, a single crystalline form, Form 4, of COMPOUND 1 is characterized by the XRPD pattern shown in FIG. 4, and data shown in Table 4, obtained using CuKα radiation. In a particular embodiment, the polymorph can be characterized by one or more of the peaks taken from FIG. 4, as shown in Table 4. For example, the polymorph can be characterized by one or two or three or four or five or six or seven or eight or nine of the peaks shown in Table 4.

**Table 4**

| **Angle** | **Intensity %** |
|---|---|
| 7.2 | 53.3 |
| 10.1 | 26.7 |
| 11.5 | 20.5 |
| 13.6 | 100.0 |
| 18.5 | 72.0 |
| 19.3 | 46.9 |
| 20.3 | 39.4 |
| 21.9 | 55.4 |
| 23.5 | 77.5 |

In another embodiment, Form 4 can be characterized by the peaks identified at 2θ angles of 7.2, 13.6, 18.5, 19.3, 21.9, and 23.5°. In another embodiment, Form 4 can be characterized by the peaks identified at 2θ angles of 13.6, 18.5, and 23.5°.

### Form 5 of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol

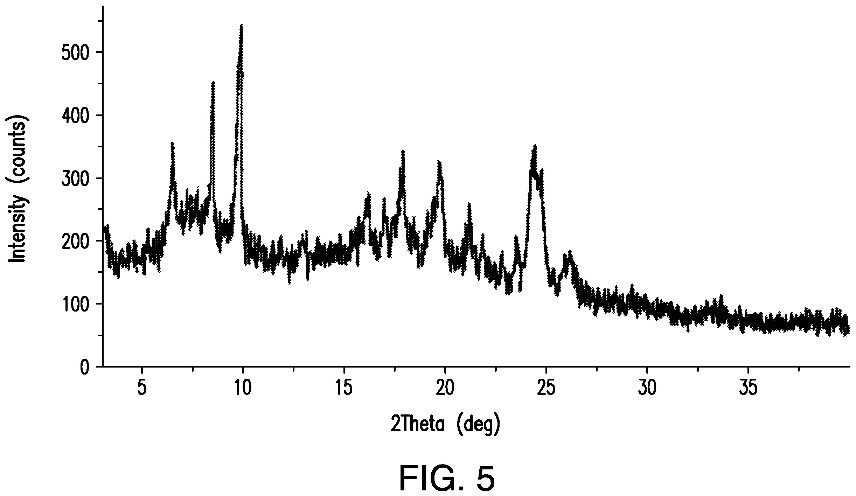

In one embodiment, a single crystalline form, Form 5, of COMPOUND 1 is characterized by the XRPD pattern shown in FIG. 5, and data shown in Table 5, obtained using CuKα radiation. In a particular embodiment, the polymorph can be characterized by one or more of the peaks taken from FIG. 5, as shown in Table 5. For example, the polymorph can be characterized by one or two or three or four or five or six or seven or eight or nine of the peaks shown in Table 5.

**Table 5**

| **Angle** | **Intensity %** |
|---|---|
| 6.4 | 45.4 |
| 8.4 | 84.0 |
| 9.8 | 100.0 |
| 16.1 | 26.0 |
| 16.9 | 22.7 |
| 17.8 | 43.6 |
| 19.7 | 40.4 |
| 21.1 | 20.5 |
| 26.1 | 15.9 |

In another embodiment, Form 5 can be characterized by the peaks identified at 2θ angles of 6.4, 8.4, 9.8, 17.8, and 19.7°. In another embodiment, Form 5 can be characterized by the peaks identified at 2θ angles of 8.4 and 9.8°.

### Form 6 of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol

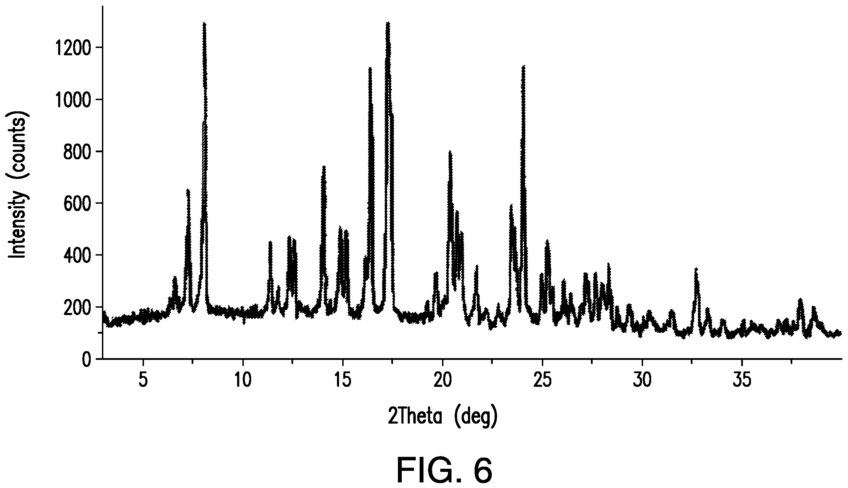

In one embodiment, a single crystalline form, Form 6, of COMPOUND 1 is characterized by the XRPD pattern shown in FIG. 6, and data shown in Table 6, obtained using CuKα radiation. In a particular embodiment, the polymorph can be characterized by one or more of the peaks taken from FIG. 6, as shown in Table 6. For example, the polymorph can be characterized by one or two or three or four or five or six or seven or eight of the peaks shown in Table 6.

**Table 6**

| **Angle** | **Intensity%** |
|---|---|
| 8.1 | 97.9 |
| 11.4 | 24.9 |
| 14.1 | 51.5 |
| 15.2 | 28.4 |
| 16.4 | 85.0 |
| 17.3 | 100.0 |
| 20.5 | 54.7 |
| 24.1 | 88.7 |

In another embodiment, Form 6 can be characterized by the peaks identified at 2θ angles of 8.1, 14.1, 16.4, 17.3, 20.5, and 24.1°. In another embodiment, Form 6 can be characterized by the peaks identified at 2θ angles of 8.1, 16.4, 17.3, and 24.1°.

### Form 3 of mesylate salt of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol (COMPOUND A)

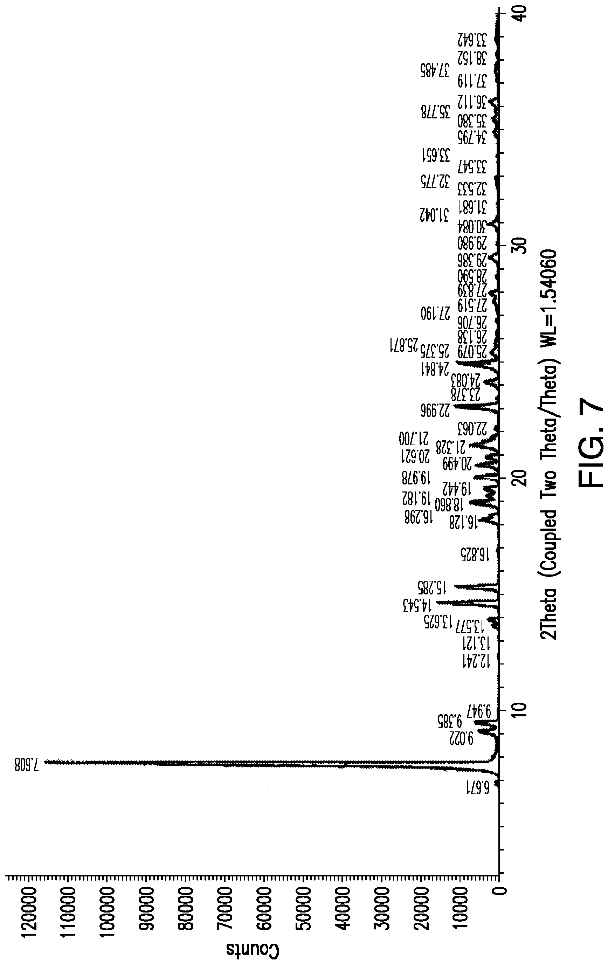

In one embodiment, Form 3 of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-olmethanesulfonate is characterized by the X-ray powder diffraction (XRPD) pattern shown in Figure 7, and data shown in Table A, obtained using CuKa radiation. In a particular embodiment, the polymorph is characterized by one or more of the peaks taken from Figure 7, as shown in Table A. For example, the polymorph is characterized by one or two or three or four or five or six or seven or eight or nine or ten of the peaks shown in Table A.

**Table A**

| **Angle 2-Theta°** | **Intensity** % |
|---|---|
| 7.5 | 100.0 |
| 9.0 | 16.5 |
| 9.3 | 27.2 |
| 14.5 | 48.5 |
| 15.2 | 17.2 |
| 18.0 | 17.0 |
| 18.8 | 32.6 |
| 19.9 | 18.7 |
| 21.3 | 19.3 |
| 24.8 | 33.8 |

In another embodiment, Form 3 is characterized by the peaks identified at 2θ angles of 7.5, 9.3, 14.5, 18.8, 21.3, and 24.8°. In a further embodiment, Form 3 is characterized by the peaks are identified at 2θ angles of 7.5, 14.5, 18.8, and 24.8°. In another, embodiment, Form 3 is characterized by the peaks identified at 2θ angles of 7.5, 14.5, and 24.8°.
Any references to pharmaceutical compositions is a reference to a pharmaceutical composition for use in a method of treating a myelodysplastic syndrome in a subject provided herein.

### Compositions and routes of administration

In one embodiment, provided herein is a pharmaceutical composition comprising a therapeutically effective amount of a mutant IDH2 inhibitor. In one embodiment, the mutant IDH2 inhibitor is COMPOUND 1.

In one embodiment, the compounds utilized in the methods provided herein may be formulated together with a pharmaceutically acceptable carrier or adjuvant into pharmaceutically acceptable compositions prior to be administered to a subject. In another embodiment, such pharmaceutically acceptable compositions further comprise additional therapeutic agents in amounts effective for achieving a modulation of disease or disease symptoms, including those described herein.

Pharmaceutically acceptable carriers, adjuvants and vehicles that may be used in the pharmaceutical compositions of one aspect include, but are not limited to, ion exchangers, alumina, aluminum stearate, lecithin, self-emulsifying drug delivery systems (SEDDS) such as d-α-tocopherol polyethyleneglycol 1000 succinate, surfactants used in pharmaceutical dosage forms such as TWEENs or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as α-, β-, and γ-cyclodextrin, or chemically modified derivatives such as hydroxyalkylcyclodextrins, including 2- and 3-hydroxypropyl-β-cyclodextrins, or other solubilized derivatives may also be advantageously used to enhance delivery of COMPOUND 1 described herein.

In one embodiment, the pharmaceutical composition comprises COMPOUND 1 and an excipient. In one embodiment, the pharmaceutical composition that comprises COMPOUND 1 and an excipient, is for oral administration. In one embodiment, the excipient is a diluent, a binder, a disintegrant, a wetting agent, a stabilizer, a glidant, or a lubricant.

In one embodiment, the diluent is a microcrystalline cellulose.

In one embodiment, the binder is a hydroxypropyl cellulose.

In one embodiment, the disintegrant is sodium starch glycolate.

In one embodiment, the wetting agent is sodium lauryl sulfate.

In one embodiment, the stabilizer is hypromellose acetate succinate.

In one embodiment, the glidant is colloidal silicon dioxide.

In one embodiment, the lubricant is magnesiun stearate.

Oral delivery formats for COMPOUND 1 include, but are not limited to, tablets, capsules, caplets, solutions, suspensions, and syrups, and may also comprise a plurality of granules, beads, powders or pellets that may or may not be encapsulated. Such formats may also be referred to herein as the "drug core" which contains COMPOUND 1.

Particular embodiments herein provide solid oral dosage forms that are tablets or capsules. In certain embodiments, the formulation is a tablet comprising COMPOUND 1. In certain embodiments, the formulation is a capsule comprising COMPOUND 1. In certain embodiments, the tablets or capsules provided herein optionally comprise one or more excipients, such as, for example, glidants, diluents, lubricants, colorants, disintegrants, granulating agents, binding agents, polymers, and coating agents. In certain embodiments, the formulation is an immediate release tablet. In certain embodiments, the formulation is a controlled release tablet releasing the active pharmaceutical ingredient (API), e.g., substantially in the stomach. In certain embodiments, the formulation is a hard gelatin capsule. In certain embodiments, the formulation is a soft gelatin capsule. In certain embodiments, the capsule is a hydroxypropyl methylcellulose (HPMC) capsule. In certain embodiments, the formulation is an immediate release capsule. In certain embodiments, the formulation is an immediate or controlled release capsule releasing the API, e.g., substantially in the stomach. In certain embodiments, the formulation is a rapidly disintegrating tablet that dissolves substantially in the mouth following administration. Certain embodiments provided herein encompass the use of COMPOUND 1 for the preparation of a pharmaceutical composition in a method for treating a myelodysplastic syndrome in a subject, wherein the myelodysplastic syndrome is characterized by the presence of a mutant allele of IDH2 and a mutant allele of a second gene, wherein the second gene is selected from the group consisting of ASXL1 and SRSF2, wherein the composition is prepared for oral administration. In certain embodiments, the myelodysplastic syndrome is characterized by the presence of a mutant allele of IDH2 and the absence of a mutant allele of at least one other gene, wherein the other gene is selected from the group consisting of KRAS, TP53, SETBP1, and U2AF1, wherein the composition is prepared for oral administration. In certain embodiments, the myelodysplastic syndrome characterized by the presence of a mutant allele of IDH2 and a mutant allele of a second gene, wherein the second gene is selected from the group consisting of ASXL1 and SRSF2, and the absence of a mutant allele of at least one other gene, wherein the other gene is selected from the group consisting of KRAS, TP53, SETBP1, and U2AF1, wherein the composition is prepared for oral administration. In certain examples, the myelodysplastic syndrome is characterized by the presence of a mutant allele of IDH2 and a mutant allele of at least one second gene, wherein the second gene is selected from the group consisting of ASXL1, SRSF2, KRAS, DNMT3A, MPL, CSF3R, FAT3, and CBL, wherein the composition is prepared for oral administration. In certain examples, the myelodysplastic syndrome is characterized by the presence of a mutant allele of IDH2 and the absence of a mutant allele of at least one other gene, wherein the other gene is selected from the group consisting of ASXL1, SRSF2, KRAS, TP53, SETBP1, U2AF1, TCF3, STAG2, NRAS, JAK2 and BRAF, wherein the composition is prepared for oral administration. In certain examples, the myelodysplastic syndrome characterized by the presence of a mutant allele of IDH2 and a mutant allele of at least one second gene, wherein the second gene is selected from the group consisting of ASXL1, SRSF2, DNMT3A, MPL, CSF3R, FAT3, and CBL, and the absence of a mutant allele of at least one other gene, wherein the other gene is selected from the group consisting of KRAS, TP53, SETBP1, U2AF1, TCF3, STAG2, NRAS, JAK2 and BRAF, wherein the composition is prepared for oral administration. In certain embodiments the myelodysplastic syndrome is characterized by the presence of a mutant allele of IDH2 and a mutant allele of at least one second gene, wherein the second gene is selected from the group consisting of MPL, DNMT3A, CSF3R, FAT3, or CBL, wherein the composition is prepared for oral administration. In certain examples, the myelodysplastic syndrome is characterized by the presence of a mutant allele of IDH2 and the absence of a mutant allele of at least one other gene, wherein the other gene is selected from the group consisting of TP53, SETBP1, U2AF1, TCF3, STAG2, NRAS, JAK2 and BRAF, wherein the composition is prepared for oral administration.

Particular embodiments herein provide pharmaceutical formulations (e.g., immediate release oral formulations and/or formulations that release the API substantially in the stomach) comprising COMPOUND 1 that achieve a particular AUC value (*e.g.,* AUC(0-t) or AUC(0-∞)) in the subject (e.g., human) to which the formulation is orally administered. Particular embodiments provide oral formulations that achieve an AUC value of at least about 25 ng-hr/mL, at least about 50 ng-hr/mL, at least about 75 ng-hr/mL, at least about 100 ng-hr/mL, at least about 150 ng-hr/mL, at least about 200 ng-hr/mL, at least about 250 ng-hr/mL, at least about 300 ng-hr/mL, at least about 350 ng-hr/mL, at least about 400 ng-hr/mL, at least about 450 ng-hr/mL, at least about 500 ng-hr/mL, at least about 550 ng-hr/mL, at least about 600 ng-hr/mL, at least about 650 ng-hr/mL, at least about 700 ng-hr/mL, at least about 750 ng-hr/mL, at least about 800 ng-hr/mL, at least about 850 ng-hr/mL, at least about 900 ng-hr/mL, at least about 950 ng-hr/mL, at least about 1000 ng-hr/mL, at least about 1100 ng-hr/mL, at least about 1200 ng-hr/mL, at least about 1300 ng-hr/mL, at least about 1400 ng-hr/mL, at least about 1500 ng-hr/mL, at least about 1600 ng-hr/mL, at least about 1700 ng-hr/mL, at least about 1800 ng-hr/mL, at least about 1900 ng-hr/mL, at least about 2000 ng-hr/mL, at least about 2250 ng-hr/mL, or at least about 2500 ng-hr/mL. In particular embodiments, the AUC determination is obtained from a time-concentration pharmacokinetic profile obtained from the blood samples of animals or human volunteers following dosing.

Particular embodiments herein provide pharmaceutical formulations (e.g., immediate release oral formulations and/or formulations that release the API substantially in the stomach) comprising COMPOUND 1 that achieve a particular maximum plasma concentration ("Cmax") in the subject to which the formulation is orally administered. Particular embodiments provide oral formulations that achieve a Cmax of COMPOUND 1 of at least about 25 ng/mL, at least about 50 ng/mL, at least about 75 ng/mL, at least about 100 ng/mL, at least about 150 ng/mL, at least about 200 ng/mL, at least about 250 ng/mL, at least about 300 ng/mL, at least about 350 ng/mL, at least about 400 ng/mL, at least about 450 ng/mL, at least about 500 ng/mL, at least about 550 ng/mL, at least about 600 ng/mL, at least about 650 ng/mL, at least about 700 ng/mL, at least about 750 ng/mL, at least about 800 ng/mL, at least about 850 ng/mL, at least about 900 ng/mL, at least about 950 ng/mL, at least about 1000 ng/mL, at least about 1100 ng/mL, at least about 1200 ng/mL, at least about 1300 ng/mL, at least about 1400 ng/mL, at least about 1500 ng/mL, at least about 1600 ng/mL, at least about 1700 ng/mL, at least about 1800 ng/mL, at least about 1900 ng/mL, at least about 2000 ng/mL, at least about 2250 ng/mL, or at least about 2500 ng/mL.

Particular embodiments herein provide pharmaceutical formulations (e.g., immediate release oral formulations and/or formulations that release the API substantially in the stomach) comprising COMPOUND 1 that achieve a particular time to maximum plasma concentration ("Tmax") in the subject to which the formulation is orally administered. Particular embodiments provide oral formulations that achieve a Tmax of COMPOUND 1 of less than about 10 min., less than about 15 min., less than about 20 min., less than about 25 min., less than about 30 min., less than about 35 min., less than about 40 min., less than about 45 min., less than about 50 min., less than about 55 min., less than about 60 min., less than about 65 min., less than about 70 min., less than about 75 min., less than about 80 min., less than about 85 min., less than about 90 min., less than about 95 min., less than about 100 min., less than about 105 min., less than about 110 min., less than about 115 min., less than about 120 min., less than about 130 min., less than about 140 min., less than about 150 min., less than about 160 min., less than about 170 min., less than about 180 min., less than about 190 min., less than about 200 min., less than about 210 min., less than about 220 min., less than about 230 min., or less than about 240 min. In particular embodiments, the Tmax value is measured from the time at which the formulation is orally administered.

Particular embodiments herein provide oral dosage forms comprising COMPOUND 1 wherein the oral dosage forms have an enteric coating. Particular embodiments provide a permeable or partly permeable (e.g., "leaky") enteric coating with pores. In particular embodiments, the permeable or partly permeable enteric-coated tablet releases COMPOUND 1 in an immediate release manner substantially in the stomach.

Provided herein are dosage forms designed to maximize the absorption and/or efficacious delivery of COMPOUND 1, upon oral administration, *e*.*g*., for release substantially in the stomach. Accordingly, certain embodiments herein provide a solid oral dosage form of COMPOUND 1 using pharmaceutical excipients designed for immediate release of the API upon oral administration, e.g., substantially in the stomach. Particular immediate release formulations comprise a specific amount of COMPOUND 1 and optionally one or more excipients. In certain embodiments, the formulation may be an immediate release tablet or an immediate release capsule (such as, *e.g.,* an HPMC capsule).

Provided herein are methods of making the formulations provided herein comprising COMPOUND 1 provided herein (*e*.*g*., immediate release oral formulations and/or formulations that release the API substantially in the stomach). In particular embodiments, the formulations provided herein may be prepared using conventional methods known to those skilled in the field of pharmaceutical formulation, as described, *e*.*g*., in pertinent textbooks. *See, e.g.,* REMINGTON, THE SCIENCE AND PRACTICE OF PHARMACY, 20th Edition, Lippincott Williams & Wilkins, (2000); ANSEL et al., PHARMACEUTICAL DOSAGE FORMS AND DRUG DELIVERY SYSTEMS, 7th Edition, Lippincott Williams & Wilkins, (1999); GIBSON, PHARMACEUTICAL PREFORMULATION AND FORMULATION, CRC Press (2001).

In particular embodiments, formulations provided herein (*e*.*g*., immediate release oral formulations, formulations that release the API substantially in the stomach, or rapidly disintegrating formulations that dissolve substantially in the mouth) comprise COMPOUND 1 in a specific amount. In particular embodiments, the specific amount of COMPOUND 1 in the formulation is, *e.g.,* about 10 mg. In one embodiment, the specific amount is about 20 mg. In one embodiment, the specific amount is about 40 mg. In one embodiment, the specific amount is about 60 mg. In one embodiment, the specific amount is about 80 mg. In one embodiment, the specific amount is about 100 mg. In one embodiment, the specific amount is about 120 mg. In one embodiment, the specific amount is about 140 mg. In one embodiment, the specific amount is about 150 mg. In one embodiment, the specific amount is about 160 mg. In one embodiment, the specific amount is about 180 mg. In one embodiment, the specific amount is about 200 mg. In one embodiment, the specific amount is about 220 mg. In one embodiment, the specific amount is about 240 mg. In one embodiment, the specific amount is about 260 mg. In one embodiment, the specific amount is about 280 mg. In one embodiment, the specific amount is about 300 mg. In one embodiment, the specific amount is about 320 mg. In one embodiment, the specific amount is about 340 mg. In one embodiment, the specific amount is about 360 mg. In one embodiment, the specific amount is about 380 mg. In one embodiment, the specific amount is about 400 mg. In one embodiment, the specific amount is about 420 mg. In one embodiment, the specific amount is about 440 mg. In one embodiment, the specific amount is about 460 mg. In one embodiment, the specific amount is about 480 mg. In one embodiment, the specific amount is about 500 mg. In one embodiment, the specific amount is about 600 mg. In one embodiment, the specific amount is about 700 mg. In one embodiment, the specific amount is about 800 mg. In one embodiment, the specific amount is about 900 mg. In one embodiment, the specific amount is about 1000 mg. In one embodiment, the specific amount is about 1100 mg. In one embodiment, the specific amount is about 1200 mg. In one embodiment, the specific amount is about 1300 mg. In one embodiment, the specific amount is about 1400 mg. In one embodiment, the specific amount is about 1500 mg. In one embodiment, the specific amount is about 1600 mg. In one embodiment, the specific amount is about 1700 mg. In one embodiment, the specific amount is about 1800 mg. In one embodiment, the specific amount is about 1900 mg. In one embodiment, the specific amount is about 2000 mg. In one embodiment, the specific amount is about 2100 mg. In one embodiment, the specific amount is about 2200 mg. In one embodiment, the specific amount is about 2300 mg. In one embodiment, the specific amount is about 2400 mg. In one embodiment, the specific amount is about 2500 mg. In one embodiment, the specific amount is about 3000 mg. In one embodiment, the specific amount is about 4000 mg. In one embodiment, the specific amount is about 5000 mg.

In certain embodiments, the formulation is a tablet, wherein the tablet is manufactured using standard, art-recognized tablet processing procedures and equipment. In certain embodiments, the method for forming the tablets is direct compression of a powdered, crystalline and/or granular composition comprising COMPOUND 1 alone or in combination with one or more excipients, such as, for example, carriers, additives, polymers, or the like. In certain embodiments, as an alternative to direct compression, the tablets may be prepared using wet granulation or dry granulation processes. In certain embodiments, the tablets are molded rather than compressed, starting with a moist or otherwise tractable material. In certain embodiments, compression and granulation techniques are used.

In certain embodiments, the formulation is a capsule, wherein the capsules may be manufactured using standard, art-recognized capsule processing procedures and equipments. In certain embodiments, soft gelatin capsules may be prepared in which the capsules contain a mixture of COMPOUND 1 and vegetable oil or non-aqueous, water miscible materials such as, for example, polyethylene glycol and the like. In certain embodiments, hard gelatin capsules may be prepared containing granules of COMPOUND 1 in combination with a solid pulverulent carrier, such as, for example, lactose, saccharose, sorbitol, mannitol, potato starch, corn starch, amylopectin, cellulose derivatives, or gelatin. In certain embodiments, a hard gelatin capsule shell may be prepared from a capsule composition comprising gelatin and a small amount of plasticizer such as glycerol. In certain embodiments, as an alternative to gelatin, the capsule shell may be made of a carbohydrate material. In certain embodiments, the capsule composition may additionally include polymers, colorings, flavorings and opacifiers as required. In certain embodiments, the capsule comprises HPMC.

In certain embodiments, the formulation of COMPOUND 1 is prepared using aqueous solvents without causing significant hydrolytic degradation of the compound. In particular embodiments, the formulation of COMPOUND 1 is a tablet which contains a coating applied to the drug core using aqueous solvents without causing significant hydrolytic degradation of the compound in the formulation. In certain embodiments, water is employed as the solvent for coating the drug core. In certain embodiments, the oral dosage form of COMPOUND 1 is a tablet containing a film coat applied to the drug core using aqueous solvents. In particular embodiments, water is employed as the solvent for film-coating. In particular embodiments, the tablet containing COMPOUND 1 is film-coated using aqueous solvents without effecting degradation of the pharmaceutical composition. In particular embodiments, water is used as the film coating solvent without effecting degradation of the pharmaceutical composition. In certain embodiments, an oral dosage form comprising COMPOUND 1 and an aqueous film coating effects immediate drug release upon oral delivery. In certain embodiments, the oral dosage form comprising COMPOUND 1 and an aqueous film coating effects controlled drug release to the upper gastrointestinal tract, *e*.*g*., the stomach, upon oral administration. In particular embodiments, a tablet with an aqueous-based film coating comprises COMPOUND 1 as the API.

In certain embodiments, provided herein is a controlled release pharmaceutical formulation for oral administration of COMPOUND 1, wherein the release occurs substantially in the stomach, comprising: a) a specific amount of COMPOUND 1; b) a drug release controlling component for controlling the release of COMPOUND 1 substantially in the upper gastrointestinal tract, *e*.*g*., the stomach; and c) optionally one or more excipients. In certain embodiments, the oral dosage form comprising COMPOUND 1 is prepared as a controlled release tablet or capsule which includes a drug core comprising the pharmaceutical composition and optional excipients. Optionally, a "seal coat" or "shell" is applied. In certain embodiments, a formulation provided herein comprising COMPOUND 1 provided herein is a controlled release tablet or capsule, which comprises a therapeutically effective amount of COMPOUND 1, a drug release controlling component that controls the release of COMPOUND 1 substantially in the stomach upon oral administration, and optionally, one or more excipients.

Particular embodiments provide a drug release controlling component that is a polymer matrix, which swells upon exposure to gastric fluid to effect the gastric retention of the formulation and the sustained release of COMPOUND 1 from the polymer matrix substantially in the stomach. In certain embodiments, such formulations may be prepared by incorporating COMPOUND 1 into a suitable polymeric matrix during formulation. Examples of such formulations are known in the art. *See, e.g.,* Shell et al., U.S. Patent Publication No. 2002/0051820 (Application No. 09/990,061); Shell et al., U.S. Patent Publication No. 2003/0039688 (Application No. 10/045,823); Gusler et al., U.S. Patent Publication No. 2003/0104053 (Application No. 10/029,134).

In certain embodiments, the drug release controlling component may comprise a shell surrounding the drug-containing core, wherein the shell releases COMPOUND 1 from the core by, *e*.*g*., permitting diffusion of COMPOUND 1 from the core and promoting gastric retention of the formulation by swelling upon exposure to gastric fluids to a size that is retained in the stomach. In certain embodiments, such formulations may be prepared by first compressing a mixture of COMPOUND 1 and one or more excipients to form a drug core, and compressing another powdered mixture over the drug core to form the shell, or enclosing the drug core with a capsule shell made of suitable materials. Examples of such formulations are known in the art. *See, e.g.,* Berner et al., U.S. Patent Publication No. 2003/0104062 Application No. 10/213,823).

In certain embodiments, the pharmaceutical formulations provided herein contain COMPOUND 1 and, optionally, one or more excipients to form a "drug core." Optional excipients include, *e*.*g*., diluents (bulking agents), lubricants, disintegrants, fillers, stabilizers, surfactants, preservatives, coloring agents, flavoring agents, binding agents, excipient supports, glidants, permeation enhancement excipients, plasticizers and the like, *e.g.,* as known in the art. It will be understood by those in the art that some substances serve more than one purpose in a pharmaceutical composition. For instance, some substances are binders that help hold a tablet together after compression, yet are also disintegrants that help break the tablet apart once it reaches the target delivery site. Selection of excipients and amounts to use may be readily determined by the formulation scientist based upon experience and consideration of standard procedures and reference works available in the art.

In certain embodiments, formulations provided herein comprise one or more binders. Binders may be used, *e*.*g*., to impart cohesive qualities to a tablet, and thus ensure that the tablet remains intact after compression. Suitable binders include, but are not limited to, starch (including corn starch and pregelatinized starch), gelatin, sugars (including sucrose, glucose, dextrose and lactose), polyethylene glycol, propylene glycol, waxes, and natural and synthetic gums, *e*.*g*., acacia sodium alginate, polyvinylpyrrolidone, cellulosic polymers (including hydroxypropyl cellulose, hydroxypropylmethylcellulose, methyl cellulose, ethyl cellulose, hydroxyethyl cellulose, carboxymethyl cellulose and the like), veegum, carbomer (*e*.*g*., carbopol), sodium, dextrin, guar gum, hydrogenated vegetable oil, magnesium aluminum silicate, maltodextrin, polymethacrylates, povidone (*e*.*g*., KOLLIDON, PLASDONE), microcrystalline cellulose, among others. Binding agents also include, *e*.*g*., acacia, agar, alginic acid, cabomers, carrageenan, cellulose acetate phthalate, ceratonia, chitosan, confectioner's sugar, copovidone, dextrates, dextrin, dextrose, ethylcellulose, gelatin, glyceryl behenate, guar gum, hydroxyethyl cellulose, hydroxyethylmethyl cellulose, hydroxypropyl cellulose, hydroxypropyl starch, hypromellose, inulin, lactose, magnesium aluminum silicate, maltodextrin, maltose, methylcellulose, poloxamer, polycarbophil, polydextrose, polyethylene oxide, polymethylacrylates, povidone, sodium alginate, sodium carboxymethylcellulose, starch, pregelatinized starch, stearic acid, sucrose, and zein. The binding agent can be, relative to the drug core, in the amount of about 2% w/w of the drug core; about 4% w/w of the drug core, about 6% w/w of the drug core, about 8% w/w of the drug core, about 10% w/w of the drug core, about 12% w/w of the drug core, about 14% w/w of the drug core, about 16% w/w of the drug core, about 18% w/w of the drug core, about 20% w/w of the drug core, about 22% w/w of the drug core, about 24% w/w of the drug core, about 26% w/w of the drug core, about 28% w/w of the drug core, about 30% w/w of the drug core, about 32% w/w of the drug core, about 34% w/w of the drug core, about 36% w/w of the drug core, about 38% w/w of the drug core, about 40% w/w of the drug core, about 42% w/w of the drug core, about 44% w/w of the drug core, about 46% w/w of the drug core, about 48% w/w of the drug core, about 50% w/w of the drug core, about 52% w/w of the drug core, about 54% w/w of the drug core, about 56% w/w of the drug core, about 58% w/w of the drug core, about 60% w/w of the drug core, about 62% w/w of the drug core, about 64% w/w of the drug core, about 66% w/w of the drug core; about 68% w/w of the drug core, about 70% w/w of the drug core, about 72% w/w of the drug core, about 74% w/w of the drug core, about 76% w/w of the drug core, about 78% w/w of the drug core, about 80% w/w of the drug core, about 82% w/w of the drug core, about 84% w/w of the drug core, about 86% w/w of the drug core, about 88% w/w of the drug core, about 90% w/w of the drug core, about 92% w/w of the drug core, about 94% w/w of the drug core, about 96% w/w of the drug core, about 98% w/w of the drug core, or more, if determined to be appropriate. In certain embodiments, a suitable amount of a particular binder is determined by one of ordinary skill in the art.

In certain embodiments, formulations provided herein comprise one or more diluents. Diluents may be used, *e*.*g*., to increase bulk so that a practical size tablet is ultimately provided. Suitable diluents include dicalcium phosphate, calcium sulfate, lactose, cellulose, kaolin, mannitol, sodium chloride, dry starch, microcrystalline cellulose (*e*.*g*., AVICEL), microfine cellulose, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (*e*.*g*., EUDRAGIT), potassium chloride, sodium chloride, sorbitol and talc, among others. Diluents also include, *e*.*g*., ammonium alginate, calcium carbonate, calcium phosphate, calcium sulfate, cellulose acetate, compressible sugar, confectioner's sugar, dextrates, dextrin, dextrose, erythritol, ethylcellulose, fructose, fumaric acid, glyceryl palmitostearate, isomalt, kaolin, lacitol, lactose, mannitol, magnesium carbonate, magnesium oxide, maltodextrin, maltose, medium-chain triglycerides, microcrystalline cellulose, microcrystalline silicified cellulose, powered cellulose, polydextrose, polymethylacrylates, simethicone, sodium alginate, sodium chloride, sorbitol, starch, pregelatinized starch, sucrose, sulfobutylether-β-cyclodextrin, talc, tragacanth, trehalose, and xylitol. Diluents may be used in amounts calculated to obtain a desired volume for a tablet or capsule; in certain embodiments, a diluent is used in an amount of about 5% or more, about 10% or more, about 15% or more, about 20% or more, about 22% or more, about 24% or more, about 26% or more, about 28% or more, about 30% or more, about 32% or more, about 34% or more, about 36% or more, about 38% or more, about 40% or more, about 42% or more, about 44% or more, about 46% or more, about 48% or more, about 50% or more, about 52% or more, about 54% or more, about 56% or more, about 58% or more, about 60% or more, about 62% or more, about 64% or more, about 68% or more, about 70% ore more, about 72% or more, about 74% or more, about 76% or more, about 78% or more, about 80% or more, about 85% or more, about 90% or more, or about 95% or more, weight/weight, of a drug core; between about 10% and about 90% w/w of the drug core; between about 20% and about 80% w/w of the drug core; between about 30% and about 70% w/w of the drug core; between about 40% and about 60% w/w of the drug core. In certain embodiments, a suitable amount of a particular diluent is determined by one of ordinary skill in the art.

In certain embodiments, formulations provided herein comprise one or more lubricants. Lubricants may be used, e.g., to facilitate tablet manufacture; examples of suitable lubricants include, for example, vegetable oils such as peanut oil, cottonseed oil, sesame oil, olive oil, corn oil, and oil of theobroma, glycerin, magnesium stearate, calcium stearate, and stearic acid. In certain embodiments, stearates, if present, represent no more than approximately 2 weight % of the drug-containing core. Further examples of lubricants include, *e*.*g*., calcium stearate, glycerin monostearate, glyceryl behenate, glyceryl palmitostearate, magnesium lauryl sulfate, magnesium stearate, myristic acid, palmitic acid, poloxamer, polyethylene glycol, potassium benzoate, sodium benzoate, sodium chloride, sodium lauryl sulfate, sodium stearyl fumarate, stearic acid, talc, and zinc stearate. In particular embodiments, the lubricant is magnesium stearate. In certain embodiments, the lubricant is present, relative to the drug core, in an amount of about 0.2% w/w of the drug core, about 0.4% w/w of the drug core, about 0.6% w/w of the drug core, about 0.8% w/w of the drug core, about 1.0% w/w of the drug core, about 1.2% w/w of the drug core, about 1.4% w/w of the drug core, about 1.6% w/w of the drug core, about 1.8% w/w of the drug core, about 2.0% w/w of the drug core, about 2.2% w/w of the drug core, about 2.4% w/w of the drug core, about 2.6% w/w of the drug core, about 2.8% w/w of the drug core, about 3.0% w/w of the drug core, about 3.5% w/w of the drug core, about 4% w/w of the drug core, about 4.5% w/w of the drug core, about 5% w/w of the drug core, about 6% w/w of the drug core, about 7% w/w of the drug core, about 8% w/w of the drug core, about 10% w/w of the drug core, about 12% w/w of the drug core, about 14% w/w of the drug core, about 16% w/w of the drug core, about 18% w/w of the drug core, about 20% w/w of the drug core, about 25% w/w of the drug core, about 30% w/w of the drug core, about 35% w/w of the drug core, about 40% w/w of the drug core, between about 0.2% and about 10% w/w of the drug core, between about 0.5% and about 5% w/w of the drug core, or between about 1% and about 3% w/w of the drug core. In certain embodiments, a suitable amount of a particular lubricant is determined by one of ordinary skill in the art.

In certain embodiments, formulations provided herein comprise one or more disintegrants. Disintegrants may be used, *e*.*g*., to facilitate disintegration of the tablet, and may be, *e*.*g*., starches, clays, celluloses, algins, gums or crosslinked polymers. Disintegrants also include, *e*.*g*., alginic acid, carboxymethylcellulose calcium, carboxymethylcellulose sodium (*e*.*g*., AC-DI-SOL, PRIMELLOSE), colloidal silicon dioxide, croscarmellose sodium, crospovidone (*e*.*g*., KOLLIDON, POLYPLASDONE), guar gum, magnesium aluminum silicate, methyl cellulose, microcrystalline cellulose, polacrilin potassium, powdered cellulose, pregelatinized starch, sodium alginate, sodium starch glycolate (*e*.*g*., EXPLOTAB) and starch. Additional disintegrants include, *e*.*g*., calcium alginate, chitosan, sodium docusate, hydroxypropyl cellulose, and povidone. In certain embodiments, the disintegrant is, relative to the drug core, present in the amount of about 1% w/w of the drug core, about 2% w/w of the drug core, about 3% w/w of the drug core, about 4% w/w of the drug core, about 5% w/w of the drug core, about 6% w/w of the drug core, about 7% w/w of the drug core, about 8% w/w of the drug core, about 9% w/w of the drug core, about 10% w/w of the drug core, about 12% w/w of the drug core, about 14% w/w of the drug core, about 16% w/w of the drug core, about 18% w/w of the drug core, about 20% w/w of the drug core, about 22% w/w of the drug core, about 24% w/w of the drug core, about 26% w/w of the drug core, about 28% w/w of the drug core, about 30% w/w of the drug core, about 32% w/w of the drug core, greater than about 32% w/w of the drug core, between about 1% and about 10% w/w of the drug core, between about 2% and about 8% w/w of the drug core, between about 3% and about 7% w/w of the drug core, or between about 4% and about 6% w/w of the drug core. In certain embodiments, a suitable amount of a particular disintegrant is determined by one of ordinary skill in the art.

In certain embodiments, formulations provided herein comprise one or more stabilizers. Stabilizers (also called absorption enhancers) may be used, *e*.*g*., to inhibit or retard drug decomposition reactions that include, by way of example, oxidative reactions. Stabilizing agents include, *e*.*g*., d-alpha-tocopheryl polyethylene glycol 1000 succinate (Vitamin E TPGS), acacia, albumin, alginic acid, aluminum stearate, ammonium alginate, ascorbic acid, ascorbyl palmitate, bentonite, butylated hydroxytoluene, calcium alginate, calcium stearate, calcium carboxymethylcellulose, carrageenan, ceratonia, colloidal silicon dioxide, cyclodextrins, diethanolamine, edetates, ethylcellulose, ethyleneglycol palmitostearate, glycerin monostearate, guar gum, hydroxypropyl cellulose, hypromellose, invert sugar, lecithin, magnesium aluminum silicate, monoethanolamine, pectin, poloxamer, polyvinyl alcohol, potassium alginate, potassium polacrilin, povidone, propyl gallate, propylene glycol, propylene glycol alginate, raffinose, sodium acetate, sodium alginate, sodium borate, sodium carboxymethyl cellulose, sodium stearyl fumarate, sorbitol, stearyl alcohol, sufobutyl-b-cyclodextrin, trehalose, white wax, xanthan gum, xylitol, yellow wax, and zinc acetate. In certain embodiments, the stabilizer is, relative to the drug core, present in the amount of about 1% w/w of the drug core, about 2% w/w of the drug core, about 3% w/w of the drug core, about 4% w/w of the drug core, about 5% w/w of the drug core, about 6% w/w of the drug core, about 7% w/w of the drug core, about 8% w/w of the drug core, about 9% w/w of the drug core, about 10% w/w of the drug core, about 12% w/w of the drug core, about 14% w/w of the drug core, about 16% w/w of the drug core, about 18% w/w of the drug core, about 20% w/w of the drug core, about 22% w/w of the drug core, about 24% w/w of the drug core, about 26% w/w of the drug core, about 28% w/w of the drug core, about 30% w/w of the drug core, about 32% w/w of the drug core, between about 1% and about 10% w/w of the drug core, between about 2% and about 8% w/w of the drug core, between about 3% and about 7% w/w of the drug core, or between about 4% and about 6% w/w of the drug core. In certain embodiments, a suitable amount of a particular stabilizer is determined by one of ordinary skill in the art.

In certain embodiments, formulations provided herein comprise one or more glidants. Glidants may be used, *e*.*g*., to improve the flow properties of a powder composition or granulate or to improve the accuracy of dosing. Excipients that may function as glidants include, *e*.*g*., colloidal silicon dioxide, magnesium trisilicate, powdered cellulose, starch, tribasic calcium phosphate, calcium silicate, powdered cellulose, colloidal silicon dioxide, magnesium silicate, magnesium trisilicate, silicon dioxide, starch, tribasic calcium phosphate, and talc. In certain embodiments, the glidant is, relative to the drug core, present in the amount of less than about 1% w/w of the drug core, about 1% w/w of the drug core, about 2% w/w of the drug core, about 3% w/w of the drug core, about 4% w/w of the drug core, about 5% w/w of the drug core, about 6% w/w of the drug core, about 7% w/w of the drug core, about 8% w/w of the drug core, about 9% w/w of the drug core, about 10% w/w of the drug core, about 12% w/w of the drug core, about 14% w/w of the drug core, about 16% w/w of the drug core, about 18% w/w of the drug core, about 20% w/w of the drug core, about 22% w/w of the drug core, about 24% w/w of the drug core, about 26% w/w of the drug core, about 28% w/w of the drug core, about 30% w/w of the drug core, about 32% w/w of the drug core, between about 1% and about 10% w/w of the drug core, between about 2% and about 8% w/w of the drug core, between about 3% and about 7% w/w of the drug core, or between about 4% and about 6% w/w of the drug core. In certain embodiments, a suitable amount of a particular glidant is determined by one of ordinary skill in the art.

In certain embodiments, the formulations for use in the methods provided herein are tablets comprising about 20-30 % COMPOUND 1; intra-granular excipients comprising about 30-45% microcrystalline cellulose, about 2% hydroxypropyl cellulose, about 6% sodium starch glycolate, about 1% sodium lauryl sulfate, about 1% hypromellose acetate succinate, about 1.5% colloidal silicon dioxide, and about 0.75% magnesium stearate; and extra-granular excipients comprising about 5-50% microcrystalline cellulose, about 2% sodium starch glycolate, about 0.5% colloidal silicon dioxide, and about 0.75% magnesium stearate, all based on total weight of the tablet. In one embodiment, COMPOUND 1 is COMPOUND A. In one embodiment, COMPOUND 1 is polymorph Form 3 of COMPOUND A. In one embodiment, the tablet for use in the methods provided herein is film coated tablet.

In certain embodiments, the formulations for use in the methods provided herein are tablets comprising about 20-30 % COMPOUND 1; intra-granular excipients comprising about 30-45% microcrystalline cellulose, about 2% hydroxypropyl cellulose, about 6% sodium starch glycolate, about 1% sodium lauryl sulfate, about 1% hypromellose acetate succinate, about 1.5% colloidal silicon dioxide, and about 0.75% magnesium stearate; and extra-granular excipients comprising about 9-25% microcrystalline cellulose, about 2% sodium starch glycolate, about 0.5% colloidal silicon dioxide, and about 0.75% magnesium stearate, all based on total weight of the tablet. In one embodiment, COMPOUND 1 is COMPOUND A. In one embodiment, Compound 1 is polymorph Form 3 of COMPOUND A. In one embodiment, the tablet for use in the methods provided herein is film coated tablet.

In certain embodiments, the formulations for use in the methods provided herein are tablets comprising COMPOUND 1 in an amount from about 20% to about 30%, an intragranular excipient selected from about 34.5%, 44.5% and to about 39.5% microcrystalline cellulose, about 2% hydroxypropyl cellulose by and 6%, sodium starch glycolate, and an extragranular excipient selected from about 20 % microcrystalline cellulose and about 2%, sodium starch glycolate by weight based on total weight of the tablet. In one embodiment, Compound 1 is COMPOUND A. In one embodiment, COMPOUND 1 is polymorph Form 3 of COMPOUND A. In one embodiment, the tablet for use in the methods provided herein is film coated tablet.

In certain embodiments, the formulations for use in the methods provided herein are tablets comprising about 30 % COMPOUND 1; intra-granular excipients comprising about 45% microcrystalline cellulose, about 2% hydroxypropyl cellulose, about 6% sodium starch glycolate, about 1% sodium lauryl sulfate, about 1% hypromellose acetate succinate, about 1.5% colloidal silicon dioxide, and about 0.75% magnesium stearate; and extra-granular excipients comprising about 9.5% microcrystalline cellulose, about 2% sodium starch glycolate, about 0.5% colloidal silicon dioxide, and about 0.75% magnesium stearate. In one embodiment, COMPOUND 1 is COMPOUND A. In one embodiment, COMPOUND 1 is polymorph Form 3 of COMPOUND A. In one embodiment, the tablet for use in the methods provided herein is film coated tablet.

In certain embodiments, the formulations for use in the methods provided herein are tablets comprising about 30 % COMPOUND 1; intra-granular excipients comprising about 34.50% microcrystalline cellulose, about 2% hydroxypropyl cellulose, about 6% sodium starch glycolate, about 1% sodium lauryl sulfate, about 1% hypromellose acetate succinate, about 1.5% colloidal silicon dioxide, and about 0.75% magnesium stearate; and extra-granular excipients comprising about 20% microcrystalline cellulose, about 2% sodium starch glycolate, about 0.5% colloidal silicon dioxide, and about 0.75% magnesium stearate. In one embodiment, COMPOUND 1 is COMPOUND A. In one embodiment, COMPOUND 1 is polymorph Form 3 of COMPOUND A. In one embodiment, the tablet for use in the methods provided herein is film coated tablet.

In certain embodiments, the formulations for use in the methods provided herein are tablets comprising about 20 % COMPOUND 1; intra-granular excipients comprising about 44.50% microcrystalline cellulose, about 2% hydroxypropyl cellulose, about 6% sodium starch glycolate, about 1% sodium lauryl sulfate, about 1% hypromellose acetate succinate, about 1.5% colloidal silicon dioxide, and about 0.75% magnesium stearate; and extra-granular excipients comprising about 20% microcrystalline cellulose, about 2% sodium starch glycolate, about 0.5% colloidal silicon dioxide, and about 0.75% magnesium stearate. In one embodiment, COMPOUND 1 is COMPOUND A. In one embodiment, COMPOUND 1 is polymorph Form 3 In one embodiment, the tablet for use in the methods provided herein is film coated tablet.

In certain embodiments, the formulations for use in the methods provided herein are tablets comprising about 25 % COMPOUND 1; intra-granular excipients comprising about 39.50% microcrystalline cellulose, about 2% hydroxypropyl cellulose, about 6% sodium starch glycolate, about 1% sodium lauryl sulfate, about 1% hypromellose acetate succinate, about 1.5% colloidal silicon dioxide, and about 0.75% magnesium stearate; and extra-granular excipients comprising about 20% microcrystalline cellulose, about 2% sodium starch glycolate, about 0.5% colloidal silicon dioxide, and about 0.75% magnesium stearate. In one embodiment, COMPOUND 1 is COMPOUND A. In one embodiment, COMPOUND 1 is polymorph Form 3 of COMPOUND A. In one embodiment, the tablet for use in the methods provided herein is film coated tablet.

In certain embodiments, the formulations for use in the methods provided herein are tablets comprising COMPOUND 1, colloidal silicon dioxide, hydroxypropyl cellulose, hypromellose acetate succinate, iron oxide yellow, magnesium stearate, microcrystalline cellulose, polyethylene glycol, polyvinyl alcohol, sodium lauryl sulfate, sodium starch glycolate, talc, and titanium dioxide. In one embodiment, COMPOUND 1 is COMPOUND A. In one embodiment, Compound 1 is polymorph Form 3 of COMPOUND A. In one embodiment, the tablet for use in the methods provided herein is film coated tablet.

In certain embodiments, the pharmaceutical compositions provided herein may be administered orally, parenterally, by inhalation spray, topically, rectally, nasally, buccally, vaginally or via an implanted reservoir, preferably by oral administration or administration by injection. In one embodiment, the pharmaceutical compositions may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intraarticular, intraarterial, intrasynovial, intrasternal, intrathecal, intralesional and intracranial injection or infusion techniques.

In certain embodiments, the pharmaceutical compositions provided herein may be in the form of a sterile injectable preparation, for example, as a sterile injectable aqueous or oleaginous suspension. This suspension may be formulated according to techniques known in the art using suitable dispersing or wetting agents (such as, for example, TWEEN 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically acceptable dosage forms such as emulsions and or suspensions. Other commonly used surfactants such as TWEENs or SPANs and/or other similar emulsifying agents or bioavailability enhancers which are commonly used in the manufacture of pharmaceutically acceptable solid, liquid, or other dosage forms may also be used for the purposes of formulation.

In certain embodiments, the pharmaceutical compositions provided herein may also be administered in the form of suppositories for rectal administration. These compositions can be prepared by mixing COMPOUND 1 with a suitable non-irritating excipient which is solid at room temperature but liquid at the rectal temperature and therefore will melt in the rectum to release the active components. Such materials include, but are not limited to, cocoa butter, beeswax and polyethylene glycols.

Topical administration of the pharmaceutical compositions provided herein is useful when the desired treatment involves areas or organs readily accessible by topical application. For application topically to the skin, the pharmaceutical composition should be formulated with a suitable ointment containing the active components suspended or dissolved in a carrier. In certain embodiments, carriers for topical administration of the compounds provided herein include, but are not limited to, mineral oil, liquid petroleum, white petroleum, propylene glycol, polyoxyethylene polyoxypropylene compound, emulsifying wax and water. Alternatively, the pharmaceutical composition can be formulated with a suitable lotion or cream containing the active compound suspended or dissolved in a carrier with suitable emulsifying agents. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions provided herein may also be topically applied to the lower intestinal tract by rectal suppository formulation or in a suitable enema formulation. Topically-transdermal patches are also included herein.

In certain embodiments, the pharmaceutical compositions provided herein may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

In certain embodiments, the compositions provided herein can, for example, be administered by injection, intravenously, intraarterially, subdermally, intraperitoneally, intramuscularly, or subcutaneously; or orally, buccally, nasally, transmucosally, topically, in an ophthalmic preparation, or by inhalation, with a dosage ranging from about 0.5 to about 100 mg/kg of body weight, alternatively dosages between 1 mg and 1000 mg/dose, every 4 to 120 hours, or according to the requirements of the particular drug. The methods herein contemplate administration of an effective amount of compound or compound composition to achieve the desired or stated effect. In one embodiment, the pharmaceutical compositions are administered from about 1 to about 6 times per day or alternatively, as a continuous infusion. Such administration can be used as a chronic or acute therapy. The amount of active ingredient that may be combined with the carrier materials to produce a single dosage form varies depending upon the host treated and the particular mode of administration. A typical preparation contains from about 5% to about 95% active compound (w/w). Alternatively, such preparations contain from about 20% to about 80% active compound.

Lower or higher doses than those recited above may be required. Specific dosage and treatment regimens for any particular subject depends upon a variety of factors, including the activity of the specific compound employed, the age, body weight, general health status, sex, diet, time of administration, rate of excretion, drug combination, the severity and course of the disease, condition or symptoms, the subject's disposition to the disease, condition or symptoms, and the judgment of the treating physician.

Upon improvement of a subject's condition, a maintenance dose of a compound, composition or combination provided herein may be administered, if necessary. Subsequently, the dosage or frequency of administration, or both, may be reduced, as a function of the symptoms, to a level at which the improved condition is retained when the symptoms have been alleviated to the desired level. Subjects may, however, require intermittent treatment on a long-term basis upon any recurrence of disease symptoms.

### COMPOUND 1 for use in methods of treatment

COMPOUND 1 can be used in the treatment of a myelodysplastic syndrome in a subject as provided herein.

It has been observed that the mutation status of a second gene in addition to IDH2, wherein the second gene is selected from the group consisting of ASXL1 and SRSF2, is associated with responses in a myelodysplastic syndrome characterized by the presence of a mutant allele of IDH2 when treated with COMPOUND 1. While not intending to be bound by any particular theory of operation, mutations of a second gene, wherein the second gene is selected from the group consisting of ASXL1 and SRSF2, may be associated with positive clinical outcomes in the treatment with COMPOUND 1 of MDS characterized by the presence of a mutant allele of IDH2.

It has been further observed that the mutation status of at least one other gene in addition to IDH2, wherein the other gene is selected from the group consisting of KRAS, TP53, SETBP1, and U2AF1, is associated with responses in a myelodysplastic syndrome characterized by the presence of a mutant allele of IDH2 when treated with COMPOUND 1. While not intending to be bound by any particular theory of operation, the presence of mutation of at least one other gene, wherein the other gene is selected from the group consisting of KRAS, TP53, SETBP1, and U2AF1, may be associated with resistance to treatment with COMPOUND 1 of MDS characterized by the presence of a mutant allele of IDH2.

It has been observed that the mutation status of ASXL1 is associated with responses in a myelodysplastic syndrome characterized by the presence of a mutant allele of IDH2 when treated with COMPOUND 1. It has been observed that the mutation status of SRSF2 is associated with responses in a myelodysplastic syndrome characterized by the presence of a mutant allele of IDH2 when treated with COMPOUND 1. While not intending to be bound by any particular theory of operation, mutations of ASXL1 and/or SRSF2 may be associated with positive clinical outcomes to the treatment with COMPOUND 1 in MDS characterized by the presence of a mutant allele of IDH2.

It has been observed that the mutation status of at least one second gene in addition to IDH2, wherein the second gene is selected from the group consisting of ASXL1, SRSF2, KRAS, DNMT3A, MPL, CSF3R, FAT3, and CBL, is associated with responses in a myelodysplastic syndrome characterized by the presence of a mutant allele of IDH2 when treated with COMPOUND 1. While not intending to be bound by any particular theory of operation, mutations of at least one second gene, wherein the second gene is selected from the group consisting of ASXL1, SRSF2, KRAS, DNMT3A, MPL, CSF3R, FAT3, and CBL, may be associated with positive clinical outcomes in the treatment with COMPOUND 1 of MDS characterized by the presence of a mutant allele of IDH2.

It has been further observed that the mutation status of at least one other gene in addition to IDH2, wherein the other gene is selected from the group consisting of ASXL1, SRSF2, KRAS, TP53, SETBP1, U2AF1, TCF3, STAG2, NRAS, JAK2 and BRAF is associated with responses in a myelodysplastic syndrome characterized by the presence of a mutant allele of IDH2 when treated with COMPOUND 1. While not intending to be bound by any particular theory of operation, the presence of mutation of at least one other gene, wherein the other gene is selected from the group consisting of ASXL1, SRSF2, KRAS, TP53, SETBP1, U2AF1, TCF3, STAG2, NRAS, JAK2 and BRAF, may be associated with resistance to treatment with COMPOUND 1 of MDS characterized by the presence of a mutant allele of IDH2.

It has been observed that the mutation status of at least one second gene in addition to IDH2, wherein the second gene is selected from the group consisting of MPL, DNMT3A, CSF3R, FAT3, or CBL, is associated with responses in a myelodysplastic syndrome characterized by the presence of a mutant allele of IDH2 when treated with COMPOUND 1. While not intending to be bound by any particular theory of operation, mutations of at least one second gene, wherein the second gene is selected from the group consisting of MPL, DNMT3A, CSF3R, FAT3, or CBL, may be associated with positive clinical outcomes in the treatment with COMPOUND 1 of MDS characterized by the presence of a mutant allele of IDH2.

It has been further observed that the mutation status of at least one other gene in addition to IDH2, wherein the other gene is selected from the group consisting of TP53, SETBP1, U2AF1, TCF3, STAG2, NRAS, JAK2 and BRAF is associated with responses in a myelodysplastic syndrome characterized by the presence of a mutant allele of IDH2 when treated with COMPOUND 1. While not intending to be bound by any particular theory of operation, the presence of mutation of at least one other gene, wherein the other gene is selected from the group consisting of TP53, SETBP1, U2AF1, TCF3, STAG2, NRAS, JAK2 and BRAF, may be associated with resistance to treatment with COMPOUND 1 of MDS characterized by the presence of a mutant allele of IDH2.

Specifically, COMPOUND 1 can be used in treating a myelodysplastic syndrome in a subject as provided in the following embodiments.

In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of SRSF2, KRAS and MPL.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is DNMT3A.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1, SRSF2, and CSF3R.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1, SRSF2, MPL, FAT3 and CBL.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of TP53, SETBP1, U2AF1, TCF3, STAG2, NRAS, JAK2 and BRAF.

In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2; wherein the myelodysplastic syndrome is further characterized by the presence of one or more mutant alleles of one or more additional genes.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the presence of one or more mutant alleles of one or more additional genes selected from MPL, CSF3R, FAT3, and CBL.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the presence of one or more mutant alleles of CSF3R.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the presence of one or more mutant alleles of one or more additional genes selected from MPL, FAT3, and CBL.

In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and at least one mutant allele of a second gene, wherein the second gene is selected from the group consisting of ASXL1, SRSF2 and MPL; wherein the myelodysplastic syndrome is further characterized by the presence of one or more mutant alleles of one or more additional genes selected from FAT3, and CBL.

In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1. In certain examples, the myelodysplastic syndrome is further characterized by the presence of one or more mutant alleles of one or more additional genes.

In certain examples, the myelodysplastic syndrome is further characterized by the presence of one or more mutant alleles of one or more additional genes selected from CSF3R, FAT3, and CBL.

In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2. In certain examples, the myelodysplastic syndrome is further characterized by the presence of one or more mutant alleles of one or more additional genes.

In certain embodiments, the myelodysplastic syndrome is further characterized by the presence of one or more mutant alleles of one or more additional genes selected from MPL, CSF3R, FAT3, and CBL.

In certain embodiments, the second gene is MPL. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of MPL. In certain embodiments, the myelodysplastic syndrome is further characterized by the presence of one or more mutant alleles of one or more additional genes selected from ASXL1, SRSF2, FAT3, and CBL.

In certain embodiments, the second gene is DNMT3A. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of DNMT3A.

In certain examples, the myelodysplastic syndrome is characterized by the presence of mutant alleles of ASXL1 and SRSF2. In certain embodiments, provided herein is compound 1 for use in a compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2. In certain examples, the myelodysplastic syndrome is further characterized by the presence of one or more mutant alleles of one or more additional genes.

In certain examples, the myelodysplastic syndrome is characterized by the absence of a mutant allele of at least one other gene, wherein the other gene is selected from the group consisting of KRAS, TP53, SETBP1, and U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of at least one other gene, wherein the other gene is selected from the group consisting of KRAS, TP53, SETBP1, and U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2, and the absence of a mutant allele of at least one other gene, wherein the other gene is selected from the group consisting of KRAS, TP53, SETBP1, and U2AF1. In certain examples, the myelodysplastic syndrome is further characterized by the presence of one or more mutant alleles of one or more additional genes. In certain examples, the myelodysplastic syndrome is characterized by the presence of a mutant allele of ASXL1. In certain embodiments, the second gene is ASXL1. In certain embodiments, provided herein is compound 1 for use in a method of treating a myelodysplastic syndrome in a subject comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is characterized by the presence of a mutant allele of SRSF2. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is characterized by the presence of mutant alleles of ASXL1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of at least one other gene, wherein the other gene is selected from the group consisting of KRAS, TP53, SETBP1, and U2AF1.

In certain examples, the myelodysplastic syndrome is characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of at least one other gene, wherein the other gene is selected from the group consisting of KRAS, TP53, SETBP1, U2AF1, TCF3, STAG2, NRAS, JAK2 and BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2, and the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes.

In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some such examples, the additional gene is selected from SRSF2, MPL, CSF3R, FAT3 and CBL. In certain examples, the myelodysplastic syndrome is characterized by the presence of a mutant allele of SRSF2. In certain embodiments, the second gene is SRSF2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of at least one other gene, wherein the other gene is selected from the group consisting of TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some such examples, the additional gene is selected from KRAS and MPL. In certain examples, the myelodysplastic syndrome is characterized by the presence of mutant alleles of ASXL1 and SRSF2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXI,1 and SRSF2; wherein the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some such examples, the additional gene is selected from MPL, CSF3R, FAT3 and CBL.

In certain examples, the myelodysplastic syndrome is characterized by the presence of mutant allele of KRAS. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of KRAS; wherein the myelodysplastic syndrome is further characterized by the absence of a mutant allele of at least one other gene, wherein the other gene is selected from the group consisting of TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some such examples, the additional gene is selected from SRSF2 and MPL.

In certain examples, the myelodysplastic syndrome is characterized by the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the absence of a mutant allele of KRAS. In certain examples, the myelodysplastic syndrome is further characterized by the presence of one or more mutant alleles of one or more additional genes. In certain examples, the myelodysplastic syndrome is characterized by the presence of a mutant allele of ASXL1. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of KRAS. In certain examples, the myelodysplastic syndrome is characterized by the presence of a mutant allele of SRSF2. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of KRAS. In certain examples, the myelodysplastic syndrome is characterized by the presence of mutant alleles of ASXL1 and SRSF2. In certain embodiments, provided herein is compound 1 for use in compound a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of KRAS.

In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the presence of a mutant allele of MPL. In certain examples, the myelodysplastic syndrome is further characterized by the presence of one or more mutant alleles of one or more additional genes. In some such examples, the additional gene is KRAS. In certain examples, the myelodysplastic syndrome is characterized by the presence of mutant alleles of ASXL1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the presence of a mutant allele of MPL. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the presence of a mutant allele of CSF3R. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the presence of a mutant allele of MPL, FAT3 and CBL.

In certain examples, the myelodysplastic syndrome is characterized by the absence of a mutant allele of TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2; wherein the myelodysplastic syndrome is further characterized by the absence of a mutant allele of TP53. In certain examples, the myelodysplastic syndrome is further characterized by the presence of one or more mutant alleles of one or more additional genes. In certain examples, the myelodysplastic syndrome is characterized by the presence of a mutant allele of ASXL1. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of TP53. In certain examples, the myelodysplastic syndrome is characterized by the presence of a mutant allele of SRSF2. In certain embodiments, the second gene is SRSF2. In certain embodiments, provided herein is compound 1 for use in of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of TP53. In certain examples, the myelodysplastic syndrome is characterized by the presence of mutant alleles of ASXL1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of TP53. In certain examples, the myelodysplastic syndrome is further characterized by the presence of one or more mutant alleles of one or more additional genes. In some such embodiments, the additional gene is selected from DNMT3A, MPL, CSF3R, FAT3 and CBL.

In certain examples, the myelodysplastic syndrome is characterized by the absence of a mutant allele of SETBP1. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of SETBP1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2; wherein the myelodysplastic syndrome is further characterized by the absence of a mutant allele of SETBP1. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of a mutant allele of ASXL1. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of SETBP1. In certain examples, the myelodysplastic syndrome is characterized by the presence of a mutant allele of SRSF2. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of SETBP1. In certain examples, the myelodysplastic syndrome is further characterized by the presence of one or more mutant alleles of one or more additional genes. In some examples, the additional gene is selected from KRAS and MPL. In certain examples, the myelodysplastic syndrome is characterized by the presence of mutant alleles of ASXL1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of SETBP1. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain examples, the myelodysplastic syndrome is characterized by the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2; wherein the myelodysplastic syndrome is further characterized by the absence of a mutant allele of U2AF1. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of a mutant allele of ASXL1. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of U2AF1. In certain examples, the myelodysplastic syndrome is characterized by the presence of a mutant allele of SRSF2. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of U2AF1. In certain examples, the myelodysplastic syndrome is characterized by the presence of mutant alleles of ASXL1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of U2AF1. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain examples, the myelodysplastic syndrome is characterized by the absence of a mutant allele of TCF3. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of TCF3. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the absence of a mutant allele of TCF3. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of a mutant allele of ASXL1. In certain embodiments, the second gene is ASXL1. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of TCF3. In certain examples, the myelodysplastic syndrome is characterized by the presence of a mutant allele of SRSF2. In certain embodiments, the second gene is SRSF2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of TCF3. In certain examples, the myelodysplastic syndrome is further characterized by the presence of one or more mutant alleles of one or more additional genes. In some examples, the additional gene is selected from KRAS, DNMT3A, MPL, CSF3R, FAT3 and CBL. In certain examples, the myelodysplastic syndrome is characterized by the presence of mutant alleles of ASXL1 and SRSF2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of TCF3. In certain examples, the myelodysplastic syndrome is further characterized by the presence of one or more mutant alleles of one or more additional genes. In some examples, the additional gene is selected from DNMT3A, MPL, CSF3R, FAT3 and CBL.

In certain examples, the myelodysplastic syndrome is characterized by the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the absence of a mutant allele of STAG2. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of a mutant allele of ASXL1. In certain embodiments, the second gene is ASXL1. In certain exampled, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of STAG2. In certain examples, the myelodysplastic syndrome is characterized by the presence of a mutant allele of SRSF2. In certain embodiments, the second gene is SRSF2. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of STAG2. In certain examples, the myelodysplastic syndrome is characterized by the presence of mutant alleles of ASXL1 and SRSF2. In certain examplents, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of STAG2. In certain examples, the myelodysplastic syndrome is further characterized by the presence of one or more mutant alleles of one or more additional genes. In some examples, the additional gene is selected from DNMT3A, MPL, CSF3R, FAT3 and CBL.

In certain examples, the myelodysplastic syndrome is characterized by the absence of a mutant allele of KRAS and TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of KRAS and TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the absence of a mutant allele of KRAS and TP53. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of a mutant allele of ASXL1. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of KRAS and TP53. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of a mutant allele of SRSF2. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of KRAS and TP53. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of mutant alleles of ASXL1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of KRAS and TP53. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain examples, the myelodysplastic syndrome is characterized by the absence of a mutant allele of KRAS, TP53, SETBP1, and U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of KRAS, TP53, SETBP1, and U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2; wherein the myelodysplastic syndrome is further characterized by the absence of a mutant allele of KRAS, TP53, SETBP1, and U2AF1. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of a mutant allele of ASXL1. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of KRAS, TP53, SETBP1, and U2AF1. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the presence of a mutant allele of SRSF2. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of KRAS, TP53, SETBP1, and U2AF1. In certain examples, the myelodysplastic syndrome is characterized by the presence of mutant alleles of ASXL1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of KRAS, TP53, SETBP1, and U2AF1. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain examples, the myelodysplastic syndrome is characterized by the absence of a mutant allele of KRAS, TP53, TCF3, and STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of KRAS, TP53, TCF3, and STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of a second gene, wherein the second gene is selected from the group consisting of ASXL1 and SRSF2; wherein the myelodysplastic syndrome is further characterized by the absence of a mutant allele of KRAS, TP53, TCF3, and STAG2. In certain examples, the myelodysplastic syndrome is further characterized by the presence of one or more mutant alleles of one or more additional genes. In some examples, the additional gene is selected from DNMT3A, MPL, CSF3R, FAT3 and CBL. In certain examples, the myelodysplastic syndrome is characterized by the presence of a mutant allele of ASXL1. In certain embodiments, the second gene is ASXL1. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of ASXL1; wherein the myelodysplastic syndrome is further characterized by the absence of a mutant allele of KRAS, TP53, TCF3, and STAG2. In certain examples, the myelodysplastic syndrome is further characterized by the presence of one or more mutant alleles of one or more additional genes. In some examples, the additional gene is selected from DNMT3A, MPL, CSF3R, FAT3 and CBL. In certain examples, the myelodysplastic syndrome is characterized by the presence of a mutant allele of SRSF2. In certain examples, the second gene is SRSF2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and a mutant allele of SRSF2; wherein the myelodysplastic syndrome is further characterized by the absence of a mutant allele of KRAS, TP53, TCF3, and STAG2. In certain examples, the myelodysplastic syndrome is further characterized by the presence of one or more mutant alleles of one or more additional genes. In some examples, the additional gene is selected from DNMT3A, MPL, CSF3R, FAT3 and CBL. In certain examples, the myelodysplastic syndrome is characterized by the presence of mutant alleles of ASXL 1 and SRSF2. In certain eexamples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and of mutant alleles of ASXL1 and SRSF2; wherein the myelodysplastic syndrome is further characterized by the absence of a mutant allele of KRAS, TP53, TCF3, and STAG2. In certain examples, the myelodysplastic syndrome is further characterized by the presence of one or more mutant alleles of one or more additional genes. In some examples, the additional gene is selected from DNMT3A, MPL, CSF3R, FAT3 and CBL.

In certain examples, the myelodysplastic syndrome is characterized by the absence of a mutant allele of SETBP1, NRAS, JAK2, and BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of IDH2 and the absence of a mutant allele of SETBP1, NRAS, JAK2, and BRAF. In certain examples, the myelodysplastic syndrome is further characterized by the presence of one or more mutant alleles of one or more additional genes. In some example, the additional gene is selected from DNMT3A, MPL, CSF3R, FAT3 and CBL.

In certain examples, the myelodysplastic syndrome is characterized by the absence of a mutant allele of KRAS and SETBP1. In certain examples, the myelodysplastic syndrome is characterized by the absence of a mutant allele of KRAS and U2AF1. In certain examples, the myelodysplastic syndrome is characterized by the absence of a mutant allele of TP53 and SETBP1. In certain examples, the myelodysplastic syndrome is characterized by the absence of a mutant allele of TP53 and U2AF1. In certain examples, the myelodysplastic syndrome is characterized by the absence of a mutant allele of SETBP1 and U2AF1. In certain examples, the myelodysplastic syndrome is characterized by the absence of a mutant allele of KRAS, TP53, and SETBP1. In certain examples, the myelodysplastic syndrome is characterized by the absence of mutaa mutant allele tion of KRAS, TP53, and U2AF1. In certain examples, the myelodysplastic syndrome is characterized by the absence of a mutant allele of KRAS, SETBP1, and U2AF1. In certain examples, the myelodysplastic syndrome is characterized by the absence of a mutant allele of TP53, SETBP1, and U2AF1. In certain examples, the myelodysplastic syndrome is characterized by the absence of a mutant allele of TP53, SETBP1, U2AF1, TCF3, STAG2, NRAS, JAK2 and BRAF.

In certain embodiments, the myelodysplastic syndrome to be treated with compound 1 is characterized by a mutant allele of IDH2, wherein the IDH2 mutation results in a new ability of the enzyme to catalyze the NADPH dependent reduction of α-ketoglutarate to R(-)-2-hydroxyglutarate in a patient. In certain embodiments, the mutant allele of IDH2 has an R140X mutation or R172X mutation. In certain embodiments, the mutant allele of IDH2 has an R140X mutation (mIDH2-R140). In certain embodiments, the R140X mutation is a R140Q mutation (mIDH2-R140Q). In certain embodiments, the R140X mutation is a R140W mutation (mIDH2-R140W). In certain embodiments, the R140X mutation is a R140L mutation (mIDH2-R140L). In certain embodiments, the mutant allele of IDH2 has an R172X mutation (mIDH2-R172). In certain embodiments, the R172X mutation is a R172K mutation (mIDH2-R172K). In certain embodiments, the R172X mutation is a R172G mutation (mIDH2-R172G).

In certain embodiments the mutant allele of IDH2 is mIDH2-R140. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140 and a mutant allele of a second gene, wherein the second gene is selected from the group consisting of ASXL1 and SRSF2. In certain examples, the myelodysplastic syndrome is further characterized by the presence of one or more mutant alleles of one or more additional genes. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140 and a mutant allele of ASXL1. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140 and a mutant allele of SRSF2. In certain examplents, the myelodysplastic syndrome is characterized by the presence of mutant alleles of ASXL 1 and SRSF2. In certain examples, described herein is a method of treating a myelodysplastic syndrome in a subject, comprising administering to the subject a therapeutically effective amount of COMPOUND 1, wherein the myelodysplastic syndrome is characterized by the presence of mIDH2-R140 and of mutant alleles of ASXL1 and SRSF2. In certain examples, the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DBMT3A, MPL, CSF3R, FAT3 and CBL.

In certain embodiments, the mutant allele of IDH2 is mIDH2-R172. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172 and a mutant allele of a second gene, wherein the second gene is selected from the group consisting of ASXL1 and SRSF2. In certain examples, the myelodysplastic syndrome is further characterized by the presence of one or more mutant alleles of one or more additional genes. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172 and a mutant allele of ASXL1. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172 and a mutant allele of SRSF2. In certain examples, the myelodysplastic syndrome is characterized by the presence of mutant alleles of ASXL 1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172 and of mutant alleles of ASXL1 and SRSF2. In certain examples, the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain embodiments, the mutant allele of IDH2 is mIDH2-R140Q, mIDH2-R140W, mIDH2-R140L, mIDH2-R172K, or mIDH2-R172G.

In certain embodiments, the mutant allele of IDH2 is mIDH2-R140Q. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140Q and a mutant allele of a second gene, wherein the second gene is selected from the group consisting of ASXL1 and SRSF2. In certain examples, the myelodysplastic syndrome is further characterized by the presence of one or more mutant alleles of one or more additional genes. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140Q and a mutant allele of ASXL1. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140Q and a mutant allele of SRSF2. In certain examples, the myelodysplastic syndrome is characterized by the presence of mutant alleles of ASXL 1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140Q and of mutant alleles of ASXL1 and SRSF2. In certain examples, the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain embodiments, the mutant allele of IDH2 is mIDH2-R140W. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140W and a mutant allele of a second gene, wherein the second gene is selected from the group consisting of ASXL1 and SRSF2. In certain examples, the myelodysplastic syndrome is further characterized by the presence of one or more mutant alleles of one or more additional genes. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140W and a mutant allele of ASXL1. In certain embodiments, the second gene is SRSF2. In certain embodiments, provided herein is compound 1 for use in a method method of treating a myelodysplastic syndrome in a subject, comprising administering to the subject a therapeutically effective amount of COMPOUND 1, wherein the myelodysplastic syndrome is characterized by the presence of mIDH2-R140W and a mutant allele of SRSF2. In certain examples, the myelodysplastic syndrome is characterized by the presence of mutant alleles of ASXL1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140W and of mutant alleles of ASXL1 and SRSF2. In certain examples, the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain embodiments, the mutant allele of IDH2 is mIDH2-R140L. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140L and a mutant allele of a second gene, wherein the second gene is selected from the group consisting of ASXL1 and SRSF2. In certain examples, the myelodysplastic syndrome is further characterized by the presence of one or more mutant alleles of one or more additional genes. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140L and a mutant allele of ASXL1. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140L and a mutant allele of SRSF2. In certain examples, the myelodysplastic syndrome is characterized by the presence of mutant alleles of ASXL 1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140L and of mutant alleles of ASXL1 and SRSF2. In certain examples, the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain embodiments, the mutant allele of IDH2 is mIDH2-R172K. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172K and a mutant allele of a second gene, wherein the second gene is selected from the group consisting of ASXL1 and SRSF2. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172K and a mutant allele of ASXL1. In certain embodiments, the second gene is SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172K and a mutant allele of SRSF2. In certain examples, the myelodysplastic syndrome is characterized by the presence of mutant alleles of ASXL 1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172K and of mutant alleles of ASXL1 and SRSF2. In certain examples, the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain embodiments, the mutant allele of IDH2 is mIDH2-R172G. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172G and a mutant allele of a second gene, wherein the second gene is selected from the group consisting of ASXL1 and SRSF2. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain embodiments, the second gene is ASXL1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172G and a mutant allele of ASXL1. In certain embodiments, the second gene is SRSF2. In certain embodiments, provided herein is a compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172G and a mutant allele of SRSF2. In certain examples, the myelodysplastic syndrome is characterized by the presence of mutant alleles of ASXL 1 and SRSF2. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R172G and of mutant alleles of ASXL1 and SRSF2. In certain examples, the myelodysplastic syndrome is further characterized by the absence of a mutant allele of at least one other gene, wherein the other gene is selected from the group consisting of KRAS, TP53, SETBP1, and U2AF1. In certain examples, the myelodysplastic syndrome is further characterized by the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain embodiments, the mutant allele of IDH2 is mIDH2-R140. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of mIDH2-R140 and the absence of a mutant allele of at least one other gene, wherein the other gene is selected from the group consisting of KRAS, TP53, SETBP1, and U2AF1. In certain examples, described herein is a method of treating a myelodysplastic syndrome in a subject, comprising administering to the subject a therapeutically effective amount of COMPOUND 1, wherein the myelodysplastic syndrome is characterized by the presence of mIDH2-R140 and the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.In certain examples, the myelodysplastic syndrome is characterized by the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140 and the absence of a mutant allele of KRAS. In certain examples, the myelodysplastic syndrome is characterized by the absence of a mutant allele of TP53. In certain embodiments, provided herein is compound 1 for use in a method of treating a myelodysplastic syndrome in a subject comprising administering to the subject a therapeutically effective amount of COMPOUND 1, wherein the myelodysplastic syndrome is characterized by the presence of a mutant allele of mIDH2-R140 and the absence of a mutant allele of TP53. In certain examples, the myelodysplastic syndrome is characterized by the absence of a mutant allele of SETBP1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140 and the absence of a mutant allele of SETBP1. In certain examples, the myelodysplastic syndrome is characterized by the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140 and the absence of a mutant allele of U2AF1. In certain examples, the myelodysplastic syndrome is characterized by the absence of a mutant allele of TCF3. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140 and the absence of a mutant allele of TCF3. In certain examples, the myelodysplastic syndrome is characterized by the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140 and the absence of a mutant allele of STAG2. In certain examples, the myelodysplastic syndrome is characterized by the absence of a mutant allele of NRAS. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140 and the absence of a mutant allele of NRAS. In certain examples, the myelodysplastic syndrome is characterized by the absence of a mutant allele of JAK2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140 and the absence of a mutant allele of JAK2. In certain examples, the myelodysplastic syndrome is characterized by the absence of a mutant allele of BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140 and the absence of a mutant allele of BRAF. In certain examples, the myelodysplastic syndrome is characterized by the presence of a mutant allele of a second gene, wherein the other gene is selected from the group consisting of ASXL1 and SRSF2. In certain examples, the myelodysplastic syndrome is characterized by the presence of a mutant allele of a second gene, wherein the other gene is selected from the group consisting of ASXL1, SRSF2, MPL, DNMT3A, CSF3R, FAT3, and CBL. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.

In certain embodiments, the mutant allele of IDH2 is mIDH2-R172. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of at least one other gene, wherein the other gene is selected from the group consisting of KRAS, TP53, SETBP1, and U2AF1. In certain examples, described herein is a method of treating a myelodysplastic syndrome in a subject, comprising administering to the subject a therapeutically effective amount of COMPOUND 1, wherein the myelodysplastic syndrome is characterized by the presence of mIDH2-R172 and the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL. In certain examples, the myelodysplastic syndrome is characterized by the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of KRAS. In certain examples, the myelodysplastic syndrome is characterized by the absence of a mutant allele of TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of TP53. In certain examples, the myelodysplastic syndrome is characterized by the absence of a mutant allele of SETBP1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of SETBP1. In certain examples, the myelodysplastic syndrome is characterized by the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of U2AF1. In certain examples, the myelodysplastic syndrome is characterized by the absence of a mutant allele of TCF3. In certain examples, describedherein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of TCF3. In certain examples, the myelodysplastic syndrome is characterized by the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of STAG2. In certain examples, the myelodysplastic syndrome is characterized by the absence of a mutant allele of NRAS. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of NRAS. In certain examples, the myelodysplastic syndrome is characterized by the absence of a mutant allele of JAK2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of JAK2. In certain examples, the myelodysplastic syndrome is characterized by the absence of a mutant allele of BRAF. In certain examples,described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172 and the absence of a mutant allele of BRAF. In certain examples, the myelodysplastic syndrome is characterized by the presence of a mutant allele of a second gene, wherein the other gene is selected from the group consisting of ASXL1 and SRSF2. In certain examples, the myelodysplastic syndrome is characterized by the presence of a mutant allele of a second gene, wherein the other gene is selected from the group consisting of ASXL1, SRSF2, MPL, DNMT3A, CSF3R, FAT3, and CBL.

In certain embodiments, the mutant allele of IDH2 is mIDH2-R140Q. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of at least one other gene, wherein the other gene is selected from the group consisting of KRAS, TP53, SETBP1, and U2AF1. In certain examples, described herein is a method of treating a myelodysplastic syndrome in a subject, comprising administering to the subject a therapeutically effective amount of COMPOUND 1, wherein the myelodysplastic syndrome is characterized by the presence of mIDH2-R140Q and the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some examples, the additional gene is selected from DNMT3A, MPL, CSF3R, FAT3 and CBL.In certain examples, the myelodysplastic syndrome is characterized by the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of KRAS. In certain examples, the myelodysplastic syndrome is characterized by the absence of a mutant allele of TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of TP53. In certain examples, the myelodysplastic syndrome is characterized by the absence of a mutant allele of SETBP1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of SETBP1. In certain examples, the myelodysplastic syndrome is characterized by the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of U2AF1. In certain examples, the myelodysplastic syndrome is characterized by the absence of a mutant allele of TCF3. In certain examples,described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of TCF3. In certain examples, the myelodysplastic syndrome is characterized by the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of STAG2. In certain examples, the myelodysplastic syndrome is characterized by the absence of a mutant allele of NRAS. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of NRAS. In certain examples, the myelodysplastic syndrome is characterized by the absence of a mutant allele of JAK2. In certain examped, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of JAK2. In certain examples, the myelodysplastic syndrome is characterized by the absence of a mutant allele of BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140Q and the absence of a mutant allele of BRAF. In certain examples, the myelodysplastic syndrome is characterized by the presence of a mutant allele of a second gene, wherein the other gene is selected from the group consisting of ASXL1 and SRSF2. In certain examples, the myelodysplastic syndrome is characterized by the presence of a mutant allele of a second gene, wherein the other gene is selected from the group consisting of ASXL1, SRSF2, MPL, DNMT3A, CSF3R, FAT3, and CBL.

In certain embodiments, the mutant allele of IDH2 is mIDH2-R140W. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of at least one other gene, wherein the other gene is selected from the group consisting of KRAS, TP53, SETBP1, and U2AF1. In certain examples, described herein is a method of treating a myelodysplastic syndrome in a subject, comprising administering to the subject a therapeutically effective amount of COMPOUND 1, wherein the myelodysplastic syndrome is characterized by the presence of mIDH2-R140W and the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.In certain examples, the myelodysplastic syndrome is characterized by the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of KRAS. In certain examples, the myelodysplastic syndrome is characterized by the absence of a mutant allele of TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of TP53. In certain examples, the myelodysplastic syndrome is characterized by the absence of a mutant allele of SETBP1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of SETBP1. In certain examples, the myelodysplastic syndrome is characterized by the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of U2AF1. In certain examples, the myelodysplastic syndrome is characterized by the absence of a mutant allele of TCF3. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of TCF3. In certain examples, the myelodysplastic syndrome is characterized by the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of STAG2. In certain examples, the myelodysplastic syndrome is characterized by the absence of a mutant allele of NRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of NRAS. In certain examples, the myelodysplastic syndrome is characterized by the absence of a mutant allele of JAK2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of JAK2. In certain examples, the myelodysplastic syndrome is characterized by the absence of a mutant allele of BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140W and the absence of a mutant allele of BRAF. In certain examples, the myelodysplastic syndrome is characterized by the presence of a mutant allele of a second gene, wherein the other gene is selected from the group consisting of ASXL1 and SRSF2. In certain examples, the myelodysplastic syndrome is characterized by the presence of a mutant allele of a second gene, wherein the other gene is selected from the group consisting of ASXL1, SRSF2, MPL, DNMT3A, CSF3R, FAT3, and CBL.

In certain embodiments, the mutant allele of IDH2 is mIDH2-R140L. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of at least one other gene, wherein the other gene is selected from the group consisting of KRAS, TP53, SETBP1, and U2AF1. In certain examples, described herein is a method of treating a myelodysplastic syndrome in a subject, comprising administering to the subject a therapeutically effective amount of COMPOUND 1, wherein the myelodysplastic syndrome is characterized by the presence of mIDH2-R140L and the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.In certain examples, the myelodysplastic syndrome is characterized by the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of KRAS. In certain examples, the myelodysplastic syndrome is characterized by the absence of a mutant allele of TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of TP53. In certain examples, the myelodysplastic syndrome is characterized by the absence of a mutant allele of SETBP1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of SETBP1. In certain examples, the myelodysplastic syndrome is characterized by the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of U2AF1. In certain examples, the myelodysplastic syndrome is characterized by the absence of a mutant allele of TCF3. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of TCF3. In certain examples, the myelodysplastic syndrome is characterized by the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of STAG2. In certain examples, the myelodysplastic syndrome is characterized by the absence of a mutant allele of NRAS. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of NRAS. In certain examples, the myelodysplastic syndrome is characterized by the absence of a mutant allele of JAK2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of JAK2. In certain examples, the myelodysplastic syndrome is characterized by the absence of a mutant allele of BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R140L and the absence of a mutant allele of BRAF. In certain examples, the myelodysplastic syndrome is characterized by the presence of a mutant allele of a second gene, wherein the other gene is selected from the group consisting of ASXL1 and SRSF2. In certain examples, the myelodysplastic syndrome is characterized by the presence of a mutant allele of a second gene, wherein the other gene is selected from the group consisting of ASXL1, SRSF2, MPL, DNMT3A, CSF3R, FAT3, and CBL.

In certain embodiments, the mutant allele of IDH2 is mIDH2-R172K. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of at least one other gene, wherein the other gene is selected from the group consisting of KRAS, TP53, SETBP1, and U2AF1. In certain examples, described herein is a method of treating a myelodysplastic syndrome in a subject, comprising administering to the subject a therapeutically effective amount of COMPOUND 1, wherein the myelodysplastic syndrome is characterized by the presence of mIDH2-R172K and the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some examples, the additional gene is selected from DNMT3A, MPL, CSF3R, FAT3 and CBL.In certain examples, the myelodysplastic syndrome is characterized by the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of KRAS. In certain examples, the myelodysplastic syndrome is characterized by the absence of a mutant allele of TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of TP53. In certain examples, the myelodysplastic syndrome is characterized by the absence of a mutant allele of SETBP1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of SETBP1. In certain examples, the myelodysplastic syndrome is characterized by the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of U2AF1. In certain examples, the myelodysplastic syndrome is characterized by the absence of a mutant allele of TCF3. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of TCF3. In certain examples, the myelodysplastic syndrome is characterized by the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of STAG2. In certain examples, the myelodysplastic syndrome is characterized by the absence of a mutant allele of NRAS. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of NRAS. In certain examples, the myelodysplastic syndrome is characterized by the absence of a mutant allele of JAK2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of JAK2. In certain examples, the myelodysplastic syndrome is characterized by the absence of a mutant allele of BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172K and the absence of a mutant allele of BRAF. In certain examples, the myelodysplastic syndrome is characterized by the presence of a mutant allele of a second gene, wherein the other gene is selected from the group consisting of ASXL1 and SRSF2. In certain examples, the myelodysplastic syndrome is characterized by the presence of a mutant allele of a second gene, wherein the other gene is selected from the group consisting of ASXL1, SRSF2, MPL, DNMT3A, CSF3R, FAT3, and CBL.

In certain embodiments, the mutant allele of IDH2 is mIDH2-R172G. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of at least one other gene, wherein the other gene is selected from the group consisting of KRAS, TP53, SETBP1, and U2AF1. In certain examples, described herein is a method of treating a myelodysplastic syndrome in a subject, comprising administering to the subject a therapeutically effective amount of COMPOUND 1, wherein the myelodysplastic syndrome is characterized by the presence of mIDH2-R172G and the absence of a mutant allele of at least one other gene, wherein the other gene is selected from the group consisting of KRAS, TP53, SETBP1, U2AF1, TCF3, STAG2, NRAS, JAK2 and BRAF. In certain examples, the myelodysplastic syndrome is further characterized by the presence of one or more mutant alleles of one or more additional genes. In some embodiments, the additional gene is selected from DNMT3A, MPL, CSF3R, FAT3 and CBL.In certain examples, the myelodysplastic syndrome is characterized by the absence of a mutant allele of KRAS. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of KRAS. In certain examples, the myelodysplastic syndrome is characterized by the absence of a mutant allele of TP53. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of TP53. In certain examples, the myelodysplastic syndrome is characterized by the absence of a mutant allele of SETBP1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of SETBP1. In certain examples, the myelodysplastic syndrome is characterized by the absence of a mutant allele of U2AF1. In certain embodiments, provided herein is compound 1 for use in a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of U2AF1. In certain examples, the myelodysplastic syndrome is characterized by the absence of a mutant allele of TCF3. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of TCF3. In certain examples, the myelodysplastic syndrome is characterized by the absence of a mutant allele of STAG2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of STAG2. In certain examples, the myelodysplastic syndrome is characterized by the absence of a mutant allele of NRAS. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of NRAS. In certain examples, the myelodysplastic syndrome is characterized by the absence of a mutant allele of JAK2. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of JAK2. In certain examples, the myelodysplastic syndrome is characterized by the absence of a mutant allele of BRAF. In certain examples, described herein is a method of treating a myelodysplastic syndrome in a subject, comprising administering to the subject a therapeutically effective amount of COMPOUND 1, wherein the myelodysplastic syndrome is characterized by the presence of a mutant allele of mIDH2-R172G and the absence of a mutant allele of BRAF. In certain examples, the myelodysplastic syndrome is characterized by the presence of a mutant allele of a second gene, wherein the other gene is selected from the group consisting of ASXL1 and SRSF2. In certain examples, the myelodysplastic syndrome is characterized by the presence of a mutant allele of a second gene, wherein the other gene is selected from the group consisting of ASXL1, SRSF2, MPL, DNMT3A, CSF3R, FAT3, and CBL.

In certain embodiments, COMPOUND 1 for use in the methods disclosed herein is a mesylate salt of 2-methyl-1-[(4-[6-(trifluoromethyl)pyridin-2-yl]-6-{ [2-(trifluoromethyl)pyridin-4-yl]amino}-1,3,5-triazin-2-yl)amino]propan-2-ol (COMPOUND A).

In certain embodiments, the myelodysplastic syndrome (MDS) is MDS with refractory anemia with excess blasts (subtype RAEB-1 or RAEB-2). In certain embodiments, the MDS is untreated. In certain embodiments, COMPOUND 1 is administered as a first line treatment for MDS. In certain embodiments, COMPOUND 1 is administered as a second line, third line, or fourth line treatment for MDS. In certain embodiments, the MDS presentation is subsequent to AML. In one embodiment, the subject is an adult MDS patient.

In one embodiment, a malignancy can be analyzed by sequencing cell samples to determine the presence and specific nature of (e.g., the changed amino acid present at) a mutation at amino acid 140 and/or 172 of IDH2.

In another aspect, without being bound by theory, certain IDH2 mutations result in a new ability of the enzyme to catalyze the NADPH dependent reduction of α-ketoglutarate to R(-)-2-hydroxyglutarate, in particular R140Q and/or R172K mutations of IDH2. Thus, the compounds, compositions and methods provided herein are useful to treat a myelodysplastic syndrome that is characterized by the presence of a mutant allele of IDH2 imparting such activity and in particular an IDH2 R140Q and/or R172K mutation.

In certain embodiments, at least 30, 40, 50, 60, 70, 80 or 90% of the cancerous cells carry an IDH2 mutation, and in particular mIDH2-R140Q, mIDH2-R140W, or mIDH2-R140L and/or mIDH2-R172K or mIDH2-R172G mutation, at the time of diagnosis or treatment.

In certain embodiments, the efficacy of treatment of a myelodysplastic syndrome is monitored by measuring the levels of 2HG in the subject. Typically levels of 2HG are measured prior to treatment, wherein an elevated level is indicated for the use of COMPOUND 1. Once the elevated levels are established, the level of 2HG is determined during the course of and/or following termination of treatment to establish efficacy. In certain embodiments, the level of 2HG is only determined during the course of and/or following termination of treatment. A reduction of 2HG levels during the course of treatment and following treatment is indicative of efficacy. Similarly, a determination that 2HG levels are not elevated during the course of or following treatment is also indicative of efficacy. Typically, 2HG measurements are utilized together with other well known determinations of efficacy of malignancy treatment, such as reduction in number and size of tumors and/or other cancer associated lesions, improvement in the general health of the subject, and alterations in other biomarkers that are associated with malignancy treatment efficacy.

2HG can be detected in a sample by the methods of PCT Publication No. WO 2011/050210 and US Publication No. US2012/0121515. In an exemplary method, 2HG can be detected in a sample by LC/MS. The sample is mixed 80:20 with methanol, and centrifuged at 3,000 rpm for 20 minutes at 4 degrees Celsius. The resulting supernatant can be collected and stored at -80 degrees Celsius prior to LC-MS/MS to assess 2-hydroxyglutarate levels. A variety of different liquid chromatography (LC) separation methods can be used. Each method can be coupled by negative electrospray ionization (ESI, -3.0 kV) to triple-quadrupole mass spectrometers operating in multiple reaction monitoring (MRM) mode, with MS parameters optimized on infused metabolite standard solutions. Metabolites can be separated by reversed phase chromatography using 10 mM tributyl-amine as an ion pairing agent in the aqueous mobile phase, according to a variant of a previously reported method (Luo et al. J Chromatogr A 1147, 153-64, 2007). One method allows resolution of TCA metabolites: t = 0, 50% B; t = 5, 95% B; t= 7, 95% B; t= 8, 0% B, where B refers to an organic mobile phase of 100% methanol. Another method is specific for 2-hydroxyglutarate, running a fast linear gradient from 50% -95% B (buffers as defined above) over 5 minutes. A Synergi Hydro-RP, 100mm × 2 mm, 2.1 µm particle size (Phenomonex) can be used as the column, as described above. Metabolites can be quantified by comparison of peak areas with pure metabolite standards at known concentration. Metabolite flux studies from ¹³C-glutamine can be performed as described, *e.g.,* in Munger et al. Nat Biotechnol 26, 1179-86, 2008.

In certain embodiments, 2HG is directly evaluated.

In certain embodiments, a derivative of 2HG formed in process of performing the analytic method is evaluated. By way of example such a derivative can be a derivative formed in MS analysis. Derivatives can include a salt adduct, *e.g.,* a Na adduct, a hydration variant, or a hydration variant which is also a salt adduct, e.g., a Na adduct, e.g., as formed in MS analysis.

In certain embodiments a metabolic derivative of 2HG is evaluated. Examples include species that build up or are elevated, or reduced, as a result of the presence of 2HG, such as glutarate or glutamate that will be correlated to 2HG, *e.g.,* R-2HG.

Exemplary 2HG derivatives include dehydrated derivatives such as the compounds provided below or a salt adduct thereof:

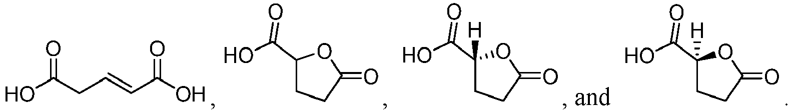

2HG is known to accumulate in the inherited metabolic disorder 2-hydroxyglutaric aciduria. This disease is caused by deficiency in the enzyme 2-hydroxyglutarate dehydrogenase, which converts 2HG to α-KG (Struys, E. A. et al. Am. J. Hum. Genet. 76, 358-60 (2005)). Patients with 2-hydroxyglutarate dehydrogenase deficiencies accumulate 2HG in the brain as assessed by MRI and CSF analysis, develop leukoencephalopathy, and have an increased risk of developing brain tumors (Aghili, M., Zahedi, F. & Rafiee, J Neurooncol 91, 233-6 (2009); Kolker, S., Mayatepek, E. & Hoffmann, G. F. Neuropediatrics 33, 225-31 (2002); Wajner, M., Latini, A., Wyse, A. T. & Dutra-Filho, C. S. J Inherit Metab Dis 27, 427-48 (2004)). Furthermore, elevated brain levels of 2HG result in increased ROS levels (Kolker, S. et al. Eur J Neurosci 16, 21-8 (2002); Latini, A. et al. Eur J Neurosci 17, 2017-22 (2003)), potentially contributing to an increased risk of cancer. The ability of 2HG to act as an NMDA receptor agonist may contribute to this effect (Kolker, S. et al. Eur J Neurosci 16, 21-8 (2002)). 2HG may also be toxic to cells by competitively inhibiting glutamate and/or αKG utilizing enzymes. These include transaminases which allow utilization of glutamate nitrogen for amino and nucleic acid biosynthesis, and αKG-dependent prolyl hydroxylases such as those which regulate Hif1-alpha levels.

Treatment methods described herein can additionally comprise various evaluation steps prior to and/or following treatment with COMPOUND 1.

In one embodiment, prior to and/or after treatment with COMPOUND 1, the method further comprises the step of evaluating the growth, size, weight, invasiveness, stage and/or other phenotype of a myelodysplastic syndrome.

In certain embodiments, prior to and/or after treatment with COMPOUND 1, the method further comprises the step of evaluating the IDH2 genotype of the myelodysplastic syndrome. This may be achieved by ordinary methods in the art, such as DNA sequencing, immuno analysis, and/or evaluation of the presence, distribution or level of 2HG.

In one embodiment, prior to and/or after treatment with COMPOUND 1, the method further comprises the step of determining the 2HG level in the subject. This may be achieved by spectroscopic analysis, *e.g.,* magnetic resonance-based analysis, *e.g.,* MRI and/or MRS measurement, sample analysis of bodily fluid, such as serum or spinal cord fluid analysis, or by analysis of surgical material, *e.g.,* by mass-spectroscopy.

In certain embodiments, depending on the disease to be treated and the subject's condition, COMPOUND 1 may be administered by oral, parenteral (*e.g.,* intramuscular, intraperitoneal, intravenous, CIV, intracistemal injection or infusion, subcutaneous injection, or implant), inhalation, nasal, vaginal, rectal, sublingual, or topical (*e.g.,* transdermal or local) routes of administration. COMPOUND 1 may be formulated alone or together with one or more active agent(s), in suitable dosage unit with pharmaceutically acceptable excipients, carriers, adjuvants and vehicles, appropriate for each route of administration.

In certain embodiments, the amount of COMPOUND 1 administered in the methods provided herein may range, *e.g.,* between about 5 mg/day and about 2,000 mg/day. In one embodiment, the range is between about 10 mg/day and about 2,000 mg/day. In one embodiment, the range is between about 20 mg/day and about 2,000 mg/day. In one embodiment, the range is between about 50 mg/day and about 1,000 mg/day. In one embodiment, the range is between about 100 mg/day and about 1,000 mg/day. In one embodiment, the range is between about 100 mg/day and about 500 mg/day. In one embodiment, the range is between about 150 mg/day and about 500 mg/day. In one embodiment, the range is or between about 150 mg/day and about 250 mg/day. In certain embodiments, particular dosages are, *e.g.,* about 10 mg/day. In one embodiment, the dose is about 20 mg/day. In one embodiment, the dose is about 50 mg/day. In one embodiment, the dose is about 60 mg/day. In one embodiment, the dose is about 75 mg/day. In one embodiment, the dose is about 100 mg/day. In one embodiment, the dose is about 120 mg/day. In one embodiment, the dose is about 150 mg/day. In one embodiment, the dose is about 200 mg/day. In one embodiment, the dose is about 250 mg/day. In one embodiment, the dose is about 300 mg/day. In one embodiment, the dose is about 350 mg/day. In one embodiment, the dose is about 400 mg/day. In one embodiment, the dose is about 450 mg/day. In one embodiment, the dose is about 500 mg/day. In one embodiment, the dose is about 600 mg/day. In one embodiment, the dose is about 700 mg/day. In one embodiment, the dose is about 800 mg/day. In one embodiment, the dose is about 900 mg/day. In one embodiment, the dose is about 1,000 mg/day. In one embodiment, the dose is about 1,200 mg/day. In one embodiment, the dose is or about 1,500 mg/day. In certain embodiments, particular dosages are, *e.g.,* up to about 10 mg/day. In one embodiment, the particular dose is up to about 20 mg/day. In one embodiment, the particular dose is up to about 50 mg/day. In one embodiment, the particular dose is up to about 60 mg/day. In one embodiment, the particular dose is up to about 75 mg/day. In one embodiment, the particular dose is up to about 100 mg/day. In one embodiment, the particular dose is up to about 120 mg/day. In one embodiment, the particular dose is up to about 150 mg/day. In one embodiment, the particular dose is up to about 200 mg/day. In one embodiment, the particular dose is up to about 250 mg/day. In one embodiment, the particular dose is up to about 300 mg/day. In one embodiment, the particular dose is up to about 350 mg/day. In one embodiment, the particular dose is up to about 400 mg/day. In one embodiment, the particular dose is up to about 450 mg/day. In one embodiment, the particular dose is up to about 500 mg/day. In one embodiment, the particular dose is up to about 600 mg/day. In one embodiment, the particular dose is up to about 700 mg/day. In one embodiment, the particular dose is up to about 800 mg/day. In one embodiment, the particular dose is up to about 900 mg/day. In one embodiment, the particular dose is up to about 1,000 mg/day. In one embodiment, the particular dose is up to about 1,200 mg/day. In one embodiment, the particular dose is up to about 1,500 mg/day.

In certain embodiments, COMPOUND 1 for methods described herein is administered at a dose of about 20 to 2000 mg/day. In certain embodiments, COMPOUND 1 is administered at a dose of about 50 to 500 mg/day. In certain embodiments, the dose is about 50 mg/day. In certain embodiments, the dose is about 60 mg/day. In certain embodiments, the dose is about 100 mg/day. In certain embodiments, the dose is about 150 mg/day. In certain embodiments, the dose is about 200 mg/day. In certain embodiments, the dose is about 300 mg/day.

In one embodiment, the amount of COMPOUND 1 in the pharmaceutical composition or dosage form provided herein may range, *e.g.,* between about 5 mg and about 2,000 mg. In one embodiment, the range is between about 10 mg and about 2,000 mg. In one embodiment, the range is between about 20 mg and about 2,000 mg. In one embodiment, the range is between about 50 mg and about 1,000 mg. In one embodiment, the range is between about 50 mg and about 500 mg. In one embodiment, the range is between about 50 mg and about 250 mg. In one embodiment, the range is between about 100 mg and about 500 mg. In one embodiment, the range is between about 150 mg and about 500 mg. In one embodiment, the range is between about 150 mg and about 250 mg. In certain embodiments, particular amounts are, *e.g.,* about 10 mg. In one embodiment, the particular amount is about 20 mg. In one embodiment, the particular amount is about 30 mg. In one embodiment, the particular amount is about 50 mg. In one embodiment, the particular amount is about 60 mg. In one embodiment, the particular amount is about 75 mg. In one embodiment, the particular amount is about 100 mg. In one embodiment, the particular amount is about 120 mg. In one embodiment, the particular amount is about 150 mg. In one embodiment, the particular amount is about 200 mg. In one embodiment, the particular amount is about 250 mg. In one embodiment, the particular amount is about 300 mg. In one embodiment, the particular amount is about 350 mg. In one embodiment, the particular amount is about 400 mg. In one embodiment, the particular amount is about 450 mg. In one embodiment, the particular amount is about 500 mg. In one embodiment, the particular amount is about 600 mg. In one embodiment, the particular amount is about 650 mg. In one embodiment, the particular amount is about 700 mg. In one embodiment, the particular amount is about 800 mg. In one embodiment, the particular amount is about 900 mg. In one embodiment, the particular amount is about 1,000 mg. In one embodiment, the particular amount is about 1,200 mg. In one embodiment, the particular amount is or about 1,500 mg. In certain embodiments, particular amounts are, *e.g.,* up to about 10 mg. In one embodiment, the particular amount is up to about 20 mg. In one embodiment, the particular amount is up to about 50 mg. In one embodiment, the particular amount is up to about 60 mg. In one embodiment, the particular amount is up to about 75 mg. In one embodiment, the particular amount is up to about 100 mg. In one embodiment, the particular amount is up to about 120 mg. In one embodiment, the particular amount is up to about 150 mg. In one embodiment, the particular amount is up to about 200 mg. In one embodiment, the particular amount is up to about 250 mg. In one embodiment, the particular amount is up to about 300 mg. In one embodiment, the particular amount is up to about 350 mg. In one embodiment, the particular amount is up to about 400 mg. In one embodiment, the particular amount is up to about 450 mg. In one embodiment, the particular amount is up to about 500 mg. In one embodiment, the particular amount is up to about 600 mg. In one embodiment, the particular amount is up to about 700 mg. In one embodiment, the particular amount is up to about 800 mg. In one embodiment, the particular amount is up to about 900 mg. In one embodiment, the particular amount is up to about 1,000 mg. In one embodiment, the particular amount is up to about 1,200 mg. In one embodiment, the particular amount is up to about 1,500 mg.

In one embodiment, COMPOUND 1 can be delivered as a single dose such as, e.g., a single bolus injection, or oral tablets or pills; or over time such as, *e.g.,* continuous infusion over time or divided bolus doses over time. In one embodiment, COMPOUND 1 can be administered repetitively if necessary, for example, until the patient experiences stable disease or regression, or until the patient experiences disease progression or unacceptable toxicity. Stable disease or lack thereof is determined by methods known in the art such as evaluation of patient's symptoms, physical examination, visualization of the tumor that has been imaged using X-ray, CAT, PET, or MRI scan and other commonly accepted evaluation modalities.

In certain embodiments, COMPOUND 1 for methods described herein is administered once daily.

In certain embodiments, COMPOUND 1 is administered to a patient in cycles (*e.g.,* daily administration for one week, then a rest period with no administration for up to three weeks). Cycling therapy involves the administration of an active agent for a period of time, followed by a rest for a period of time, and repeating this sequential administration. Cycling therapy can reduce the development of resistance, avoid or reduce the side effects, and/or improves the efficacy of the treatment.

In one embodiment, a method provided herein comprises administering COMPOUND 1 in 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or greater than 40 cycles. In certain embodiments, COMPOUND 1 for methods described herein is administered for 1 to 25 cycles. In one embodiment, the median number of cycles administered in a group of patients is about 1. In one embodiment, the median number of cycles administered in a group of patients is about 2. In one embodiment, the median number of cycles administered in a group of patients is about 3. In one embodiment, the median number of cycles administered in a group of patients is about 4. In one embodiment, the median number of cycles administered in a group of patients is about 5. In one embodiment, the median number of cycles administered in a group of patients is about 6. In one embodiment, the median number of cycles administered in a group of patients is about 7. In one embodiment, the median number of cycles administered in a group of patients is about 8. In one embodiment, the median number of cycles administered in a group of patients is about 9. In one embodiment, the median number of cycles administered in a group of patients is about 10. In one embodiment, the median number of cycles administered in a group of patients is about 11. In one embodiment, the median number of cycles administered in a group of patients is about 12. In one embodiment, the median number of cycles administered in a group of patients is about 13. In one embodiment, the median number of cycles administered in a group of patients is about 14. In one embodiment, the median number of cycles administered in a group of patients is about 15. In one embodiment, the median number of cycles administered in a group of patients is about 16. In one embodiment, the median number of cycles administered in a group of patients is about 17. In one embodiment, the median number of cycles administered in a group of patients is about 18. In one embodiment, the median number of cycles administered in a group of patients is about 19. In one embodiment, the median number of cycles administered in a group of patients is about 20. In one embodiment, the median number of cycles administered in a group of patients is about 21. In one embodiment, the median number of cycles administered in a group of patients is about 22. In one embodiment, the median number of cycles administered in a group of patients is about 23. In one embodiment, the median number of cycles administered in a group of patients is about 24. In one embodiment, the median number of cycles administered in a group of patients is about 25. In one embodiment, the median number of cycles administered in a group of patients is about 26. In one embodiment, the median number of cycles administered in a group of patients is about 27. In one embodiment, the median number of cycles administered in a group of patients is about 28. In one embodiment, the median number of cycles administered in a group of patients is about 29. In one embodiment, the median number of cycles administered in a group of patients is about 30. In one embodiment, the median number of cycles administered in a group of patients is greater than about 30 cycles.

In certain embodiments, treatment cycles comprise multiple doses of COMPOUND 1 administered to a subject in need thereof over multiple days (*e.g.,* 1, 2, 3, 4, 5, 6, 7, 8, 9, 10, 11, 12, 13, 14, or greater than 14 days), optionally followed by treatment dosing holidays (e.g., 1, 2, 3, 4, 5, 6, 7, 8, 9, 10, 11, 12, 13, 14, 15, 16, 17, 18, 19, 20, 21, 22, 23, 24, 25, 26, 27, 28, or greater than 28 days).

In certain embodiments, COMPOUND 1 for methods described herein is administered in 28-day cycles.

In certain embodiments, COMPOUND 1 for methods described herein is administered orally.

In certain embodiments, COMPOUND 1 for methods described herein is administered once daily orally in 28-day cycles at the dose of about 100 mg/day.

In a particular example, COMPOUND A is for use in a method for treating a myelodysplastic syndrome in a subject, wherein the method comprises orally administering to the subject a therapeutically effective amount of COMPOUND A, wherein the myelodysplastic syndrome is characterized by the presence of a mutant allele of IDH2 and a mutant allele of at least one second gene, wherein the second gene is selected from the group consisting of ASXL1 and SRSF2, wherein COMPOUND A is administered once daily in 28-day cycles at a dose of 5, 10, 25, 50, 100, 150 or 200 mg. In one example, COMPOUND A is administered in a dose of 100 mg/day.

In a particular example, COMPOUND A is for use in a method for treating a myelodysplastic syndrome in a subject, wherein the method comprises orally administering to the subject a therapeutically effective amount of COMPOUND A, wherein the myelodysplastic syndrome is characterized by the presence of a mutant allele of IDH2 and a mutant allele of at least one second gene, wherein the second gene is selected from the group consisting of ASXL1 and SRSF2, wherein COMPOUND A is administered once daily in 28-day cycles at a dose of 5, 10, 25, 50, 100, 150 or 200 mg, wherein the myelodysplastic syndrome is subsequent to acute myeloid leukemia (AML), preferably including relapsed AML or refractory AML or untreated AML. In one example, COMPOUND A is administered in a dose of 100 mg/day, wherein the myelodysplastic syndrome is subsequent to acute myeloid leukemia (AML), preferably including relapsed AML or refractory AML or untreated AML.

In a more specific example, COMPOUND A is for use in a method for treating a myelodysplastic syndrome in a subject, wherein the method comprises orally administering to the subject a therapeutically effective amount of COMPOUND A, wherein the myelodysplastic syndrome is characterized by the presence of a mutant allele of IDH2 and a mutant allele of at least one second gene, wherein the second gene is selected from the group consisting of ASXL1 and SRSF2, wherein COMPOUND A is administered once daily in 28-day cycles at a dose of 5, 10, 25, 50, 100, 150 or 200 mg, in one example, at a dose of 100 mg/day, wherein the myelodysplastic syndrome is subsequent to acute myeloid leukemia (AML), including relapsed AML or refractory AML or untreated AML, and wherein the myelodysplastic syndrome is with refractory anemia.

In a more specific example, COMPOUND A is for use in a method for treating a myelodysplastic syndrome in a subject, wherein the method comprises orally administering to the subject a therapeutically effective amount of COMPOUND A, wherein the myelodysplastic syndrome is characterized by the presence of a mutant allele of IDH2 and a mutant allele of at least one second gene, wherein the second gene is selected from the group consisting of ASXL1 and SRSF2, wherein COMPOUND A is administered once daily in 28-day cycles at a dose of 5, 10, 25, 50, 100, 150 or 200 mg, in one example, at a dose of 100 mg/day, wherein the myelodysplastic syndrome is subsequent to acute myeloid leukemia (AML), including relapsed AML or refractory AML or untreated AML and wherein the myelodysplastic syndrome is with refractory anemia with excess blasts, subtype RAEB-1 or RAEB-2.

In a particular example, COMPOUND A is for use in a method for treating a myelodysplastic syndrome in a subject, wherein the method comprises orally administering to the subject a therapeutically effective amount of COMPOUND A, wherein the myelodysplastic syndrome is characterized by the presence of a mutant allele of IDH2 and a mutant allele of at least one second gene, wherein the second gene is selected from the group consisting of ASXL1 and SRSF2, wherein COMPOUND A is administered once daily in 28-day cycles at a dose of 5, 10, 25, 50, 100, 150 or 200 mg, in one example, at a dose of 100 mg/day, wherein the subject was relapsed or refractory to prior myelodysplastic syndrome therapy or was not a candidate for standard therapies.

In one example, described herein is a method of identifying a subject suitable for methods of treatment described herein, comprising: (a) obtaining a biological sample from a subject having a myelodysplastic syndrome; (b) screening the biological sample for an IDH2 mutation and a mutation of a second gene, wherein the second gene is selected from the group consisting of ASXL1 and SRSF2; and (c) if the myelodysplastic syndrome is characterized by the presence of a mutant allele of IDH2 and the presence of a mutant allele of a second gene, wherein the second gene is selected from the group consisting of ASXL1 and SRSF2, identifying the subject as a cancer subject suitable for treatment using the methods described herein. In another example, the subjects identified as subjects suitable for the methods of treatment described herein are treated with COMPOUND 1.

In one example, described herein is a method of identifying a subject suitable for methods of treatment described herein, comprising: (a) obtaining a biological sample from a subject having a myelodysplastic syndrome; (b) screening the biological sample for an IDH2 mutation and the mutation of at least one other gene, wherein the other gene is selected from the group consisting of KRAS, TP53, SETBP1, and U2AF1; and (c) if the myelodysplastic syndrome is characterized by the presence of a mutant allele of IDH2 and the absence of a mutant allele of at least one other gene, wherein the other gene is selected from the group consisting of KRAS, TP53, SETBP1, and U2AF1, identifying the subject as a cancer subject suitable for treatment using the methods described herein. In another example, the subjects identified as subjects suitable for the methods of treatment described herein are treated with COMPOUND 1.

In one example, described herein is a method of identifying a subject suitable for methods of treatment described herein, comprising: (a) obtaining a biological sample from a subject having a myelodysplastic syndrome; (b) screening the biological sample for an IDH2 mutation and the mutation of at least one other gene, wherein the other gene is selected from the group consisting of KRAS, TP53, SETBP1, U2AF1, TCF3, STAG2, NRAS, JAK2 and BRAF; and (c) if the myelodysplastic syndrome is characterized by the presence of a mutant allele of IDH2 and the absence of a mutant allele of at least one other gene, wherein the other gene is selected from the group consisting of KRAS, TP53, SETBP1, U2AF1, TCF3, STAG2, NRAS, JAK2 and BRAF, identifying the subject as a cancer subject suitable for treatment using the methods described herein. In another example, the subjects identified as subjects suitable for the methods of treatment described herein are treated with COMPOUND 1.

In one example, described herein is a method of identifying a subject suitable for methods of treatment described herein, comprising: (a) obtaining a biological sample from a subject having a myelodysplastic syndrome; (b) screening the biological sample for an IDH2 mutation and the mutation of at least one other gene, wherein the other gene is selected from the group consisting of SETBP1, NRAS, JAK2 and BRAF; and (c) if the myelodysplastic syndrome is characterized by the presence of a mutant allele of IDH2 and the absence of a mutant allele of at least one other gene, wherein the other gene is selected from the group consisting of SETBP1, NRAS, JAK2 and BRAF, identifying the subject as a cancer subject suitable for treatment using the methods described herein. In another example, the subjects identified as subjects suitable for the methods of treatment described herein are treated with COMPOUND 1.

In one example, described herein is a method of identifying a subject suitable for methods of treatment described herein, comprising: (a) obtaining a biological sample from a subject having a myelodysplastic syndrome; (b) screening the biological sample for an IDH2 mutation, a mutation of a second gene, wherein the second gene is selected from the group consisting of ASXL1 and SRSF2, and the absence of a mutant allele of at least one other gene, wherein the other gene is selected from the group consisting of KRAS, TP53, SETBP1, and U2AF1; and (c) if the myelodysplastic syndrome is characterized by the presence of a mutant allele of IDH2, the presence of a mutant allele of a second gene, wherein the second gene is selected from the group consisting of ASXL1 and SRSF2, and the absence of a mutant allele of at least one other gene, wherein the other gene is selected from the group consisting of KRAS, TP53, SETBP1, and U2AF1, identifying the subject as a cancer subject suitable for treatment using the methods described herein. In another example, the subjects identified as subjects suitable for the methods of treatment described herein are treated with COMPOUND 1.

In one example, described herein is a method of identifying a subject suitable for methods of treatment described herein, comprising: (a) obtaining a biological sample from a subject having a myelodysplastic syndrome; (b) screening the biological sample for an IDH2 mutation, a mutation of at least one second gene, wherein the second gene is selected from the group consisting of ASXL1 and SRSF2, and the absence of a mutant allele of at least one other gene, wherein the other gene is selected from the group consisting of TP53, SETBP1, U2AF1, TCF3 STAG2, NRAS, JAK2 and BRAF; and (c) if the myelodysplastic syndrome is characterized by the presence of a mutant allele of IDH2, the presence of a mutant allele of at least one second gene, wherein the second gene is selected from the group consisting of ASXL1 and SRSF2, and the absence of a mutant allele of at least one other gene, wherein the other gene is selected from the group consisting of TP53, SETBP1, U2AF1, TCF3,STAG2, NRAS, JAK2 and BRAF, identifying the subject as a cancer subject suitable for treatment using the methods described herein. In another example, the subjects identified as subjects suitable for the methods of treatment described herein are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2. The method comprises identifying one or more subjects with a myelodysplastic syndrome characterized by mutation of at least one second gene, wherein the second gene is selected from the group consisting of ASXL1 and SRSF2, from the plurality of subjects suitable for treatment with COMPOUND 1. In one example, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2. The method comprises identifying one or more subjects with a myelodysplastic syndrome characterized by mutation of at least one second gene, wherein the second gene is selected from the group consisting of MPL, DNMT3A, CSF3R, FAT3, or CBL, from the plurality of subjects suitable for treatment with COMPOUND 1. In one example, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2 and the absence of a mutant allele of at least one other gene, wherein the other gene is selected from the group consisting of KRAS, TP53, SETBP1, and U2AF1. The method comprises identifying one or more subjects with a myelodysplastic syndrome characterized by the absence of a mutant allele of at least one other gene, wherein the other gene is selected from the group consisting of KRAS, TP53, SETBP1, and U2AF1, from the plurality of subjects suitable for treatment with COMPOUND 1. In one embodiment, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2 and the absence of a mutant allele of at least one other gene, wherein the other gene is selected from the group consisting of KRAS, TP53, SETBP1, U2AF1, TCF3, STAG2, NRAS, JAK2 and BRAF. The method comprises identifying one or more subjects with a myelodysplastic syndrome characterized by the absence of a mutant allele of at least one other gene, wherein the other gene is selected from the group consisting of KRAS, TP53, SETBP1, U2AF1, TCF3, STAG2, NRAS, JAK2 and BRAF, from the plurality of subjects suitable for treatment with COMPOUND 1. In one example, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2 and the absence of a mutant allele of at least one other gene, wherein the other gene is selected from the group consisting of SETBP1, NRAS, JAK2 and BRAF. The method comprises identifying one or more subjects with a myelodysplastic syndrome characterized by the absence of a mutant allele of at least one other gene, wherein the other gene is selected from the group consisting of SETBP1, NRAS, JAK2 and BRAF, from the plurality of subjects suitable for treatment with COMPOUND 1. In one example, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2 and the absence of a mutant allele of at least one other gene, wherein the other gene is selected from the group consisting of TP53, SETBP1, U2AF1, TCF3, STAG2, NRAS, JAK2 and BRAF. The method comprises identifying one or more subjects with a myelodysplastic syndrome characterized by the absence of a mutant allele of at least one other gene, wherein the other gene is selected from the group consisting of TP53, SETBP1, U2AF1, TCF3, STAG2, NRAS, JAK2 and BRAF, from the plurality of subjects suitable for treatment with COMPOUND 1. In one example, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2 and a mutant allele of a second gene, wherein the second gene is selected from the group consisting of ASXL1 and SRSF2. The method comprises identifying one or more subjects with a myelodysplastic syndrome characterized by the absence of a mutant allele of at least one other gene, wherein the other gene is selected from the group consisting of KRAS, TP53, SETBP1, and U2AF1, from the plurality of subjects suitable for treatment with COMPOUND 1. In one example, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2 and a mutant allele of at least one second gene, wherein the second gene is selected from the group consisting of ASXL1 and SRSF2. The method comprises identifying one or more subjects with a myelodysplastic syndrome characterized by the absence of a mutant allele of at least one other gene, wherein the other gene is selected from the group consisting of KRAS, TP53, SETBP1, U2AF1, TCF3, and STAG2 from the plurality of subjects suitable for treatment with COMPOUND 1. In one example, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2 and a mutant allele of at least one second gene, wherein the second gene is selected from the group consisting of ASXL1 and SRSF2. The method comprises identifying one or more subjects with a myelodysplastic syndrome characterized by the presence of a mutant allele of at least one other gene, wherein the other gene is selected from the group consisting of MPL, DNMT3A, CSF3R, FAT3, or CBL from the plurality of subjects suitable for treatment with COMPOUND 1. In one example, one or more suitable subjects are treated with COMPOUND 1.

In another example, described herein is a method for identifying one or more subjects suitable for treatment with COMPOUND 1 using methods provided herein from a plurality of subjects, with a myelodysplastic syndrome characterized by the presence of a mutant allele of IDH2 and a mutant allele of at least one second gene, wherein the second gene is selected from the group consisting of ASXL1 and SRSF2. The method comprises identifying one or more subjects with a myelodysplastic syndrome characterized by the presence of a mutant allele of at least one other gene, wherein the other gene is selected from the group consisting of MPL, DNMT3A, CSF3R, FAT3, or CBL from the plurality of subjects suitable for treatment with COMPOUND 1, and the absence of a mutant allele of at least one additional gene, wherein the additional gene is selected from the group consisting of TP53, SETBP1, U2AF1, TCF3, STAG2, NRAS, JAK2 and BRAF. In one example, one or more suitable subjects are treated with COMPOUND 1.

In certain embodiment, the inhibitory activity of COMPOUND 1 against IDH2 mutants can be tested by methods described in US Publication No. US2013/0190287.

### Patient Population

In certain embodiments of the methods provided herein, the subject to be treated is an animal, for example a mammal or a non-human primate. In particular embodiments, the subject is a human patient. The subject can be male or female.

Particularly, subjects amenable to treatment according to the methods provided herein include subjects with a myelodysplastic syndrome, wherein the myelodysplastic syndrome is characterized by the presence of a mutant allele of IDH2 and a mutant allele of a second gene, wherein the second gene is selected from the group consisting of ASXL1 and SRSF2.

In certain embodiments, subjects amenable to treatment according to the methods provided herein include subjects with a myelodysplastic syndrome, wherein the myelodysplastic syndrome is characterized by the presence of a mutant allele of IDH2 and the absence of a mutant allele of at least one other gene, wherein the other gene is selected from the group consisting of KRAS, TP53, SETBP1, and U2AF1.

In certain examples, subjects amenable to treatment according to the methods provided herein include subjects with a myelodysplastic syndrome, wherein the myelodysplastic syndrome is characterized by the presence of a mutant allele of IDH2 and the absence of a mutant allele of at least one other gene, wherein the other gene is selected from the group consisting of KRAS, TP53, SETBP1, U2AF1, TCF3, STAG2, NRAS, JAK2 and BRAF.

In certain examples, subjects amenable to treatment according to the methods provided herein include subjects with a myelodysplastic syndrome, wherein the myelodysplastic syndrome is characterized by the presence of a mutant allele of IDH2 and the absence of a mutant allele of at least one other gene, wherein the other gene is selected from the group consisting of TP53, SETBP1, U2AF1, TCF3, STAG2, NRAS, JAK2 and BRAF.

In certain examples, subjects amenable to treatment according to the methods provided herein include subjects with a myelodysplastic syndrome, wherein the myelodysplastic syndrome is characterized by the presence of a mutant allele of IDH2 and the absence of a mutant allele of at least one other gene, wherein the other gene is selected from the group consisting of SETBP1, NRAS, JAK2 and BRAF.

In certain embodiments, subjects amenable to treatment with compound 1 for use in a method of treating a myelodysplastic syndrome as provided herein include subjects with a myelodysplastic syndrome, wherein the myelodysplastic syndrome is characterized by the presence of a mutant allele of IDH2 and a mutant allele of a second gene, wherein the second gene is selected from the group consisting of ASXL1 and SRSF2; wherein the myelodysplastic syndrome is further characterized by the absence of a mutant allele of at least one other gene, wherein the other gene is selected from the group consisting of KRAS, TP53, SETBP1, and U2AF1.

In certain examples, subjects amenable to treatment according to the methods provided herein include subjects with a myelodysplastic syndrome, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the absence of a mutant allele of at least one other gene, wherein the other gene is selected from the group consisting of KRAS, TP53, SETBP1, U2AF1, TCF3, and STAG2.

In certain examples, subjects amenable to treatment according to the methods provided herein include subjects with a myelodysplastic syndrome, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the absence of a mutant allele of at least one other gene, wherein the other gene is selected from the group consisting of TP53, SETBP1, U2AF1, TCF3, STAG2, NRAS, JAK2 and BRAF.

In certain embodiments, subjects amenable to treatment with compound 1 for use in a method of treating a myelodysplastic syndrome as provided herein include subjects with a myelodysplastic syndrome, wherein the myelodysplastic syndrome is characterized by the presence of a mutant allele of IDH2 and a mutant allele of at least one second gene, wherein the second gene is selected from the group consisting of ASXL1 and SRSF2; wherein the myelodysplastic syndrome is further characterized by the presence of a mutant allele of at least one other gene, wherein the other gene is selected from the group consisting of MPL, DNMT3A, CSF3R, FAT3, or CBL.

Also encompassed is treating a subject regardless of the subject's age, although some diseases or disorders are more common in certain age groups. In some embodiments, the subject is a human patient at least 18 years old. In some embodiments, the patient is 10, 15, 18, 21, 24, 35, 40, 45, 50, 55, 65, 70, 75, 80, or 85 years old or older.

In certain embodiments, compound 1 for use in a method of treating encompasses the treatment of subjects who have not been previously treated for a myelodysplastic syndrome. In other embodiments, compound 1 for use in a method of treating encompasses treating subjects who have been previously treated but are non-responsive to standard therapies as well as those who are currently being treated for a myelodysplastic syndrome. For example, the subjects may have been previously treated or are currently being treated with a standard treatment regimen for a myelodysplastic syndrome known to the practitioner of skill in the art.

### Examples

As used , the symbols and conventions used in the examples, regardless of whether a particular abbreviation is specifically defined, are consistent with those used in the contemporary scientific literature, for example, the Journal of the American Chemical Society or the Journal of Biological Chemistry. Specifically, but without limitation, the following abbreviations may be used in the examples and throughout the specification: MDS = myelodysplastic syndrome; ASXL1 = Additional Sex Combs Like 1; BRAF = B-Raf Proto-Oncogene, Serine/Threonine Kinase; CBL = Casitas B-Lineage Lymphoma Proto-Oncogene; CSF3R = Colony Stimulating Factor 3 Receptor; DNMT3A = DNA Cytosine-5-Methyltransferase 3 Alpha; FAT3 = FAT Atypical Cadherin 3; JAK2 = Janus kinase 2; KRAS = Kirsten rat sarcoma viral oncogene homolog; mIDH2 = mutant isocitrate dehydrogenase 2; MPL = Myeloproliferative Leukemia Protein; NRAS = Neuroblastoma RAS Viral Oncogene Homolog; SETBP1 = SET Binding Protein 1; SRSF2 = Serine/Arginine-Rich Splicing Factor 2; STAG2 = Stromal Antigen 2; TCF = Transcription Factor 3; TP53 = tumor protein p53; U2AF1 = U2 Small Nuclear RNA Auxiliary Factor 1; CR = complete response; HI = Hematological Improvement; mCR = marrow complete response; NR = No Response; and PR = partial response.

### Example 1. A Phase 1/2, Multicenter, Open-Label, Dose-Escalation and Expansion, Safety, Pharmacokinetic, Pharmacodynamic, and Clinical Activity Study of Orally Administered COMPOUND A in Subjects with Advanced Hematologic Malignancies with an IDH2 Mutation.

### Phase 1 (Dose Escalation and Part 1 Expansion) Objectives:

### Primary Objectives

To assess the safety and tolerability of treatment with COMPOUND A administered continuously as a single agent dosed orally on Days 1 to 28 of a 28-day cycle in subjects with advanced hematologic malignancies.

To determine a maximum tolerated dose (MTD) or a maximum administered dose (MAD) and/or the recommended Phase 2 dose (RP2D) of COMPOUND A in subjects with advanced hematologic malignancies, for example MDS.

### Secondary Objectives

To describe the dose-limiting toxicities (DLTs) of COMPOUND A in subjects with advanced hematologic malignancies, for example MDS.

To characterize the pharmacokinetics (PK) of COMPOUND A and its metabolite in subjects with advanced hematologic malignancies, for example MDS.

To characterize the PK/pharmacodynamic (PD) relationship of COMPOUND A and 2-hydroxygluturate (2-HG).

To characterize the clinical activity associated with COMPOUND A in subjects with advanced hematologic malignancies, for example MDS.

### Phase 2 Objectives:

### Primary Objectives

To assess the efficacy of COMPOUND A as treatment for subjects with relapsed or refractory AML with an IDH2 mutation.

### Secondary Objectives

To further evaluate the safety profile of COMPOUND A in subjects with relapsed or refractory AML with an IDH2 mutation.

To characterize the pharmacokinetics (PK) of COMPOUND A and its metabolite in subjects with relapsed or refractory AML with an IDH2 mutation.

To characterize the PK/pharmacodynamic (PD) relationship of COMPOUND A and 2-hydroxygluturate (2-HG).

### Study Design:

This was a Phase 1/2, multicenter, open-label, 3-part (Phase 1 dose escalation, Phase 1 Part 1 Expansion, and Phase 2), safety, PK/PD, and clinical activity evaluation of orally administered COMPOUND A in subjects with advanced hematologic malignancies, for example MDS, that harbor an IDH2 mutation. The study included a dose escalation phase to determine MTD/MAD and/or RP2D, an expansion phase (Part 1) to further evaluate the safety, tolerability, and clinical activity of COMPOUND A, and a Phase 2 to assess the clinical efficacy of COMPOUND A at the RP2D and to further evaluate safety in subjects with refractory and relapsed AML carrying an IDH2 mutation.

In the Phase 1 portion, the study included a dose escalation phase to determine MTD/MAD and/or the RP2D and an expansion phase (Part 1 Expansion) to further evaluate the safety, tolerability and clinical activity of COMPOUND A in select populations. The Phase 2 portion (previously Part 2 Expansion) further informed on the efficacy, safety, tolerability and clinical activity of COMPOUND A in subjects with refractory or relapsed AML with an IDH2 mutation.

Dose Escalation Phase: The dose escalation phase utilized a standard "3 + 3" design. During the dose escalation phase, consented eligible subjects with relapsed or refractory acute myelogenous leukemia (AML), untreated AML ≥60 years of age who were not candidates for standard therapy, or myelodysplastic syndrome with refractory anemia with excess blasts were enrolled into sequential cohorts of increasing doses of COMPOUND A not to exceed 650 mg QD dose. Each dose cohort enrolled a minimum of 3 subjects. The first 3 subjects enrolled in each dosing cohort during the dose escalation portion of the study received a single dose of study drug on Day -3 (i.e., 3 days prior to the start of daily dosing) and underwent safety and PK/PD assessments over 72 hours to evaluate drug concentrations and 2-HG and α-KG levels. The next dose of study drug was on Cycle 1 Day 1 (C1D1) at which time daily dosing began. The initial dosing schedule was twice daily (approximately every 12 hours). Based on the emerging data, a once daily dosing schedule also has been implemented. Alternative dosing schedules (e.g., a loading dose followed by once daily dosing) could continue to be explored in the dose escalation and expansion phases as agreed upon by the Clinical Study Team. If there were multiple subjects in the screening process at the time the third subject within a cohort began treatment, up to 2 additional subjects might be enrolled with approval of the Medical Monitor. For these additional subjects, the Day -3 through Day 1 PK/PD assessments were optional following discussion with the Medical Monitor.

The safety of dosing during the dose escalation phase was evaluated by the Clinical Study Team, comprised of the Sponsor designee (Responsible Medical Officer), Study Medical Monitor, and Investigators. The Clinical Study Team reviewed the emerging safety data from each cohort to determine if dose escalation should occur.

Toxicity severity was graded according to the National Cancer Institute Common Terminology Criteria for Adverse Events (NCI CTCAE) Version 4.03. A DLT was defined as outlined below.

### Non-hematologic:

All clinically significant non-hematologic toxicities CTCAE ≥Grade 3 with the exception of ≥Grade 3 blood bilirubin increases in subjects with a UDP (uridine diphosphate)-glucuronosyltransferase 1 family, polypeptide A1 (UGT1A1) mutation. In subjects with a UGT1A1 mutation, blood bilirubin increases of >5× upper limit of normal (ULN) might be considered a DLT.

### Hematologic:

Prolonged myelosuppression, defined as persistence of ≥Grade 3 neutropenia or thrombocytopenia (by NCI CTCAE, version 4.03, leukemia-specific criteria, i.e., marrow cellularity <5% on Day 28 or later from the start of study drug without evidence of leukemia) at least 42 days after the initiation of Cycle 1 therapy. Leukemia-specific grading was used for cytopenias (based on percentage decrease from baseline: 50 to 75% = Grade 3, >75% = Grade 4).

Due to frequent co-morbidities and concurrent medications in the population under study, attribution of adverse events (AEs) to a particular drug was challenging. Therefore, all AEs that could not clearly be determined to be unrelated to COMPOUND A were considered relevant to determining DLTs and were reviewed by the Clinical Study Team. The Clinical Study Team also reviewed any other emergent toxicities that were not explicitly defined by the DLT criteria to determine if any warranted a DLT designation.

If, after the third subject completed the 28-day DLT evaluation period (i.e., Cycle 1), no DLTs were observed, the study proceeded with dose escalation to the next cohort following safety review by the Clinical Study Team. If 1 of 3 subjects experienced a DLT during the first cycle, 3 additional subjects were enrolled in that cohort. If none of the additional 3 subjects experienced a DLT, dose escalation continued to the next cohort following safety review by the Clinical Study Team. If 2 or more subjects in a cohort experienced DLTs during the first cycle, dose escalation was halted and the next lower dose level was declared the MTD. If the MTD cohort included only 3 subjects, an additional 3 subjects were enrolled at that dose level to confirm that <2 of 6 subjects experienced a DLT at that dose. Alternatively, a dose level intermediate between the non-tolerated dose level and the previously tolerated dose level might have been explored and declared the MTD if <2 out of 6 subjects experienced a DLT at that dose.

Increases in the dose of COMPOUND A for each dose cohort was guided by an accelerated titration design, where the dose was doubled (100% increase) from one cohort to the next until COMPOUND A-related NCI CTCAE Grade 2 or greater toxicity was observed in any subject within the cohort. Following evaluation by the Clinical Study Team, subsequent increases in dose were 50% or less until the MTD was determined. The absolute percent increase in the dose was determined by the Clinical Study Team predicated on the type and severity of any toxicity seen in the prior dose cohorts. The MTD was the highest dose that causes DLTs in <2 of 6 subjects.

To optimize the number of subjects treated at a potentially clinically relevant dose, intra-subject dose escalation was permitted with approval of the Medical Monitor.

### Part 1 Expansion Phase

During the Part 1 expansion phase, safety, PK/PD, and preliminary clinical activity data were reviewed by the Clinical Study Team on an ongoing basis.

In Part 1, 4 non-randomized cohorts of approximately 25 subjects per arm with IDH2-mutated hematologic malignancies were enrolled as follows:
Arm 1: Relapsed or refractory AML and age ≥60 years, or any subject with AML regardless of age who had relapsed following a bone marrow transplant (BMT).
Arm 2: Relapsed or refractory AML and age <60 years, excluding subjects with AML who had relapsed following a BMT.
Arm 3: Untreated AML and age ≥60 years that declined standard of care chemotherapy.
Arm 4: IDH2-mutated advanced hematologic malignancies not eligible for Arms 1 to 3.

### Phase 2

Phase 2, the pivotal part of the study, further established the efficacy and safety profile of COMPOUND A at the recommended phase 2 dose (RP2D) determined in the ongoing dose escalation phase in subjects with IDH2-mutated relapsed or refractory AML defined as follows:
Subjects who relapsed after allogeneic transplantation;
Subjects in second or later relapse;
Subjects who were refractory to initial induction or re-induction treatment;
Subjects who relapsed within 1 year of initial treatment, excluding patients with favorable-risk status according to NCCN Guidelines (NCCN 2015; NCCN 2015 National Comprehensive Cancer Network. Acute Myelogenous Leukemia (Version 1.2015), http://www.nccn.org/professionals/physician_gls/PDF/aml.pdf). Favorable-risk cytogenetics: inv(16), +(16; 16), t(8;21), t(15; 17).
Approximately 125 subjects were enrolled in this part of the trial.

### General Study Conduct:

Following informed consent, all subjects underwent screening procedures within 28 days prior to the C1D1 to determine eligibility. All subjects were required to have confirmation of IDH2-mutated disease from a bone marrow aspirate and peripheral blood. For subjects in the dose escalation phase and Part 1 Expansion, documentation of IDH2 mutation status could be based on local site testing with central laboratory testing performed retrospectively. Subjects in Phase 2 were required to have IDH2- mutation status based on central laboratory testing during screening prior to study treatment. Additional screening procedures included medical, surgical, and medication history, a buccal swab for germ-line mutation analysis, physical examination, vital signs, Eastern Cooperative Oncology Group (ECOG) performance status (PS), 12-lead electrocardiogram (ECG), evaluation of left ventricular ejection fraction (LVEF), clinical laboratory assessments (hematology, chemistry, coagulation, and serum pregnancy test), bone marrow biopsy and aspirate, blood and bone marrow samples for 2-HG and α-KG measurement, and blood for determination of UGT1A1 mutation status. In addition, subjects in the Part 1 Expansion had urine samples for 2-HG and α-KG measurement and blood samples for cholesterol, and 4β-OH-cholesterol levels collected during screening.

### Dose Escalation and Part 1 Expansion

Three days prior to the start of daily dosing of COMPOUND A (Day -3), the first 3 subjects enrolled in each cohort in the dose escalation phase and the first 15 subjects enrolled in each arm of Part 1 Expansion received a single dose of COMPOUND A in clinic and had serial blood and urine samples obtained for determination of blood and urine concentrations of COMPOUND A, its metabolite (6-(6-(trifluoromethyl)pyridin-2-yl)-N2-(2-(trifluoromethyl)pyridin-4-yl)-1,3,5-triazine-2,4-diamine), 2-HG, and α-KG. A full 72-hour PK/PD profile was conducted: subjects were required to remain at the study site for 10 hours on Day -3 and return on Days -2, -1, and 1 for 24-, 48-, and 72-hour samples, respectively. During the in-clinic period on Day -3, clinical observation and serial 12-lead ECGs and vital signs assessments were conducted. Daily treatment with COMPOUND A began on C1D1; for subjects in the dose escalation phase and Part 1 Expansion who did not undergo the Day -3 PK/PD assessments, clinical observation and serial 12-lead ECGs and vital signs assessments were conducted over 8 hours following their first dose of COMPOUND A on C1D1.

Subjects in the dose escalation phase and Part 1 Expansion also underwent PK/PD assessments over a 10-hour period on C1D15, C2D1, and C4D1. Predose blood samples (trough) were obtained on C1D1 (for those subjects who did not undergo the Day -3 PK/PD assessments), C1D8, C1D22, C2D15, C3D1, C3D15, C5D1, and Day 1 of all cycles thereafter for determination of COMPOUND 1, 2-HG, and α-KG concentrations. These subjects had urine collected for PK/PD evaluation at screening; prior to dosing on C1D15, C2D1 and Day 1 of all cycles thereafter; and at the End of Treatment visit. Available bone marrow biopsy samples also were assessed for 2-HG and α-KG levels.

### Phase 2

Subjects in the Phase 2 portion of the trial were not required to undergo the Day -3 assessments; these subjects underwent an 8-hour PK/PD profile conducted on Day 1 of Cycles 1 and 2, and predose blood samples (trough) on C1D2 and C2D2 were obtained in order to assess PK/PD in a 24-hour period. Additional blood samples for PK/PD assessments were drawn predose (within 30 minutes) on Day 1 of Cycle 3, and at the End of Treatment visit. Time-matched 12-lead ECGs were conducted in triplicate on Day 1 of Cycles 1 and 2; a triplicate ECG was also obtained at the End of Treatment visit. Single 12-lead ECGs were conducted on Day 1 of every cycle beginning with Cycle 3, and at the Follow-up visit. Available bone marrow biopsy samples were assessed for 2-HG and α-KG levels.

### Other Safety Assessments (All Phases)

All subjects underwent safety assessments during the treatment period to include physical examination, vital signs, ECOG PS, 12-lead ECGs, evaluation of LVEF, and clinical laboratory assessments (hematology, chemistry, coagulation, and pregnancy testing).

### Clinical Activity Assessments:

### Phase 1 (Dose Escalation and Part 1 Expansion)

Subjects in the dose escalation phase and Part 1 Expansion had the extent of their disease assessed, including bone marrow biopsies and/or aspirates and peripheral blood, at screening, on C1D15, C2D1, and C3D1, every 28 days (peripheral blood only) or every 56 days (bone marrow biopsies and/or aspirates and peripheral blood) thereafter while on study drug treatment, independent of dose delays and/or dose interruptions, and/or at any time when progression of disease was suspected. Response to treatment and treatment decisions in all subjects was determined by the Investigators based on modified International Working Group (IWG) response criteria or other appropriate response criteria for the malignancy under study. *See* Cheson et. al. J. Clin. Oncol. 21(24):4642-9 (2003).

### Phase 2

For subjects enrolled in the Phase 2 portion of the trial, extent of disease, including bone marrow biopsies and/or aspirates and peripheral blood, were assessed at screening, on C2D1, every 28 days thereafter through 12 months, and every 56 days thereafter while on study drug treatment, independent of dose delays and/or dose interruptions, and/or at any time when progression of disease was suspected. Eligibility, treatment decisions, and response to treatment was determined by the Investigators based on modified International Working Group (IWG) response criteria. Response was also assessed retrospectively by an Independent Response Adjudication Committee (IRAC).

### End of Treatment and Follow-up:

Subjects might continue treatment with COMPOUND A until disease progression or development of unacceptable toxicity.

Evidence supports that cancer-associated IDH mutations block normal cellular differentiation and promote tumorigenesis via the abnormal production of 2-HG, a potential oncometabolite. COMPOUND A might produce antitumor effects by reversing the differentiation block induced by the IDH2 mutations and promoting appropriate cellular differentiation.

Because of the unique mechanism of action of COMPOUND A, clinical responses were different than those observed with cytotoxic agents. Responses with COMPOUND A might occur after 2 or more cycles of therapy and they might occur after an initiation period of leukocytosis in the peripheral blood and/or bone marrow with, in rare cases, corresponding clinical signs and symptoms of fever, fluid retention, hypoxia, and skin rash which had been termed a differentiation-like syndrome.

As such, standard assessment criteria developed based on the experience from the cytotoxic chemotherapeutic agents did not provide a complete and accurate response assessment for this novel class of IDH2 inhibitors. Therefore, in the setting where a subject's assessment showed signs similar to progression within the first 2 cycles, caution was exercised in discontinuing study drug, and a discussion with the Medical Monitor was required, especially in situations where the subject's clinical condition was stable as supported by, but limited to, absence of signs and symptoms of rapid deterioration indicating disease progression and/or general condition was stable or improving.

Subjects who experienced progression of disease (PD) per the applicable response criteria, had assessment of the disease repeated 28 days later in order to confirm PD with option of continuing treatment as described above while awaiting for confirmation. If repeat evaluation confirmed PD subjects discontinued study treatment and proceeded to the survival follow-up phase.

Subjects with stable or progressive disease could continue to receive study treatment with COMPOUND A at the discretion of the Investigator and with Medical Monitor approval.

All subjects underwent an end of treatment assessment (within approximately 5 days of the last dose of study drug); in addition, a follow-up safety assessment was scheduled 28 days after the last dose. Furthermore, all subjects were followed monthly for disease status, overall survival, and initiation of non-study anti-neoplastic therapy, until death, withdrawal of consent, or the end of the study, whichever occured first.

Subjects who achieved an adequate response to treatment with COMPOUND A and met other criteria required to undergo hematopoietic stem cell transplant (HSCT) could proceed to HSCT after discontinuation of study therapy. Those subjects were followed on study for outcome until relapse or end of study to support the overall clinical benefit of COMPOUND A in this setting.

Subjects who relapsed following HSCT might be eligible to restart treatment with COMPOUND A with Medical Monitor approval and at the discretion of the Investigator, if they had confirmed recurrent IDH2 mutant positive disease, no other cancer treatment (with the exception of anti-neoplastic therapies used in the course of HSCT such as conditioning regimen or induction-type regimen and anti-GVHD prophylaxis [i.e., methotrexate]) besides HSCT was administered since the last dose of COMPOUND A, the subject met the safety parameters listed in the Inclusion/Exclusion criteria, and the trial was open. Subjects resumed COMPOUND A therapy at the same dose and schedule at the time of COMPOUND A treatment discontinuation prior to HSCT.

All subjects, including those who relapsed following HSCT and elected not to restart treatment, were followed monthly thereafter for assessment of survival status and non-study anti-neoplastic therapies since discontinuation of study drug until death or end of study.

### Number of subjects (planned):

Approximately a minimum of 291 subjects in total was planned to be enrolled in the study (i.e., in the dose escalation, Part 1 Expansion, and Phase 2 portion of the trial).

Assuming that identification of the MTD/MAD required the evaluation of 13 dose levels/schedules of COMPOUND A with up to 5 subjects per dose level, with the exception that the MTD/MAD required 6 subjects, then 66 subjects were enrolled during the dose escalation part of the study. Additional subjects might be needed for cohort expansion during dose escalation, for the replacement of subjects who were not evaluable for PK/PD, safety, or clinical activity, or for evaluation of alternative dosing regimens other than the planned escalation scheme or the MTD/MAD, to optimize the RP2D and regimen(s). 5 dose levels (ranging from 30 mg to 150 mg) had been evaluated in the BID schedule and 8 dose levels (ranging from 50 mg to 650 mg) had been evaluated in the QD schedule.

Four cohorts of a minimum of 25 additional subjects in specific hematologic malignancy subsets (total a minimum of 100 subjects) were enrolled in Part 1 Expansion of the study.

The Phase 2 portion of the trial enrolled approximately 125 subjects with relapsed or refractory AML with an IDH2 mutation. Additional subjects might be needed for the replacement of subjects who were not evaluable for PK/PD, safety, and/or clinical activity, or for evaluation of alternative dosing regimens. The final total sample size might be adjusted according to the observed toxicity rate, and number of subjects enrolled for expanded evaluation.

### Inclusion Criteria

Subjects had to meet all of the following criteria to be enrolled in the study:
1. Subject had to be ≥18 years of age.
2. Subjects had to have advanced hematologic malignancy including:
   **Phase 1/ Dose escalation:**
      - Diagnosis of AML according to World Health Organization (WHO) criteria (Swerdlow et. al. WHO Classification of Tumors of Haematopoietic and Lymphoid Tissues. 4th ed. Lyon, France: IARC Press; 2008; 109-139);
         ∘ Disease refractory or relapsed (defined as the reappearance of > 5% blasts in the bone marrow).
         ∘ Untreated AML, ≥60 years of age and were not candidates for standard therapy due to age, performance status, and/or adverse risk factors, according to the treating physician and with approval of the Medical Monitor;
      - Diagnosis of MDS according to WHO classification with refractory anemia with excess blasts (subtype RAEB-1 or RAEB-2), or considered high-risk by the Revised International Prognostic Scoring System (IPSS-R; Greenberg et. al. Blood. 120(12):2454-65 (2012)) that was recurrent or refractory, or the subject was intolerant to established therapy known to provide clinical benefit for their condition (i.e., subjects had to not be candidates for regimens known to provide clinical benefit), according to the treating physician and with approval of the Medical Monitor. (Subjects with other relapsed and/or primary refractory hematologic cancers, for example CMML, who fulfilled the inclusion/excluding criteria might be considered on a case-by case basis, with approval of the Medical Monitor.)
   **Phase 1/ Part 1 Expansion:**
      - Arm 1: Relapsed or refractory AML and age ≥60 years, or any subject with AML regardless of age who had relapsed following a BMT.
      - Arm 2: Relapsed or refractory AML and age <60 years, excluding subjects with AML who had relapsed following a BMT.
      - Arm 3: Untreated AML and age ≥60 years that declined standard of care chemotherapy.
      - Arm 4: IDH2-mutated advanced hematologic malignancies not eligible for Arms 1 to 3.
   **Phase 2:**
      - Diagnosis of AML according to World Health Organization (WHO) criteria and disease relapsed or refractory as defined by:
         ∘ Subjects who relapsed after allogeneic transplantation;
         ∘ Subjects in second or later relapse;
         ∘ Subjects who were refractory to initial induction or re-induction treatment;
         ∘ Subjects who relapsed within 1 year of initial treatment, excluding patients with favorable-risk status according to NCCN Guidelines. Favorable-risk cytogenetics: inv(16), +(16; 16), t(8;21), t(15; 17).
3. Subjects had to have documented IDH2 gene-mutated disease:
   - For subjects in the dose escalation phase and Part 1 Expansion, IDH2 mutation might be based on local evaluation. (Centralized testing was performed retrospectively).
4. For subjects in the Phase 2 portion of the trial, central testing of IDH2 mutation in samples of bone marrow aspirate and peripheral blood, was required during screening to confirm eligibility. Subjects had to be amenable to serial bone marrow sampling, peripheral blood sampling, and urine sampling during the study.
   - The diagnosis and evaluation of AML or MDS was made by bone marrow aspiration and biopsy. If an aspirate was unobtainable (i.e., a "dry tap"), the diagnosis might be made from the core biopsy.
   - Screening bone marrow aspirate and peripheral blood samples were required for all subjects. A bone marrow biopsy had to be collected if adequate aspirate was not attainable unless:
      ∘ A bone marrow aspirate and biopsy was performed as part of the standard of care within 28 days prior to the start of the study treatment; and
      ∘ Slides of bone marrow aspirate, biopsy and stained peripheral blood smear were available for both local and central pathology reviewers;
5. Subjects had to be able to understand and willing to sign an informed consent. A legally authorized representative could consent on behalf of a subject who is otherwise unable to provide informed consent, if acceptable to, and approved by, the site and/or site's Institutional Review Board (IRB)/Independent Ethic Committee (IEC).
6. Subjects must have ECOG PS of 0 to 2 (Oken et. al. Am. J. Clin. Oncol. 5:649-655 (1982)).
7. Platelet count ≥20,000/µL (Transfusions to achieve this level were allowed.) Subjects with a baseline platelet count of <20,000µL due to underlying malignancy were eligible with Medical Monitor approval.
8. Subjects had to have adequate hepatic function as evidenced by:
   - Serum total bilirubin ≤1.5 × upper limit of normal (ULN), unless considered due to Gilbert's disease, a gene mutation in UGT1A1, or leukemic organ involvement, following approval by the Medical Monitor;
   - Aspartate aminotransferase (AST), alanine aminotransferase (ALT), and alkaline phosphatase (ALP) ≤3.0 × ULN, unless considered due to leukemic organ involvement.
9. Subjects had to have adequate renal function as evidenced by:
   - Serum creatinine <2.0 × ULN OR
   - Creatinine clearance >40 mL/min based on the Cockroft-Gault glomerular filtration rate (GFR) estimation: (140 - Age) × (weight in kg) × (0.85 if female)/72 × serum creatinine
10. Subjects had to be recovered from any clinically relevant toxic effects of any prior surgery, radiotherapy, or other therapy intended for the treatment of cancer. (Subjects with residual Grade 1 toxicity, for example Grade 1 peripheral neuropathy or residual alopecia, were allowed with approval of the Medical Monitor.)
11. Female subjects with reproductive potential had to agree to undergo medically supervised pregnancy test prior to starting study drug. The first pregnancy test was performed at screening (within 7 days prior to first study drug administration), and on the day of the first study drug administration and confirmed negative prior to dosing and Day 1 before dosing all subsequent cycles.
12. Female subjects with reproductive potential had to have a negative serum pregnancy test within 7 days prior to the start of therapy. Subjects with reproductive potential were defined as sexually mature women who had not undergone a hysterectomy, bilateral oophorectomy or tubal occlusion or who had not been naturally postmenopausal (i.e., who had not menstruated at all) for at least 24 consecutive months (i.e., had had menses at any time in the preceding 24 consecutive months). Females of reproductive potential as well as fertile men and their partners who were female of reproductive potential had to agree to abstain from sexual intercourse or to use two highly effective forms of contraception from the time of giving informed consent, during the study and for 120 days (females and males) following the last dose of COMPOUND A. A highly effective form of contraception was defined as hormonal oral contraceptives, injectables, patches, intrauterine devices, double-barrier method (e.g., synthetic condoms, diaphragm, or cervical cap with spermicidal foam, cream, or gel), or male partner sterilization.
13. Were able to adhere to the study visit schedule (i.e., clinic visits at the study sites were mandatory, unless noted otherwise for particular study visits) and other protocol requirements.

### Exclusion Criteria

Subjects who met any of the following criteria were not enrolled in the study:
1. Subjects who had undergone a hematopoietic stem cell transplant (HSCT) within 60 days of the first dose of COMPOUND A, or subjects on immunosuppressive therapy post HSCT at the time of screening, or with clinically significant graft-versus-host disease (GVHD). (The use of a stable dose of oral steroids post HSCT and/or topical steroids for ongoing skin GVHD was permitted with Medical Monitor approval.)
2. Subjects who received systemic anticancer therapy or radiotherapy <14 days prior to their first day of study drug administration. (Hydroxyurea was allowed prior to enrollment and after the start of COMPOUND A for the control of peripheral leukemic blasts in subjects with leukocytosis (white blood cell [WBC] counts >30,000/µL).
3. Subjects who received a small molecule investigational agent <14 days prior to their first day of study drug administration. In addition, the first dose of COMPOUND A had to not occur before a period ≥5 half-lives of the investigational agent has elapsed.
4. Subjects taking the following sensitive CYP substrate medications that had a narrow therapeutic range were excluded from the study unless they could be transferred to other medications within ≥5 half-lives prior to dosing: paclitaxel (CYP2C8) warfarin, phenytoin (CYP2C9), S-mephenytoin (CYP2C19), thioridazine (CYP2D6), theophylline and tizanidine (CYP1A2).
5. Subjects taking the P-gp and BCRP transporter-sensitive substrates digoxin and rosuvastatin had to be excluded from the study unless they could be transferred to other medications within ≥5 half-lives prior to dosing.
6. Subjects for whom potentially curative anticancer therapy was available.
7. Subjects who were pregnant or lactating.
8. Subjects with an active severe infection that required anti-infective therapy or with an unexplained fever >38.5°C during screening visits or on their first day of study drug administration (at the discretion of the Investigator, subjects with tumor fever could have been enrolled).
9. Subjects with known hypersensitivity to any of the components of COMPOUND A.
10. Subjects with New York Heart Association (NYHA) Class III or IV congestive heart failure or LVEF <40% by echocardiogram (ECHO) or multi-gated acquisition (MUGA) scan obtained within approximately 28 days of C1D1.
11. Subjects with a history of myocardial infarction within the last 6 months of screening.
12. Subjects with uncontrolled hypertension (systolic blood pressure [BP] >180 mmHg or diastolic BP >100 mmHg) at screening were excluded. Subjects requiring 2 or more medications to control hypertension were eligible with Medical Monitor approval.
13. Subjects with known unstable or uncontrolled angina pectoris.
14. Subjects with a known history of severe and/or uncontrolled ventricular arrhythmias.
15. Subjects with a QTcF (QT corrected based on Fridericia's equation) interval ≥450 msec or other factors that increase the risk of QT prolongation or arrhythmic events (e.g., heart failure, hypokalemia, family history of long QT interval syndrome) at screening. Subjects with bundle branch block and a prolonged QTc interval had to be reviewed by the Medical Monitor for potential inclusion.
16. Subjects taking medications that were known to prolong the QT interval unless they could be transferred to other medications within ≥5 half-lives prior to dosing.
17. Subjects with known infection with human immunodeficiency virus (HIV) or active hepatitis B or C.
18. Subjects with any other medical or psychological condition, deemed by the Investigator to be likely to interfere with a subject's ability to sign informed consent, cooperate, or participate in the study.
19. Subjects with known dysphagia, short-gut syndrome, gastroparesis, or other conditions that limit the ingestion or gastrointestinal absorption of drugs administered orally.
20. Subjects with clinical symptoms suggesting active central nervous system (CNS) leukemia or known CNS leukemia. Evaluation of cerebrospinal fluid was only required if there was a clinical suspicion of CNS involvement by leukemia during screening.
21. Subjects with immediately life-threatening, severe complications of leukemia such as uncontrolled bleeding, pneumonia with hypoxia or shock, and/or disseminated intravascular coagulation.
22. In the Phase 2 portion of the trial only, subjects who had previously received treatment with an inhibitor of IDH2.

### Investigational product, dosage and mode of administration:

COMPOUND A (mesylate salt of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol) was provided as 5, 10, 25, 50, 100, 150 and 200 mg free-base equivalent strength tablets to be administered orally.

### Phase 1/Dose Escalation

The first 3 subjects in each cohort in the dose escalation portion of the study and the first 15 subjects in each arm of Part 1 Expansion received a single dose of study drug on Day -3; their next dose of study drug was administered on C1D1 at which time subjects started daily dosing on Days 1 to 28 in 28-day cycles. Starting with C1D1, dosing is continuous; there were no inter-cycle rest periods. Subjects who were not required to undergo the Day -3 PK/PD assessments initiated daily dosing with COMPOUND A on C1D1.

Subjects were required to fast (water is allowed) for 2 hours prior to study drug administration and for 1 hour following study drug administration.

The dose of COMPOUND A administered to a subject was dependent upon which dose cohort is open for enrollment when the subject qualified for the study. The starting dose of COMPOUND A administered to the first cohort of subjects was 30 mg administered orally twice a day, and the maximum administered dose of COMPOUND A administered was 650 mg administered orally once a day.

### Phase 1/Part 1 Expansion and Phase 2

The starting dose of COMPOUND A recommended for evaluation was 100 mg QD. This was based on the safety, PK, pharmacodynamics and clinical activity of COMPOUND A observed to date in AG221-C-001. Evaluation of pharmacodynamic response demonstrated sustained reduction in 2-HG plasma levels by Day 1 of Cycle 2 and up to 98% inhibition in most subjects with R140Q mutation at all doses. Increasing dose was associated with higher exposure and inhibition of 2-HG in subjects with R172K mutation. Importantly, preliminary efficacy data of the 44 subjects treated at 100 mg QD has shown an overall response rate of 36.4%. Thus a dose of 100 mg should adequately achieve inhibition of 2-HG in subjects with either R140Q or R172K mutation. Moreover, the safety profile at 100 mg, including ≥ Grade 3, was consistent with that of lower doses.

Intra-subject dose escalation was possible.

### Duration of treatment:

Subjects could continue treatment with COMPOUND A until disease progression or development of unacceptable toxicity. Subjects who experienced disease progression per the applicable response criteria who were, in the opinion of the Investigator, benefiting from treatment could be allowed to continue on study drug with approval of the Medical Monitor.

### End of study:

End of study was defined as the time at which :
- all subjects had discontinued treatment with COMPOUND A and had been followed for survival for at least 12 months, or have died, been lost to follow up, or withdrew consent prior to at least 12 months of follow-up
- or the date of receipt of the last data point from the last subject that was required for primary, secondary and/or exploratory analysis, as pre-specified in the protocol and/or the Statistical Analysis Plan (SAP), whichever was the later date.

### Criteria for evaluation:

### Safety:

Monitoring of AEs, including determination of DLTs, serious adverse events (SAEs), and AEs leading to discontinuation; safety laboratory parameters; physical examination findings; vital signs; 12-lead ECGs; LVEF; and ECOG PS.

The severity of AEs was assessed by the NCI CTCAE, Version 4.03.

### Pharmacokinetics and Pharmacodynamics:

Serial blood sampling for determination of concentration-time profiles of COMPOUND A and its metabolite (6-(6-(trifluoromethyl)pyridin-2-yl)-N2-(2-(trifluoromethyl)pyridin-4-yl)-1,3,5-triazine-2,4-diamine). Urine sampling for determination of concentrations of COMPOUND A and its metabolite (6-(6-(trifluoromethyl)pyridin-2-yl)-N2-(2-(trifluoromethyl)pyridin-4-yl)-1,3,5-triazine-2,4-diamine) (dose escalation and Part 1 expansion subjects only). Blood and bone marrow sampling for determination of 2-HG and α-KG levels.

### Clinical Activity:

Serial blood and bone marrow sampling to determine response to treatment based on modified IWG response criteria or other appropriate response criteria based on the malignancy under study.

### Statistical methods:

Overall response rate (ORR), the primary efficacy endpoint, was defined as the rate of responders including complete remission (CR), CR with incomplete platelet recovery (CRp), marrow CR (mCR) (morphologic leukemia-free state [MLFS] for subjects with AML), CR with incomplete hematologic recovery (CRi), and partial remission (PR). Other measures of clinical activity including complete remission rate (CRR), duration of remission/response, event-free survival, overall survival, and time to remission/response was summarized.

For Phase 1 Dose Escalation/Part 1 Expansion, the efficacy analysis of response rates as assessed by the site Investigators using modified International Working Group (IWG) response criteria was conducted in Full Analysis Set for each dose level, expansion arm, and overall if appropriate. The analysis of Part 1 expansion arms could also include subjects from the dose-escalation phase who received the same dose/regimen as subjects in the expansion arms and who met the eligibility criteria of individual arms.

For Phase 2 portion of the trial, the primary efficacy analysis of COMPOUND A was determined by the Investigators based on modified International Working Group (IWG) response criteria. Response was also be assessed retrospectively by an Independent Response Adjudication Committee (IRAC) using the Full Analysis Set (FAS). Key supportive analyses was based on independent central review of response in FAS.

### Example 2. Study of COMPOUND A monotherapy in patients with MDS and mIDH2.

A subset of the clinical samples from the trial described in Example 1 were analyzed in this Example.

IDH2 mutations (mIDH2) are recurrent in ~5% of patients (pts) with MDS and ~15% of pts with acute myeloid leukemia (AML). mIDH2 proteins have neomorphic enzymatic activity and are associated with DNA and histone hypermethylation, altered gene expression, and blocked differentiation of hematopoietic progenitor cells. COMPOUND A is a small-molecule allosteric inhibitor of mIDH2 protein that induces hematological responses in pts with mIDH2-positive AML, including relapsed or refractory (R/R) AML. The current analysis is to evaluate the safety and clinical efficacy of COMPOUND A monotherapy in pts with MDS and mIDH2.

Methods: This analysis included pts ages ≥18 years with mIDH2-positive MDS who participated in a phase 1 study (see Example 1) with a dose-finding (dose-escalation) period followed by an expansion phase in which all pts received daily oral COMPOUND A 100 mg QD in 28-day cycles. Pts were relapsed or refractory to prior MDS therapy (Tx) or were not candidates for standard therapies. Response was measured using peripheral blood (PB) and bone marrow (BM) samples on days 15, 29, 57, and every 56 days thereafter, and by objective investigator report. Overall response rate (ORR) reflected the best response achieved by individual pts, including complete remission (CR), partial remission (PR), marrow CR (mCR), and any hematologic improvement (HI) (IWG 2006 MDS criteria). Cheson et al., Blood. 108:419-425 (2006). Evaluable pts required a response assessment at Cycle 2 Day 1 or later, or discontinued before assessment. Overall survival (OS) was estimated using Kaplan-Meier methods. Next-generation sequencing identified pre-existing co-occurring genomic alterations using the FoundationOne Heme test on purified mononuclear cells from BM or PB, to assess relationships between co-mutational status and clinical response. *See, e.g.,* Foundation Medicine, FoundationOne® Heme, Techical Information and Test Overview, available at http://foundationone.com/docs/FM_TechnicalOverviewHEM-I-001- 20150105.pdf?_hstc=197910000.0f2b8af9a5295b09fb8f9c286e3d9952.1466609126039.14697 25363136.1469808635757.8&_hssc=197910000.5.1469808635757&_hsfp=956007364 and http://foundationone.com/docs/ONE-B-001-20140430.pdf (last visited Aug. 2, 2016).

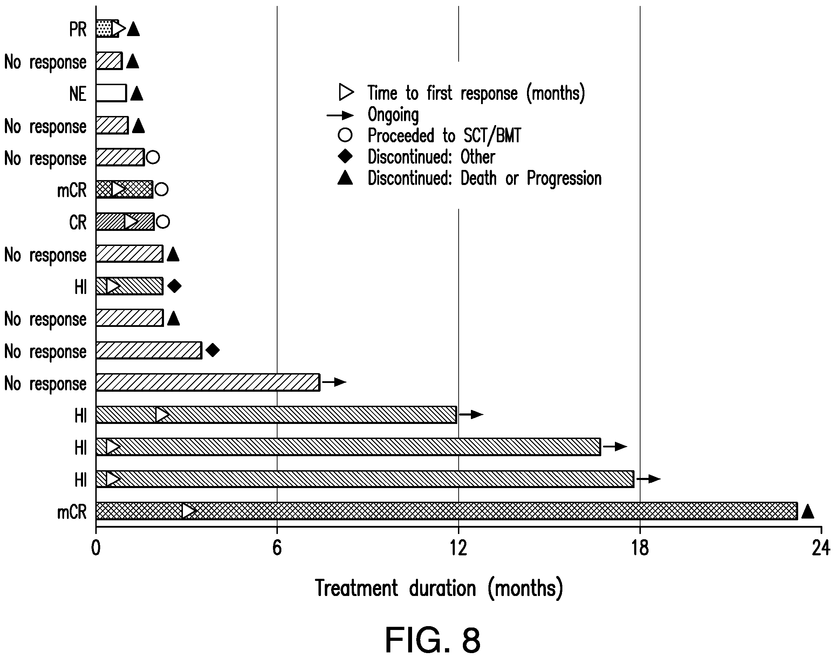

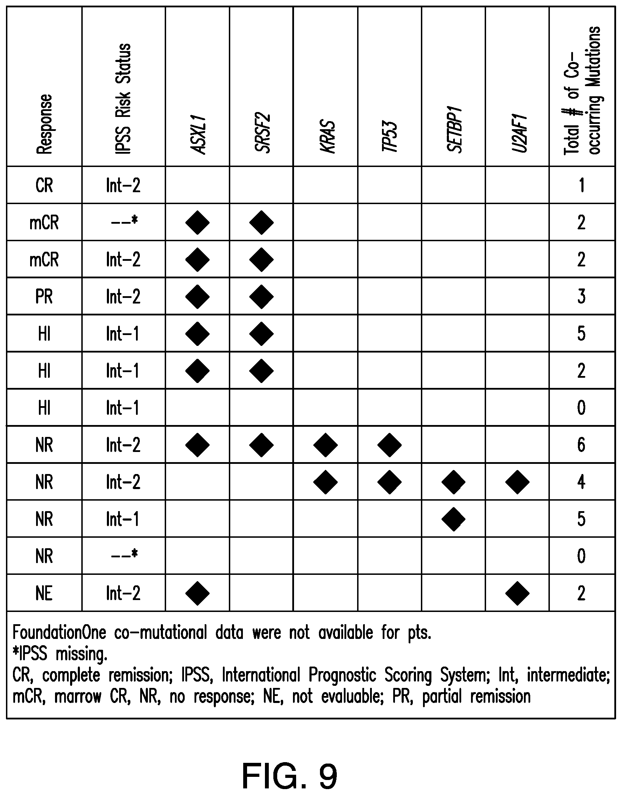

Results: Of the 16 patients with MDS in the study, 12 pts had discontinued and 4 pts continued to receive COMPOUND A at interim database lock. Reasons for discontinuation included disease progression (n=1), adverse event (AE; n=1), death (n=4), investigator decision (n=2), and other (n=1). Three pts proceeded to transplant. Median age was 67 years (range 45-*78) (see* **Table 7).** R140 mutations were more common than R172 mutations (88% vs 12%). At entry, 3 pts (19%) had relapsed following allogeneic stem cell transplant and 11 (69%) had failed prior therapy (Tx) with a hypomethylating agent (HMA). Six pts (38%) had received ≥2 prior anticancer Tx for MDS. MDS pts in the dose-finding phase received daily COMPOUND A doses of 60 mg (n=1), 150 mg (1), 200 mg (3), or 300 mg (1); 10 pts received COMPOUND A 100 mg QD. Median number of Tx cycles was 3 (range 1-25); 5 pts (31%) received ≥6 COMPOUND A cycles and 4 pts (25%) received ≥12 cycles. Grade 3-4 treatment-emergent AEs (TEAEs) were reported for 13 pts (81%); the most frequent were hyperbilirubinemia (n=5, unconjugated), pneumonia (n=4), thrombocytopenia (n=3) and hypokalemia (n=3). Seven pts (44%) had a grade 3-4 drug-related TEAE. One pt was not evaluable for response. ORR was 53% (8/15), including 1 pt who achieved CR *(see* **Table 8** and **Figure 8** (which illustrates duration of treatment, time to first response, best response, and study outcomes)). Of 10 evaluable pts who had received prior HMA Tx, 5 (50%) had a response with COMPOUND A, including the pt in CR. Responses for the 4 pts with no prior MDS Tx were a PR and mCR (1 each). Median time to CR, PR, or mCR (sustained ≥4 weeks) was 24 days (range 17-87) from beginning COMPOUND A Tx, and of HI (sustained ≥8 weeks) was 11 days (range 11-60). Two pts were reported as having disease progression while on Tx. Median OS was also not reached after a median follow-up of 4.7 months. FoundationOne data were available for 12 pts; the most frequently observed known somatic co-occurring mutations in these MDS pts were ASXL1 and SRSF2. *(see* **Figure 9** which illustrates most frequent co-occurring known somatic gene mutations, sorted by response).

Discussion: Daily Tx with oral COMPOUND A monotherapy was well tolerated and induced responses in more than one-half of these MDS pts with mIDH2, 50% of whom had higher-risk disease, and two-thirds of whom had failed prior HMA Tx. Notably, one-half of evaluable MDS pts who had failed prior HMA Tx had a response, including a CR, with COMPOUND A monotherapy. Only 2 pts experienced disease progression during Tx. Mutational testing is rapidly becoming essential to diagnosis and prognostication in MDS, and assessment of IDH2 mutations can identify MDS pts who may benefit from targeted Tx with COMPOUND A.

**Table 7. Baseline characteristics of MDS patients treated with COMPOUND A**

| **Characteristic** | | **MDS Patients (N=16)** |
|---|---|---|
| **Age (years),** median (range) | | 67 (45, 78) |
| **Gender,** % male / % female | | 73 / 27 |
| **IDH2 mutation,** % R140, % R172 | | 88 / 12 |

| **ECOG performance status,** n (%) | | |
|---|---|---|
| | 0-1 | 12 (75) |
| | 2 | 4 (25) |

| **Number of prior anti-cancer regimens,** n (%) | | |
|---|---|---|
| | 0 | 4 (25) |
| | 1 | 6 (38) |
| | ≥2 | 6 (38) |

| **Type of prior MDS treatment,** n (%) | | |
|---|---|---|
| | Hypomethylating agents | 11 (69) |
| | Lenalidomide | 2 (13) |
| | Others* | 5 (31) |
| | Untreated | 4 (25) |
| **Time since diagnosis (months),** mean [SD] | | 15.7 [10.4] |

| **IPSS risk status,** n (%) | | |
|---|---|---|
| | Low / Intermediate-1 | 5 (31) |
| | Intermediate-2 / High | 8 (50) |
| | Missing | 3 (19) |

| **IPSS-R risk status,** n (%) | | |
|---|---|---|
| | Very Low / Low | 3 (19) |
| | Intermediate | 2 (13) |
| | High / Very High | 8 (50) |
| | Missing | 3 (19) |
| **ANC (10⁹/L),** median (min, max) | | 0.7 (0.2, 32.1) |
| **Platelets (10⁹/L),** median (min, max) | | 64 (19, 246) |
| **WBC (10⁹/L),** median (min, max) | | 2.0 (0.5, 44.4) |
| **Hemoglobin (g/dL),** median (min, max) | | 8.8 (7.3, 12.2) |
| *Vosaroxin, pracinostat, cytarabine and clofarabine, ruxolitinib, and sorafenib (n=1 pt each), ECOG, Eastern Cooperative Oncology Group (*see* Oken et. al, Am. J. Clin. Oncol. 5:649-655, (1982)); MDS, myelodysplastic syndromes; IPSS, International Prognostic Scoring System (*see* Greenberg et al., Blood. 89(6):2079-2088 (1997)); IPSS-R, revised IPSS; ANC, absolute neutrophil count; WBC, white blood cells | | |

**Table 8. Overall response**

| | | **MDS Patients (N=16) n (%)** |
|---|---|---|
| **Overall response rate (CR + PR + mCR + HI)** | | **8/15 (53)** |
| Best response | | |
| | Complete Remission* | 1/9 (11) |
| | Partial Remission* | 1/9 (11) |
| | Marrow CR* | 2/9 (22) |
| | Hematologic Improvement | 4/15 (27) |
| Not Evaluable^{†} | | 1 (6) |
| *Evaluable pts had ≥5% BM blasts at baseline (or study entry) | | |
| ^{†}Pt died before beginning cycle 2 | | |
| CR, complete remission; PR, partial remission; mCR, marrow CR; | | |
| HI, hematologic improvement | | |

### Example 3. Study of COMPOUND A monotherapy in patients with MDS and mIDH2

A subset of the clinical samples from the trial described in Example 1 were analyzed in this Example.

Methods: This analysis included patients ages ≥18 years with mIDH2-positive MDS who participated in a phase 1 study (see Example 1) with a dose-finding (dose-escalation) period followed by an expansion phase in which all patients received daily oral COMPOUND A 100 mg QD in 28-day cycles. Patients were relapsed or refractory to prior MDS therapy (Tx) or were not candidates for standard therapies. Response was measured using peripheral blood (PB) and bone marrow (BM) samples on days 15, 29, 57, and every 56 days thereafter, and by objective investigator report. Overall response rate (ORR) reflected the best response achieved by individual patients, including complete remission (CR), partial remission (PR), marrow CR (mCR), and any hematologic improvement (HI) (IWG 2006 MDS criteria). Cheson et al., Blood. 108:419-425 (2006). Evaluable patients required a response assessment at Cycle 2 Day 1 or later, or discontinued before assessment. Overall survival (OS) was estimated using Kaplan-Meier methods. Next-generation sequencing identified pre-existing co-occurring genomic alterations using the FoundationOne Heme test on purified mononuclear cells from BM or PB, to assess relationships between co-mutational status and clinical response. *See, e.g.,* Foundation Medicine, FoundationOne® Heme, Techical Information and Test Overview, available at http://foundationone.com/docs/FM_TechnicalOverview_HEM-I-001 - 20150105.pdf?_hstc=197910000.0f2b8af9a5295b09fb8f9c286e3d9952.1466609126039.14697 25363136.1469808635757.8&_hssc=197910000.5.1469808635757&_hsfp=956007364 and http://foundationone.com/docs/ONE-B-001-20140430.pdf (last visited Aug. 2, 2016).

Results: For the 17 patients with MDS in the study, Median age was 67 years (range 45-78) *(see* **Table 9).** R140 mutations were more common than R172 mutations (88% vs 12%). Out of 17 patients, 13 (76%) had Eastern Cooperative Oncology Group (ECOG) performance of 0-1, and 4 (24%) had ECOG performance of 2. At entry, 4 (24%) patients had no prior anti-cancer therapy, 7 (41%) patients had undergone one prior anti-cancer regimen, and 6 (35%) patients had undergone more than two prior anti-cancer regimens. The patients had undergone the following prior anti-cancer treatments: 13 (76%) patients had been previously treated with a hypomethylating agent (HMA), 2 (12%) patients had been treated with lenalidomide, and 8 (47%) patients had been treated previously teated with other anticancer agents (Sorafenib (2 patients), vosaroxin (1 patient), procrit (1 patient), pracinostat (1 patient), cytarabine + clofarabine (1 patient), ruxolitinib (1 patient), rigosertib (1 patient), and 4 patients had not undergone any prior anti-cancer treatement. Mean time since diagnosis for the patients was 16.8 months (SD = 14.5). Additional baseline characteristics of MDS patients treated with COMPOUND A are provided in **Table 9.**

**Table 9. Baseline characteristics of MDS patients treated with COMPOUND A**

| | **Characteristic** | **MDS Patients (N=16)** |
|---|---|---|
| **Age (years),** median (range) | | 67 (45, 78) |
| **Gender,** % male / % female | | 73 / 27 |
| **IDH2 mutation,** % R140, % R172 | | 88 / 12 |
| **ECOG performance status,** n (%) | | |
| | 0-1 | 12 (75) |
| | 2 | 4 (25) |
| **Number of prior anti-cancer regimens,** n (%) | | |
| | 0 | 4 (25) |
| | 1 | 6 (38) |
| | ≥2 | 6 (38) |
| **Type of prior MDS treatment,** n (%) | | |
| | Hypomethylating agents | 11 (69) |
| | Lenalidomide | 2 (13) |
| | Others* | 5 (31) |
| | Untreated | 4 (25) |
| **Time since diagnosis (months),** mean [SD] | | 15.7 [10.4] |
| **IPSS risk status,** n (%) | | |
| | Low / Intermediate-1 | 5 (31) |
| | Intermediate-2 / High | 8 (50) |
| | Missing | 3 (19) |
| **IPSS-R risk status,** n (%) | | |
| | Very Low / Low | 3 (19) |
| | Intermediate | 2 (13) |
| | High / Very High | 8 (50) |
| | Missing | 3 (19) |
| **ANC (10⁹/L),** median (min, max) | | 0.7 (0.2, 32.1) |
| **Platelets (10⁹/L),** median (min, max) | | 64 (19, 246) |
| **WBC (10⁹/L),** median (min, max) | | 2.0 (0.5, 44.4) |
| **Hemoglobin (g/dL),** median (min, max) | | 8.8 (7.3, 12.2) |
| *Vosaroxin, pracinostat, cytarabine and clofarabine, ruxolitinib, and sorafenib (n=1 pt each), ECOG, Eastern Cooperative Oncology Group (*see* Oken et. al, Am. J. Clin. Oncol. 5:649-655, (1982)); MDS, myelodysplastic syndromes; IPSS, International Prognostic Scoring System (*see* Greenberg et al., Blood. 89(6):2079-2088 (1997)); IPSS-R, revised IPSS; ANC, absolute neutrophil count; WBC, white blood cells | | |

Grade 3-4 treatment-emergent AEs (TEAEs) were reported for 14 patients (82%); the most frequent were hyperbilirubinemia (n=5, unconjugated. includes hyperbilirubinemia and blood bilirubin increased), pneumonia (n=4), thrombocytopenia (n=4), anemia (n = 3), hypokalemia (n=3), dyspnea (n = 2) and tumor lysis syndrome (n = 2). Six patients had a grade 3-4 drug-related TEAE. COMPOUND A-related serious TEAEs were reported in 4 patients (tumor lysis syndrome [2], blood bilirubin increased, transaminitis). No treatment related deaths were reported *(see* **Table 10).**

**Table 10: Grade 3-4 Treatment-emergent Adverse Events**

| Grade 3-4 TEAEs occurring in ≥2 patients | |
|---|---|
| Preferred Term | MDS Patients (n=17) n (%) |
| Hyperbilirubinemia* | 5 (30) |
| Pneumonia | 4 (24) |
| Thrombocytopenia | 4 (24) |
| Anemia | 3 (18) |
| Hypokalemia | 3 (18) |
| Dyspnea | 2 (12) |
| Tumor lysis syndrome | 2 (12) |
| *Unconjugated. Includes hyperbilirubinemia and blood bilirubin increased | |

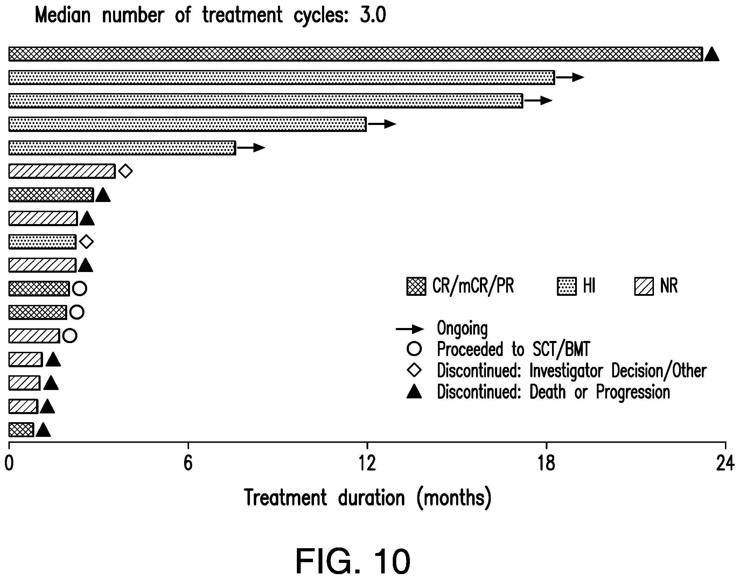

Of 13 patients who had received prior HMA therapy, 7 (54%) had a response with COMPOUND A. Of patients who attained HI, 2 had trilineage and 2 had bilineage improvement. The median time to response in the treated patients was 21 days (range 10-87). **Table 11** provides a summary of responses in patients. Figure 10 illustrates treatment durations and study outcomes. Median number of treatment of cycles was 3. Figure 10 illustraes overall survival in the treated patients indicating that at median follow-up of 7.5 months, median overall survival was not reached.

**Table 11. Overall response**

| | | **MDS Patients (N=17) n (%)** |
|---|---|---|
| **Overall response rate (CR + PR** + **mCR + HI)** | | **10/17 (59)** |
| Best response | | |
| | Complete Remission* | 1/11 (9) |
| | Partial Remission* | 1/11 (9) |
| | Marrow CR* | 3/11 (27) |
| | Any Hematologic Improvement (HI)† | 5/17 (29) |
| | HI-E | 3/15 (20) |
| | HI-P | 4/12 (33) |
| | HI-N | 4/10 (40) |
| Investigator-assessed; evaluable patients had ≥5% bone marrow blasts at baseline †HI was programmatically adjudicated per IWG 2006 criteria for MDS; denominators reflect eligibility | | |
| CR, complete remission; PR, partial remission; mCR, marrow CR; HI, hematologic | | |

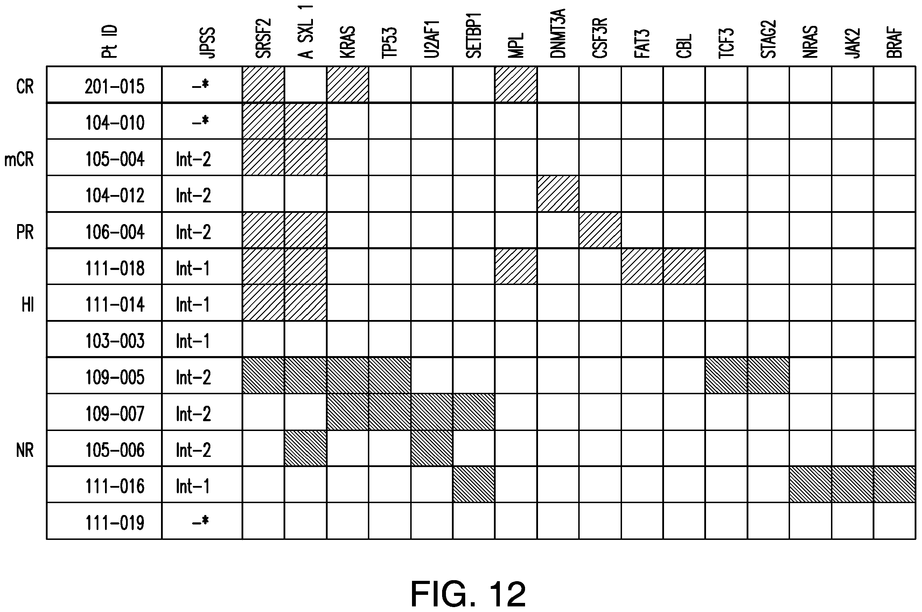

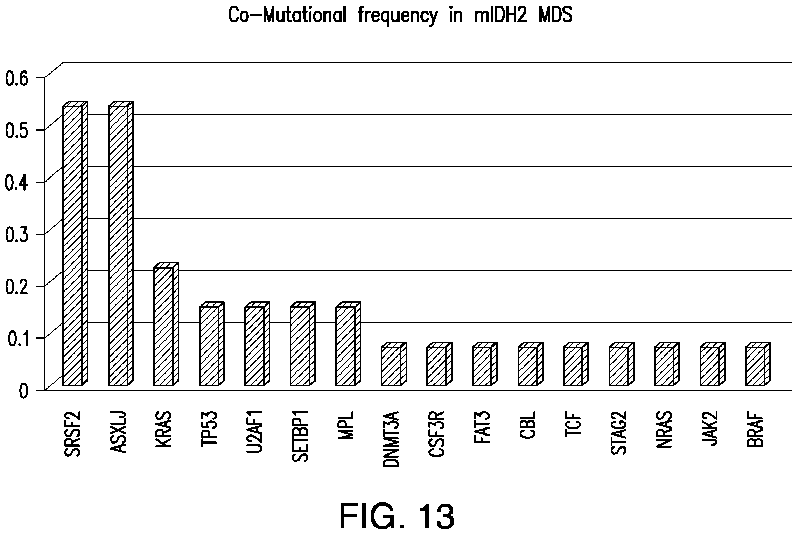

FoundationOne data were available for 13 out of 17 patients, the most frequently observed known somatic co-occurring mutations in these MDS patients were ASXI,1 and SRSF2. *(see* **Figure 12** which illustrates co-occurring mutations and clinical response to COMPOUND A monotherapy). **Figure 13** illustrates co-mutational frequency in MDS patients having mIDH2.

Analysis of MDS patients for association of gene mutations found with overall response rate (ORR) was performed on patients who received COMPOUND A and where a clinical response was captured for at least one time point during treatment (Table 12). An average of 2.6 genes were mutated in each patient beyond IDH2.

**Table 12: Association of known and likely somatic mutations identified by FoundationOne Heme panel with response to COMPOUND A**

| **Gene** | **ORR** | **ORR OR** | **ORR p-value** |
|---|---|---|---|
| SRSF2 | 0.857 | 12.000 | 0.1026 |
| TP53 | 0.000 | 0.082 | 0.1282 |
| U2AF1 | 0.000 | 0.082 | 0.1282 |
| SETBP1 | 0.000 | 0.082 | 0.1282 |
| MPL | 1.000 | 4.231 | 0.4872 |
| KRAS | 0.333 | 0.214 | 0.5105 |
| ASXL1 | 0.714 | 2.500 | 0.5921 |

In certain embodiments, a high level of co-mutational heterogeneity is present in MDS patients. In certain embodiments, 16 different additional genes other than IDH2 are present with mutations with an average of 2.6 genes mutated per patient. In certain embodiments, clinical responses (≥ HI) in MDS patients to COMPOUND A are observed in the presence of all mutations except TP53, U2AF1, SETBP1, TCF3, STAG2, NRAS, JAK2 and BRAF. In certain embodiments, patients with mutations in SRSF2, MPL and ASXL1 are enriched in MDS patients with clinical response (≥ HI) to COMPOUND A.

## Claims

1. A compound for use in a method of treating a myelodysplastic syndrome in a subject, wherein the compound is 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol having the following formula: 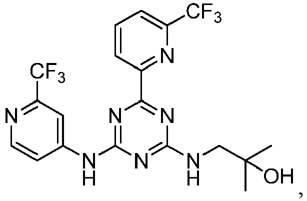 or a pharmaceutically acceptable salt, solvate, tautomer, or a polymorph thereof (COMPOUND 1), wherein the myelodysplastic syndrome is **characterized by** the presence of a mutant allele of IDH2 and the absence of a mutant allele of at least one other gene, wherein the other gene is selected from the group consisting of KRAS, TP53, SETBP1, and U2AF1.

2. The compound for use of claim 1, wherein the myelodysplastic syndrome is further **characterized by** the presence of one or more mutant alleles of one or more additional genes.

3. The compound for use of claim 1 or 2, wherein the myelodysplastic syndrome is **characterized by** the absence of a mutant allele of KRAS; or
wherein the myelodysplastic syndrome is **characterized by** the absence of a mutant allele of TP53; and/or
wherein the myelodysplastic syndrome is **characterized by** the absence of a mutant allele of SETBP1; and/or
wherein the myelodysplastic syndrome is **characterized by** the absence of a mutant allele of U2AF1.

4. The compound for use of any one of claims 1-3, wherein the myelodysplastic syndrome is **characterized by** the absence of a mutant allele of KRAS and TP53.

5. The compound for use of any one of claims 1-4, wherein the myelodysplastic syndrome is **characterized by** the presence of a mutant allele of a second gene, wherein the second gene is selected from the group consisting of ASXL1 and SRSF2.

6. The compound for use of any one of claims 1-5, wherein the myelodysplastic syndrome is **characterized by** the presence of a mutant allele of ASXL1; or
wherein the myelodysplastic syndrome is **characterized by** the presence of a mutant allele of SRSF2; or
wherein the myelodysplastic syndrome is **characterized by** the presence of mutant alleles of ASXL1 and SRSF2.

7. The compound for use of any one of claims 1-6, wherein the mutant allele of IDH2 is mIDH2-R140 or mIDH2-R172; preferably
wherein the mutant allele of IDH2 is mIDH2-R140Q, mIDH2-R140W, mIDH2-R140L, mIDH2-R172K, or mIDH2-R172G.

8. The compound for use of any one of claims 1-7, wherein COMPOUND 1 is administered at a dose of about 20 to 2000 mg/day; or
wherein COMPOUND 1 is administered at a dose of about 50 to 500 mg/day; or preferably
wherein the dose is about 60 mg/day, 100 mg/day, 150 mg/day, 200 mg/day or 300 mg/day.

9. The compound for use of any one of claims 1-8, wherein COMPOUND 1 is administered once daily; and/or
wherein COMPOUND 1 is administered for 1 to 25 cycles; or
wherein COMPOUND 1 is administered in 28-day cycles.

10. The compound for use of any one of claims 1-9, wherein COMPOUND 1 is administered orally; preferably
wherein COMPOUND 1 is administered once daily orally in 28-day cycles at the dose of about 100 mg/day.

11. The compound for use of any one of claims 1-10, wherein COMPOUND 1 is a mesylate salt of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol.

12. The compound for use of any one of claims 1-11, wherein the myelodysplastic syndrome is with excess blasts, subtype RAEB-1 or RAEB-2; and/or
wherein the myelodysplastic syndrome is untreated.

13. The compound for use of any one of claims 1-12, wherein COMPOUND 1 is administered as a first line treatment for MDS; or
wherein COMPOUND 1 is administered as a second line, third line, or fourth line of treatment for the myelodysplastic syndrome.

14. The compound for use of any one of claims 1-13, wherein the myelodysplastic syndrome is subsequent to acute myeloid leukemia.

15. A pharmaceutical composition for use in a method of treating a myelodysplastic syndrome in a subject, wherein the pharmaceutical composition comprises a therapeutically effective amount of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol having the following formula: 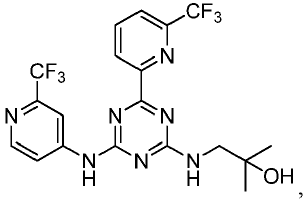 or a pharmaceutically acceptable salt, solvate, tautomer, or a polymorph thereof (COMPOUND 1) and an excipient, wherein the myelodysplastic syndrome is **characterized by** the presence of a mutant allele of IDH2 and the absence of a mutant allele of at least one other gene, wherein the other gene is selected from the group consisting of KRAS, TP53, SETBP1, and U2AF1.

## Patentansprüche

1. Verbindung zur Verwendung bei einem Verfahren zur Behandlung eines myelodysplastischen Syndroms bei einem Individuum, wobei die Verbindung 2-Methyl-1-[(4-[6-(trifluormethyl)pyridin-2-yl]-6-{[2-(trifluormethyl)pyridin-4-yl]amino}-1,3,5-triazin-2-yl)amino]propan-2-ol mit der folgenden Formel ist: 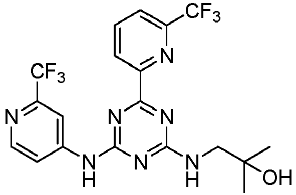 oder ein pharmazeutisch unbedenkliches Salz, Solvat, Tautomer oder ein Polymorph davon (VERBINDUNG 1), wobei das myelodysplastische Syndrom **gekennzeichnet ist durch** die Anwesenheit eines mutierten Allels von IDH2 und die Abwesenheit eines mutierten Allels von mindestens einem anderen Gen, wobei das andere Gen ausgewählt ist aus der Gruppe bestehend aus KRAS, TP53, SETBP1 und U2AF1.

2. Verbindung zur Verwendung nach Anspruch 1, wobei das myelodysplastische Syndrom ferner **gekennzeichnet ist durch** die Anwesenheit eines oder mehrerer mutierter Allele eines oder mehrerer zusätzlicher Gene.

3. Verbindung zur Verwendung nach Anspruch 1 oder 2, wobei das myelodysplastische Syndrom **gekennzeichnet ist durch** die Abwesenheit eines mutierten Allels von KRAS; oder
wobei das myelodysplastische Syndrom **gekennzeichnet ist durch** die Abwesenheit eines mutierten Allels von TP53; und/oder
wobei das myelodysplastische Syndrom **gekennzeichnet ist durch** die Abwesenheit eines mutierten Allels von SETBP1; und/oder
wobei das myelodysplastische Syndrom **gekennzeichnet ist durch** die Abwesenheit eines mutierten Allels von U2AF1.

4. Verbindung zur Verwendung nach einem der Ansprüche 1-3, wobei das myelodysplastische Syndrom **gekennzeichnet ist durch** die Abwesenheit eines mutierten Allels von KRAS und TP53.

5. Verbindung zur Verwendung nach einem der Ansprüche 1-4, wobei das myelodysplastische Syndrom **gekennzeichnet ist durch** die Anwesenheit eines mutierten Allels eines zweiten Gens, wobei das zweite Gen ausgewählt ist aus der Gruppe bestehend aus ASXL1 und SRSF2.

6. Verbindung zur Verwendung nach einem der Ansprüche 1-5, wobei das myelodysplastische Syndrom **gekennzeichnet ist durch** die Anwesenheit eines mutierten Allels von ASXL1; oder
wobei das myelodysplastische Syndrom **gekennzeichnet ist durch** die Anwesenheit eines mutierten Allels von SRSF2; oder
wobei das myelodysplastische Syndrom **gekennzeichnet ist durch** die Anwesenheit mutierter Allele von ASXL1 und SRSF2.

7. Verbindung zur Verwendung nach einem der Ansprüche 1-6, wobei das mutierte Allel von IDH2 mIDH2-R140 oder mIDH2-R172 ist; vorzugsweise wobei das mutierte Allel von IDH2 mIDH2-R140Q, mIDH2-R140W, mIDH2-R140L, mIDH2-R172K oder mIDH2-R172G ist.

8. Verbindung zur Verwendung nach einem der Ansprüche 1-7, wobei VERBINDUNG 1 in einer Dosis von etwa 20 bis 2000 mg/Tag verabreicht wird; oder
wobei VERBINDUNG 1 in einer Dosis von etwa 50 bis 500 mg/Tag verabreicht wird; oder vorzugsweise
wobei die Dosis etwa 60 mg/Tag, 100 mg/Tag, 150 mg/Tag, 200 mg/Tag oder 300 mg/Tag beträgt.

9. Verbindung zur Verwendung nach einem der Ansprüche 1-8, wobei VERBINDUNG 1 einmal täglich verabreicht wird; und/oder
wobei VERBINDUNG 1 über 1 bis 25 Zyklen verabreicht wird; oder
wobei VERBINDUNG 1 in 28-Tage-Zyklen verabreicht wird.

10. Verbindung zur Verwendung nach einem der Ansprüche 1-9, wobei VERBINDUNG 1 oral verabreicht wird; vorzugsweise
wobei VERBINDUNG 1 einmal täglich oral in 28-Tage-Zyklen in der Dosis von etwa 100 mg/Tag verabreicht wird.

11. Verbindung zur Verwendung nach einem der Ansprüche 1-10, wobei VERBINDUNG 1 ein Mesylatsalz ist von 2-Methyl-1-[(4-[6-(trifluormethyl)pyridin-2-yl]-6-{[2-(trifluormethyl)pyridin-4-yl]amino}-1,3,5-triazin-2-yl)amino]propan-2-ol.

12. Verbindung zur Verwendung nach einem der Ansprüche 1-11, wobei das myelodysplastische Syndrom ist ein myelodysplastisches Syndrom mit Blastenüberschuss, Subtyp RAEB-1 oder RAEB-2; und/oder
wobei das myelodysplastische Syndrom unbehandelt ist.

13. Verbindung zur Verwendung nach einem der Ansprüche 1-12, wobei VERBINDUNG 1 als Erstlinienbehandlung für MDS verabreicht wird; oder
wobei VERBINDUNG 1 als Zweitlinien-, Drittlinien- oder Viertlinienbehandlung für das myelodysplastische Syndrom verabreicht wird.

14. Verbindung zur Verwendung nach einem der Ansprüche 1-13, wobei das myelodysplastische Syndrom die Folge einer akuten myeloischer Leukämie ist.

15. Pharmazeutische Zusammensetzung zur Verwendung bei einem Verfahren zur Behandlung eines myelodysplastischen Syndroms bei einem Individuum, wobei die pharmazeutische Zusammensetzung eine therapeutisch wirksame Menge umfasst von 2-Methyl-1-[(4-[6-(trifluormethyl)pyridin-2-yl]-6-{[2-(trifluormethyl)pyridin-4-yl]amino}-1,3,5-triazin-2-yl)amino]propan-2-ol mit der folgenden Formel: 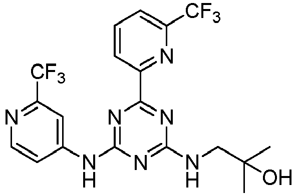 oder eines pharmazeutisch unbedenklichen Salzes, Solvats, Tautomers oder eines Polymorphs davon (VERBINDUNG 1) und einen Hilfsstoff, wobei das myelodysplastische Syndrom **gekennzeichnet ist durch** die Anwesenheit eines mutierten Allels von IDH2 und die Abwesenheit eines mutierten Allels von mindestens einem anderen Gen, wobei das andere Gen ausgewählt ist aus der Gruppe bestehend aus KRAS, TP53, SETBP1 und U2AF1.

## Revendications

1. Un composé pour l'utilisation dans un procédé de traitement d'un syndrome myélodysplasique chez un sujet, le composé étant le 2-méthyl-1-[(4-[6-(trifluorométhyl)pyridin-2-yl]-6-{[2-(trifluorométhyl)pyridin-4-yl]amino}-1,3,5-triazin-2-yl)amino]propan-2-ol ayant la formule suivante: 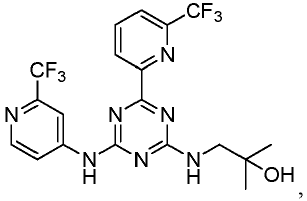 ou un sel pharmaceutiquement acceptable, un solvate, un tautomère, ou un polymorphe de ceux-ci (COMPOSÉ 1), le syndrome myélodysplasique étant **caractérisé par** la présence d'un allèle mutant de IDH2 et l'absence d'un allèle mutant d'au moins un autre gène, l'autre gène étant choisi parmi le groupe constitué par KRAS, TP53, SETBP1, et U2AF1.

2. Le composé pour l'utilisation selon la revendication 1, le syndrome myélodysplasique étant en outre **caractérisé par** la présence d'un ou plusieurs allèles mutants d'un ou plusieurs gènes supplémentaires.

3. Le composé pour l'utilisation selon la revendication 1 ou 2, le syndrome myélodysplasique étant **caractérisé par** l'absence d'un allèle mutant de KRAS ; ou
le syndrome myélodysplasique étant **caractérisé par** l'absence d'un allèle mutant de TP53; et/ou
le syndrome myélodysplasique étant **caractérisé par** l'absence d'un allèle mutant de SETBP1; et/ou
le syndrome myélodysplasique étant **caractérisé par** l'absence d'un allèle mutant de U2AF 1.

4. Le composé pour l'utilisation selon l'une quelconque des revendications 1 à 3, le syndrome myélodysplasique étant **caractérisé par** l'absence d'un allèle mutant de KRAS et TP53.

5. Le composé pour l'utilisation selon l'une quelconque des revendications 1 à 4, le syndrome myélodysplasique étant **caractérisé par** la présence d'un allèle mutant d'un deuxième gène, le deuxième gène étant choisi parmi le groupe constitué par ASXL1 et SRSF2.

6. Le composé pour l'utilisation selon l'une quelconque des revendications 1 à 5, le syndrome myélodysplasique étant **caractérisé par** la présence d'un allèle mutant de ASXL1; ou
le syndrome myélodysplasique étant **caractérisé par** la présence d'un allèle mutant de SRSF2; ou
le syndrome myélodysplasique étant **caractérisé par** la présence d'allèles mutants de ASXL1 et SRSF2.

7. Le composé pour l'utilisation selon l'une quelconque des revendications 1 à 6, l'allèle mutant de IDH2 étant mIDH2-R140 ou mIDH2-R172 ; préférablement l'allèle mutant de IDH2 étant mIDH2-R140Q, mIDH2-R140W, mIDH2-R140L, mIDH2-R172K, ou mIDH2-R172G.

8. Le composé pour l'utilisation selon l'une quelconque des revendications 1 à 7, le COMPOSÉ 1 étant administré à raison d'une dose d'environ 20 à 2 000 mg/jour; ou le COMPOSÉ 1 étant administré à raison d'une dose d'environ 50 à 500 mg/jour; ou préférablement la dose étant d'environ 60 mg/jour, 100 mg/jour, 150 mg/jour, 200 mg/jour ou 300 mg/jour.

9. Le composé pour l'utilisation selon l'une quelconque des revendications 1 à 8, le COMPOSÉ 1 étant administré une fois par jour; et/ou
le COMPOSÉ 1 étant administré pendant 1 à 25 cycles; ou
le COMPOSÉ 1 étant administré en cycles de 28 jours.

10. Le composé pour l'utilisation selon l'une quelconque des revendications 1 à 9, le COMPOSÉ 1 étant administré oralement; préférablement
le COMPOSÉ 1 étant administré une fois par jour oralement en cycles de 28 jours à la dose d'environ 100 mg/jour.

11. Le composé pour l'utilisation selon l'une quelconque des revendications 1 à 10, le COMPOSÉ 1 étant un sel de mésylate de 2-méthyl-1-[(4-[6-(trifluorométhyl)pyridin-2-yl]-6-{[2-(trifluorométhyl)pyridin-4-yl]amino}-1,3,5-triazin-2-yl)amino]propan-2-ol.

12. Le composé pour l'utilisation selon l'une quelconque des revendications 1 à 11, le syndrome myélodysplasique étant avec excès de blastes, sous-type RAEB-1 ou RAEB 2; et/ou
le syndrome myélodysplasique étant non traité.

13. Le composé pour l'utilisation selon l'une quelconque des revendications 1 à 12, le COMPOSÉ 1 étant administré comme un traitement de première ligne pour MDS; ou le COMPOSÉ 1 étant administré comme un traitement de deuxième ligne, de troisième ligne ou de quatrième ligne pour le syndrome myélodysplasique.

14. Le composé pour l'utilisation selon l'une quelconque des revendications 1 à 13, le syndrome myélodysplasique étant consécutif à une leucémie myéloïde aiguë.

15. Le composition pharmaceutique pour l'utilisation dans un procédé de traitement d'un syndrome myélodysplasique chez un sujet, la composition pharmaceutique comprenant une quantité thérapeutiquement efficace de 2-méthyl-1-[(4-[6-(trifluorométhyl)pyridin-2-yl]-6-{[2-(trifluorométhyl)pyridin-4-yl]amino}-1,3,5-triazin-2-yl)amino]propan-2-ol ayant la formule suivante: 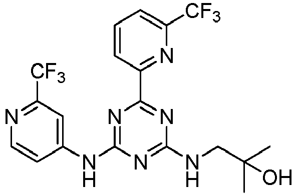 ou un sel pharmaceutiquement acceptable, un solvate, un tautomère ou un polymorphe de ceux-ci (COMPOSÉ 1) et un excipient, le syndrome myélodysplasique étant **caractérisé par** la présence d'un allèle mutant de IDH2 et l'absence d'un allèle mutant d'au moins un autre gène, l'autre gène étant choisi parmi le groupe constitué par KRAS, TP53, SETBP1, et U2AF1.
